(19) **Europäisches Patentamt European Patent Office Office européen des brevets**

(11) **EP 3 672 477 B1**

(12) **EUROPEAN PATENT SPECIFICATION**

(45) Date of publication and mention of the grant of the patent:
**24.01.2024 Bulletin 2024/04**

(21) Application number: **18847612.1**

(22) Date of filing: **27.08.2018**

(51) International Patent Classification (IPC):
**A61B 5/00** (2006.01)    **A61C 7/00** (2006.01)
**G06F 17/00** (2019.01)    **G06G 7/48** (2006.01)
**G06G 7/58** (2006.01)    **G06T 17/00** (2006.01)

(52) Cooperative Patent Classification (CPC):
**A61C 9/004; A61C 7/002; A61C 9/0053;**
A61C 2007/004

(86) International application number:
**PCT/US2018/048070**

(87) International publication number:
**WO 2019/040927 (28.02.2019 Gazette 2019/09)**

(54) **METHOD AND APPARATUS FOR ORTHODONTIC TREATMENT PLANNING**

METHODE ET APPAREIL POUR LA PLANIFICATION DU TRAITEMENT ORTHODONTIQUE

VERFAHREN UND GERÄT ZUR KIEFERORTHOPÄDISCHEN BEHANDLUNGSPLANUNG

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.08.2017 US 201762550013 P**

(43) Date of publication of application:
**01.07.2020 Bulletin 2020/27**

(73) Proprietors:
• **Chen, Shoupu**
  **Atlanta, GA 30339 (US)**
• **Inglese, Jean-Marc**
  **Atlanta, GA 30339 (US)**
• **Wong, Victor C.**
  **Atlanta, GA 30339 (US)**
• **Faure, Jacques**
  **Atlanta, GA 30339 (US)**
• **Treil, Jacques**
  **Atlanta, GA 30339 (US)**

(72) Inventors:
• **Chen, Shoupu**
  **Atlanta, GA 30339 (US)**

• **Inglese, Jean-Marc**
  **Atlanta, GA 30339 (US)**
• **Wong, Victor C.**
  **Atlanta, GA 30339 (US)**
• **Faure, Jacques**
  **Atlanta, GA 30339 (US)**
• **Treil, Jacques**
  **Atlanta, GA 30339 (US)**

(74) Representative: **Schmidbauer, Andreas Konrad**
**Wagner & Geyer Partnerschaft mbB**
**Patent- und Rechtsanwälte**
**Gewürzmühlstrasse 5**
**80538 München (DE)**

(56) References cited:
WO-A1-2015/179084    WO-A1-2015/179084
US-A1- 2002 010 568    US-A1- 2003 096 210
US-A1- 2004 073 417    US-A1- 2004 110 110
US-A1- 2006 073 436    US-A1- 2006 263 739
US-A1- 2009 191 503    US-A1- 2010 138 025
US-A1- 2012 015 316    US-A1- 2012 123 577
US-A1- 2013 204 583    US-A1- 2013 209 952
US-A1- 2014 288 894    US-A1- 2014 288 894

EP 3 672 477 B1

**Description**

**FIELD OF THE INVENTION**

The present invention relates generally to image processing in x-ray computed tomography and, more particularly, to biometrics analysis from 3-D images for guidance in orthodontic treatment.

**BACKGROUND OF THE INVENTION**

Cephalometric analysis is the study of the dental and skeletal relationships for the head and is used by dentists and orthodontists as an assessment and planning tool for improved treatment of a patient. Conventional cephalometric analysis identifies bony and soft tissue landmarks in 2-D cephalometric radiographs in order to diagnose facial features and abnormalities prior to treatment, or to evaluate the progress of treatment.

For example, a dominant abnormality that can be identified in cephalometric analysis is the anteroposterior problem of malocclusion, relating to the skeletal relationship between the maxilla and mandible. Malocclusion is classified based on the relative position of the maxillary first molar. For Class I, neutrocclusion, the molar relationship is normal but other teeth may have problems such as spacing, crowding, or over- or under-eruption. For Class II, distocclusion, the mesiobuccal cusp of the maxillary first molar rests between the first mandible molar and second premolar. For Class III, mesiocclusion, the mesiobuccal cusp of the maxillary first molar is posterior to the mesiobuccal grooves of the mandibular first molar.

An exemplary conventional 2-D cephalometric analysis method described by Steiner in an article entitled "Cephalometrics in Clinical Practice" (paper read at the Charles H. Tweed Foundation for Orthodontic Research, October 1956, pp. 8-29) assesses maxilla and mandible in relation to the cranial base using angular measures. In the procedure described, Steiner selects four landmarks: Nasion, Point A, Point B and Sella. The Nasion is the intersection of the frontal bone and two nasal bones of the skull. Point A is regarded as the anterior limit of the apical base of the maxilla. Point B is regarded as the anterior limit of the apical base of the mandible. The Sella is at the mid-point of the sella turcica. The angle SNA (from Sella to Nasion, then to Point A) is used to determine if the maxilla is positioned anteriorly or posteriorly to the cranial base; a reading of about 82 degrees is regarded as normal. The angle SNB (from Sella to Nasion then to Point B) is used to determine if the mandible is positioned anteriorly or posteriorly to the cranial base; a reading of about 80 degrees is regarded as normal.

Recent studies in orthodontics indicate that there are persistent inaccuracies and inconsistencies in results provided using conventional 2-D cephalometric analysis. One notable study is entitled "In vivo comparison of conventional and cone beam CT synthesized cephalograms" by Vandana Kumar et al. in Angle Orthodontics, September 2008, pp. 873-879.

Due to fundamental limitations in data acquisition, conventional 2-D cephalometric analysis is focused primarily on aesthetics, without the concern of balance and symmetry about the human face. As stated in an article entitled "The human face as a 3D model for cephalometric analysis" by Treil et al. in World Journal of Orthodontics, pp. 1-6, plane geometry is inappropriate for analyzing anatomical volumes and their growth; only a 3-D diagnosis is able to suitably analyze the anatomical maxillofacial complex. The normal relationship has two more significant aspects: balance and symmetry, when balance and symmetry of the model are stable, these characteristics define what is normal for each person.

US 6 879 712 B2, entitled "System and method of digitally modeling craniofacial features for the purposes of diagnosis and treatment predictions" to Tuncay et al., discloses a method of generating a computer model of craniofacial features. The three-dimensional facial features data are acquired using laser scanning and digital photographs; dental features are acquired by physically modeling the teeth. The models are laser scanned. Skeletal features are then obtained from radiographs. The data are combined into a single computer model that can be manipulated and viewed in three dimensions. The model also has the ability for animation between the current modeled craniofacial features and theoretical craniofacial features.

US 6 250 918 B1, entitled "Method and apparatus for simulating tooth movement for an orthodontic patient" to Sachdeva et al., discloses a method of determining a 3-D direct path of movement from a 3-D digital model of an actual orthodontic structure and a 3-D model of a desired orthodontic structure. This method simulates tooth movement based on each tooth's corresponding three-dimensional direct path using laser scanned crown and markers on the tooth surface for scaling. There is no true whole tooth 3-D data using the method described.

US 2009 / 0 191 503 A1 relates to a method and a system for establishing an initial position of a tooth, determining a target position of the tooth in a treatment plan, calculating a movement vector associated with the tooth movement from the initial position to the target position, determining a plurality of components corresponding to the movement vector, and determining a corresponding one or more positions of a respective one or more attachment devices relative to a surface plane of the tooth such that the one or more attachment devices engages with a dental appliance.

Document US 2014/288894A discloses a computer method to create a plan for repositioning an orthodontic patient's teeth. The computer receives an initial digital data set representing the patient's teeth at their initial positions and a final digital data set representing the teeth at their final positions. The computer then uses the data sets to generate treatment paths consisting of a series of vectors along which teeth will move from the initial positions to the final positions.

Although significant strides have been made toward developing techniques that automate entry of measurements and computation of biometric data for craniofacial features based on such measurements, there is considerable room for improvement. Even with the benefit of existing tools, the practitioner requires sufficient training in order to use the biometric data effectively. The sizable amount of measured and calculated data complicates the task of developing and maintaining a treatment plan and can increase the risks of human oversight and error.

Thus, it can be seen that there would be particular value in development of analysis utilities that generate and report cephalometric results that can help to direct orthodontic treatment planning and to track patient progress at different stages of ongoing treatment.

## SUMMARY OF THE INVENTION

The present invention relates to a method for orthodontic treatment planing as defined in claim 1, a method for fabricating a positional corrective device as defined in claim 13 and an apparatus for providing guidance for orthodontics as defined in claim 14.

Further embodiments of the invention are inter alia disclosed in the dependent claims. It is an object of the present disclosure to address the need for improved ways to analyze and apply 3-D anatomical data to ongoing orthodontic treatment. With this object in mind, the present disclosure provides a method for generating arch forms based on patient dentition as guidance tools for correcting orthodontic conditions.

According to the present invention, there is provided a method, executed at least in part by a computer apparatus, comprising:

(a) acquiring three-dimensional data from scans of maxillofacial and dental anatomy of a patient;
(b) computing a plurality of cephalometric values from the acquired three-dimensional data;
(c) processing the computed cephalometric values and generating metrics indicative of initial tooth positioning along a dental arch of the patient;
(d) generating metrics indicative of corrected tooth positioning according to a desired dental arch form;
(e) analyzing the metrics generated in steps (c) and (d) to calculate desired movement vectors (V) for individual teeth within the dental arch; and
(f) displaying the generated metrics for both the initial and corrected tooth positioning and the calculated desired movement vectors (V) along the dental arch in an overlaid manner.

A feature of the present disclosure is automatic generation and reporting of patient-specific coherent and optimized geometric primitive parameters and initial quantitative tooth movement values for orthodontic treatment.

Embodiments of the present disclosure, in a synergistic manner, integrate skills of a human operator of the system with computer capabilities for feature identification. This takes advantage of human skills of creativity, use of heuristics, flexibility, and judgment, and combines these with computer advantages, such as speed of computation, capability for exhaustive and accurate processing, and reporting and data access capabilities.

These and other aspects, objects, features and advantages of the present disclosure will be more clearly understood and appreciated from a review of the following detailed description of the preferred embodiments and appended claims, and by reference to the accompanying drawings.

## BRIEF DESCRIPTION OF THE DRAWINGS

The foregoing and other objects, features, and advantages of the invention will be apparent from the following more particular description of the embodiments of the disclosure, as illustrated in the accompanying drawings. The elements of the drawings are not necessarily to scale relative to each other.

Figure 1 is a schematic diagram showing an imaging system for providing cephalometric analysis.
Figure 2 is a logic flow diagram showing processes for 3-D cephalometric analysis according to an embodiment of the present disclosure.
Figure 3 is a view of 3-D rendered CBCT head volume images.
Figure 4 is a view of a 3-D rendered teeth volume image after teeth segmentation.
Figure 5 is a view of a user interface that displays three orthogonal views of the CBCT head volume images and operator-entered reference marks.
Figure 6 is a view of 3-D rendered CBCT head volume images with a set of 3-D reference marks displayed.
Figures 7A, 7B, and 7C are perspective views that show identified anatomical features that provide a framework for cephalometric analysis.
Figure 8 is a logic flow diagram that shows steps for accepting operator instructions that generate the framework used for cephalometric analysis.
Figures 9A, 9B, and 9C show an operator interface for specifying the location of anatomical features using operator-entered reference marks.
Figures 10A, 10B, 10C, 10D, and 10E are graphs that show how various derived parameters are calculated using the volume image data and corresponding operator-entered reference marks.
Figure 11 is a 3-D graph showing a number of derived cephalometric parameters from segmented teeth data.
Figure 12 is a 2-D graph showing the derived cephalometric parameters from segmented teeth data.

Figure 13 is another 3-D graph showing the derived cephalometric parameters from segmented teeth data.

Figure 14 is a graph showing the derived cephalometric parameters from segmented teeth data and treatment parameter.

Figure 15 is a 3-D graph that shows how tooth exclusion is learned by the system.

Figure 16A is a perspective view that shows teeth of a digital phantom.

Figure 16B is a 3-D graph showing computed axes of inertia systems for upper and lower jaws.

Figure 17A is a graph showing parallelism for specific tooth structures.

Figure 17B is a graph showing parallelism for specific tooth structures.

Figure 18A is a perspective view that shows teeth of a digital phantom with a tooth missing.

Figure 18B is a graph showing computed axes of inertia systems for upper and lower jaws for the example of Figure 18A.

Figure 19A is a graph showing lack of parallelism for specific tooth structures.

Figure 19B is a graph showing lack of parallelism for specific tooth structures.

Figure 20A is a perspective view that shows teeth of a digital phantom with tooth exclusion.

Figure 20B is a graph showing computed axes of inertia systems for upper and lower jaws for the example of Figure 20A.

Figure 21A is an example showing tooth exclusion for a missing tooth.

Figure 21B is a graph showing computed axes of inertia systems for upper and lower jaws for the example of Figure 21A.

Figure 22A is an example showing tooth exclusion for a missing tooth.

Figure 22B is a graph showing computed axes of inertia systems for upper and lower jaws for the example of Figure 22A.

Figure 23A is an image that shows the results of excluding specific teeth.

Figure 23B is a graph showing computed axes of inertia systems for upper and lower jaws for the example of Figure 23A.

Figure 24 shows a number of landmarks and coordinate axes or vectors of the DOL reference system.

Figure 25 shows landmark remapping to the alternate space of the DOL reference system.

Figure 26 shows, from a side view, an example with transformed teeth inertia systems using this re-mapping.

Figure 27 is a schematic diagram that shows an independent network for the analysis engine according to an embodiment of the present disclosure.

Figure 28 is a schematic diagram that shows a dependent or coupled network for the analysis engine according to an embodiment of the present disclosure.

Figure 29 shows pseudo-code for an algorithm using the independent network arrangement of Figure 27.

Figure 30 shows pseudo-code for an algorithm using the dependent network arrangement of Figure 28.

Figure 31A lists example parameters as numerical values and their interpretation.

Figures 31B, 31C and 31D list, for a particular patient, example parameters as numerical values and their interpretation with respect to maxillofacial asymmetry, based on exemplary total maxillofacial asymmetry parameters according to exemplary embodiments of this application.

Figure 32A shows exemplary tabulated results for a particular example with bite analysis and arches angle characteristics.

Figure 32B shows exemplary tabulated results for a particular example for torque of upper and lower incisors.

Figure 32C shows exemplary tabulated results for another example with assessment of biretrusion or biprotrusion.

Figure 32D shows an exemplary summary listing of results for cephalometric analysis of a particular patient.

Figure 32E shows a detailed listing for one of the conditions listed in Figure 35.

Figure 33 shows a system display with a recommendation message based on analysis results.

Figure 34 shows a system display with a graphical depiction to aid analysis results.

Figure 35 shows an exemplary report for asymmetry according to an embodiment of the present disclosure.

Figure 36 is a graph that shows relative left-right asymmetry for a patient in a front view.

Figure 37 is a graph showing relative overlap of left and right sides of a patient's face.

Figure 38 is a graph showing facial divergence.

Figure 39 is a logic flow diagram that shows the logic processing mechanisms and data that can be used for providing assessment and guidance to support orthodontic applications.

Figure 40 shows an image of a typical patient condition, arch-left rotation.

Figure 41 shows a 3-D mapping of tooth inertia centers along an arch.

Figure 42 shows a plot of tooth inertia centers arranged along a piecewise linear curve that connects the inertia centers, in their original positions, projected to the 2D x-y plane.

Figure 43 shows, for the inertia centers in Figure 42, the relative position of an optimized smooth poly-curve.

Figure 44 shows needed motion of the teeth from their original positions to desired positions based upon the computed optimization as displayed and reported.

Figure 45 shows a graph with tooth displacement vectors indicated, based on the above computation.

Figure 46 shows a graph with vector displacement decomposed into tangential and normal directions.

Figures 47 and 48 show adjustments for a multi-stage tooth arch form optimization strategy adopted according to the present disclosure.

Figure 49 shows a computer-generated listing of movement vectors V for each of 14 teeth of a dental arch for an orthodontic patient

Figure 50A shows the distribution of teeth in an initial, uncorrected arch for an exemplary orthodontic patient.

Figure 50B shows the digitally corrected arch of Figure 50A following treatment recommendations provided by the system of the present disclosure.

Figure 50C shows initial and optimized arches overlaid, with the original shown in outline, in a 3D view.

Figure 50D shows another example with the initial arch in outline, overlaid on the optimized arch, in a 2D axial view.

Figure 51 shows an operator interface display for controlling the processing to provide orthodontic data and for display of processing results.

Figure 52 is a schematic diagram that shows an exemplary fabrication system for forming an orthodontic appliance using the optimization methods of the present disclosure.

Figure 53 is a logic flow diagram that shows a sequence for applying results of the orthodontic optimization to the task of appliance design and fabrication.

## DETAILED DESCRIPTION

In the following detailed description of embodiments of the present disclosure, reference is made to the drawings in which the same reference numerals are assigned to identical elements in successive figures. It should be noted that these figures are provided to illustrate overall functions and relationships according to embodiments of the present invention and are not provided with intent to represent actual size or scale.

Where they are used, the terms "first", "second", "third", and so on, do not necessarily denote any ordinal or priority relation, but may be used for more clearly distinguishing one element or time interval from another.

In the context of the present disclosure, the term "image" refers to multi-dimensional image data that is composed of discrete image elements. For 2-D images, the discrete image elements are picture elements, or pixels. For 3-D images, the discrete image elements are volume image elements, or voxels. The term "volume image" is considered to be synonymous with the term "3-D image".

In the context of the present disclosure, the term "code value" refers to the value that is associated with each 2-D image pixel or, correspondingly, each volume image data element or voxel in the reconstructed 3-D volume image. The code values for computed tomography (CT) or cone-beam computed tomography (CBCT) images are often, but not always, expressed in Hounsfield units that provide information on the attenuation coefficient of each voxel.

In the context of the present disclosure, the term "geometric primitive" relates to an open or closed shape such as a rectangle, circle, line, traced curve, or other traced pattern. The terms "landmark" and "anatomical feature" are considered to be equivalent and refer to specific features of patient anatomy as displayed.

In the context of the present disclosure, the terms "viewer", "operator", and "user" are considered to be equivalent and refer to the viewing practitioner or other person who views and manipulates an image, such as a dental image, on a display monitor. An "operator instruction" or "viewer instruction" is obtained from explicit commands entered by the viewer, such as using a computer mouse or touch screen or keyboard entry.

The term "highlighting" for a displayed feature has its conventional meaning as is understood to those skilled in the information and image display arts. In general, highlighting uses some form of localized display enhancement to attract the attention of the viewer. Highlighting a portion of an image, such as an individual organ, bone, or structure, or a path from one chamber to the next, for example, can be achieved in any of a number of ways, including, but not limited to, annotating, displaying a nearby or overlaying symbol, outlining or tracing, display in a different color or at a markedly different intensity or gray scale value than other image or information content, blinking or animation of a portion of a display, or display at higher sharpness or contrast.

In the context of the present disclosure, the descriptive term "derived parameters" relates to values calculated from processing of acquired or entered data values. Derived parameters may be a scalar, a point, a line, a volume, a vector, a plane, a curve, an angular value, an image, a closed contour, an area, a length, a matrix, a tensor, or a mathematical expression.

The term "set", as used herein, refers to a non-empty set, as the concept of a collection of elements or members of a set is widely understood in elementary mathematics. The term "subset", unless otherwise explicitly stated, is used herein to refer to a non-empty proper subset, that is, to a subset of the larger set, having one or more members. For a set S, a subset may comprise the complete set S. A "proper subset" of set S, however, is strictly contained in set S and excludes at least one member of set S. Alternately, more formally stated, as the term is used in the present disclosure, a subset B can be considered to be a proper subset of set S if (i) subset B is non-empty and (ii) if intersection $B \cap S$ is also non-empty and subset B further contains only elements that are in set S and has a cardinality that is less than that of set S.

In the context of the present disclosure, a "plan view" or "2-D view" is a 2-dimensional (2-D) representation or projection of a 3-dimensional (3-D) object from the position of a horizontal plane through the object. This term is synonymous with the term "image slice" that is conventionally used to describe displaying a 2-D planar representation from within 3-D volume image data from a particular perspective. 2-D views of the 3-D volume data are considered to be substantially orthogonal if the corresponding planes at which the views are taken are disposed at 90 (+ / - 10) degrees from each other, or at an integer multiple n of 90 degrees from each other (*n\*90* degrees, +/- 10 degrees).

In the context of the present disclosure, the general term "dentition element" relates to teeth, prosthetic devices such as dentures and implants, and supporting structures for teeth and associated prosthetic device, including jaws.

The terms "poly-curve" and "polycurve" are equivalent and refer to a curve defined according to a polynomial.

The subject matter of the present disclosure relates to digital image processing and computer vision technologies, which is understood to mean technologies that digitally process data from a digital image to recognize and thereby assign useful meaning to human-understandable objects, attributes or conditions, and then to utilize the results obtained in further processing of the digital image.

As noted earlier in the background section, conventional 2-D cephalometric analysis has a number of significant drawbacks. It is difficult to center the patient's head in the cephalostat or other measuring device, making reproducibility unlikely. The two dimensional radiographs that are obtained produce overlapped head anatomy images rather than 3-D images. Locating landmarks on cephalograms can be difficult and results are often inconsistent (see the article entitled "Cephalometrics for the next millennium" by P. Planche and J. Treil in The Future of Orthodontics, ed. Carine Carels, Guy Willems, Leuven University Press, 1998, pp. 181 - 192). The job of developing and tracking a treatment plan is complex, in part, because of the significant amount of cephalometric data that is collected and calculated.

An embodiment of the present disclosure utilizes Treil's theory in terms of the selection of 3-D anatomic feature points, parameters derived from these feature points, and the way to use these derived parameters in cephalometric analysis. Reference publications authored by Treil include "The Human Face as a 3D Model for Cephalometric Analysis" Jacques Treil, B, Waysenson, J. Braga and J. Casteigt in World Journal of Orthodontics, 2005 Supplement, Vol. 6, issue 5, pp. 33-38; and "3D Tooth Modeling for Orthodontic Assessment" by J. Treil, J. Braga, J.-M. Loubes, E. Maza, J.-M. Inglese, J. Casteigt, and B. Waysenson in Seminars in Orthodontics, Vol. 15, No. 1, March 2009).

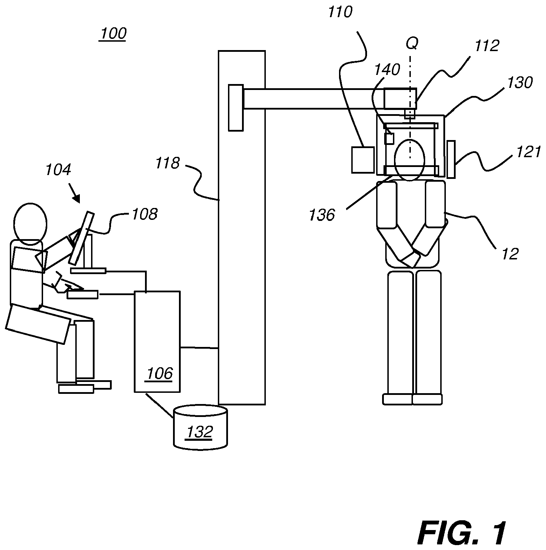

The schematic diagram of Figure 1 shows an imaging apparatus 100 for 3-D CBCT cephalometric imaging. For imaging a patient 12, a succession of multiple 2-D projection images is obtained and processed using imaging apparatus 100. A rotatable mount 130 is provided on a column 118, preferably adjustable in height to suit the size of patient 12. Mount 130 maintains an x-ray source 110 and a radiation sensor 121 on opposite sides of the head of patient 12 and rotates to orbit source 110 and sensor 121 in a scan pattern about the head. Mount 130 rotates about an axis Q that corresponds to a central portion of the patient's head, so that components attached to mount 130 orbit the head. Sensor 121, a digital sensor, is coupled to mount 130, opposite x-ray source 110 that emits a radiation pattern suitable for CBCT volume imaging. An optional head support 136, such as a chin rest or bite element, provides stabilization of the patient's head during image acquisition. A computer 106 has an operator interface 104 and a display 108 for accepting operator commands and for display of volume images of the orthodontia image data obtained by imaging apparatus 100. Computer 106 is in signal communication with sensor 121 for obtaining image data and provides signals for control of source 110 and, optionally, for control of a rotational actuator 112 for mount 130 components. Computer 106 is also in signal communication with a memory 132 for storing image data. An optional alignment apparatus 140 is provided to assist in proper alignment of the patient's head for the imaging process.

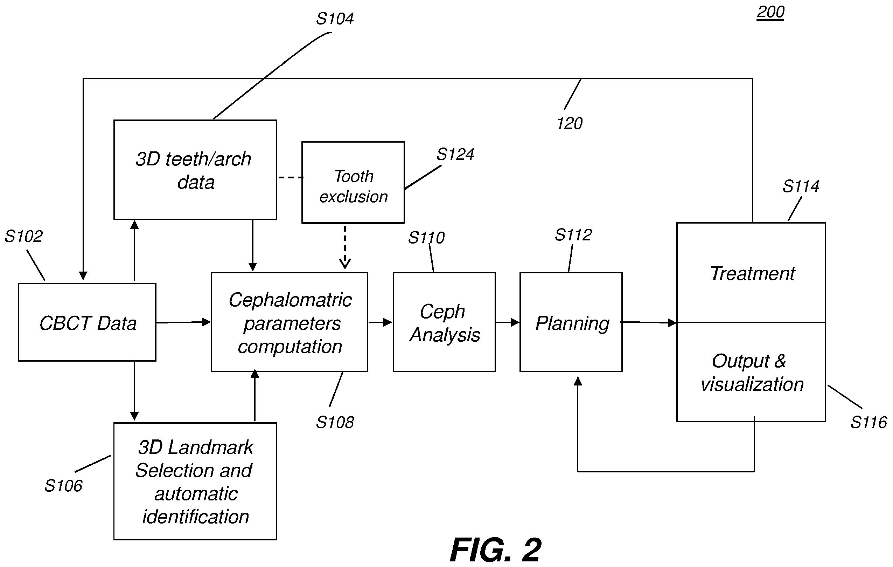

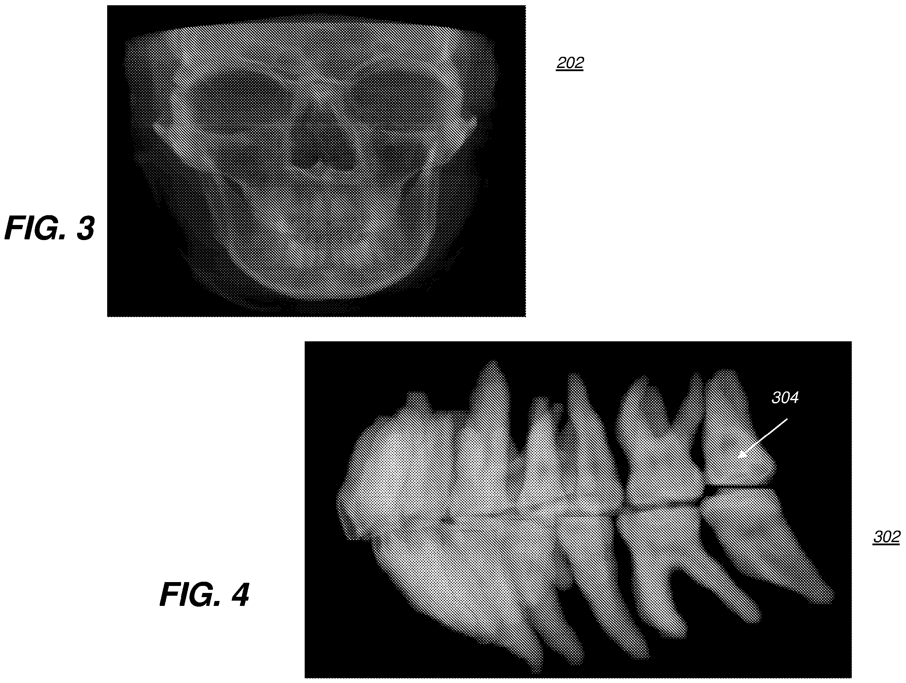

Referring to the logic flow diagram of Figure 2, there is shown a sequence 200 of steps used for acquiring orthodontia data for 3-D cephalometric analysis with a dental CBCT volume according to an embodiment of the present disclosure. The CBCT volume image data is accessed in a data acquisition step S102. A volume contains image data for one or more 2-D images (or equivalently, slices). An original reconstructed CT volume is formed using standard reconstruction algorithms using multiple 2-D projections or sinograms obtained from a CT scanner. By way of example, Figure 3 shows an exemplary dental CBCT volume 202 that contains bony anatomy, soft tissues, and teeth.

Continuing with the sequence of Figure 2, in a segmentation step S104, 3-D dentition element data are collected by applying a 3-D tooth segmentation algorithm to the dental CBCT volume 202. Segmentation algorithms for teeth and related dentition elements are well known in the dental imaging arts. Exemplary tooth segmentation algorithms are described, for example, in commonly assigned U.S. Patent Application Publication No. 2013/0022252 entitled "PANORAMIC IMAGE GENERATION FROM CBCT DENTAL IMAGES" by Chen et al.; in U.S. Patent Application Publication No. 2013/0022255 entitled "METHOD AND SYSTEM FOR TOOTH SEGMENTATION IN DENTAL IMAGES" by Chen et al.; and in U.S. Patent Application Publication No. 2013/0022254 entitled "METHOD FOR TOOTH DISSECTION IN CBCT VOLUME" by Chen.

As is shown in Figure 4, tooth segmentation results are rendered with an image 302, wherein teeth are rendered as a whole but are segmented individually. Each tooth is a separate entity called a tooth volume, for example, tooth volume 304.

Each tooth of the segmented teeth or, more broadly, each dentition element that has been segmented has, at a minimum, a 3-D position list that contains 3-D position coordinates for each of the voxels within the segmented dentition element, and a code value list of each of the voxels within the segmented element. At this point, the 3-D position for each of the voxels is defined with respect to the CBCT volume coordinate system.

In a reference mark selection step S106 in the sequence of Figure 2, the CBCT volume images display with two or more different 2-D views, obtained with respect to different view angles. The different 2-D views can be at different angles and may be different image slices, or may be orthographic or substantially orthographic projections, or may be perspective views, for example. According to an embodiment of the present disclosure, the three views are mutually orthogonal.

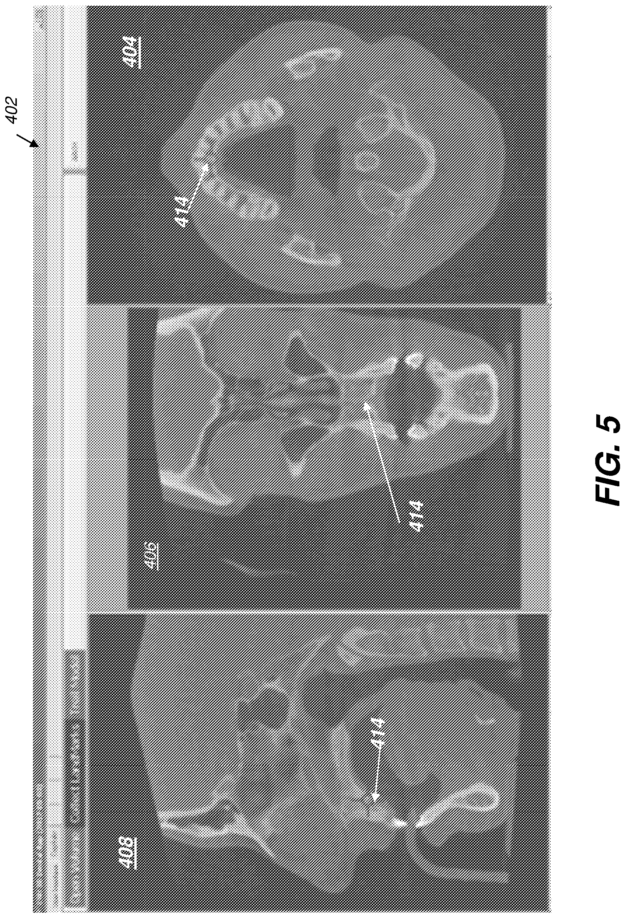

Figure 5 shows an exemplary format with a display interface 402 showing three orthogonal 2-D views. In display interface 402, an image 404 is one of the axial 2-D views of the CBCT volume image 202 (Figure 3), an image 406 is one of the coronal 2-D views of the CBCT volume image 202, and an image 408 is one of the sagittal 2-D views of the CBCT volume image 202. The display interface allows a viewer, such as a practitioner or technician, to interact with the computer system that executes various image processing/computer algorithms in order to accomplish a plurality of 3-D cephalometric analysis tasks. Viewer interaction can take any of a number of forms known to those skilled in the user interface arts, such as using a pointer such as a computer mouse joystick or touchpad, or using a touch screen for selecting an action or specifying a coordinate of the image, for interaction described in more detail subsequently.

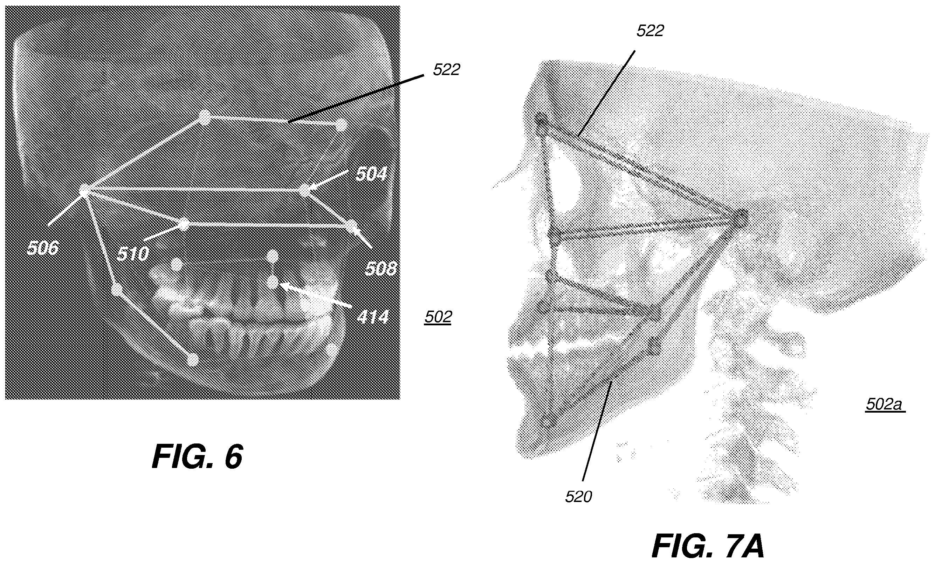

One of the 3-D cephalometric analysis tasks is to perform automatic identification in 3-D reference mark selection step S106 of Figure 2. The 3-D reference marks, equivalent to a type of 3-D landmark or feature point identified by the viewer on the displayed image, are shown in the different mutually orthogonal 2-D views of display interface 402 in Figure 5. Exemplary 3-D anatomic reference marks shown in Figure 5 are lower nasal palatine foramen at reference mark 414. As shown in the view of Figure 6, other anatomic reference marks that can be indicated by the viewer on a displayed image 502 include infraorbital foramina at reference marks 508 and 510, and malleus at reference marks 504 and 506.

In step S106 of Figure 2, the viewer uses a pointing device (such as a mouse or touch screen, for example) to place a reference mark as a type of geometric primitive at an appropriate position in any one of the three views. According to an embodiment of the present disclosure that is shown in figures herein, the reference mark displays as a circle. Using the display interface screen of Figure 5, for example, the viewer places a small circle in the view shown as image 404 at location 414 as the reference mark for a reference point. Reference mark 414 displays as a small circle in image 404 as well as at the proper position in corresponding views in images 406 and 408. It is instructive to note that the viewer need only indicate the location of the reference mark 414 in one of the displayed views 404, 406 or 408; the system responds by showing the same reference mark 414 in other views of the patient anatomy. Thus, the viewer can identify the reference mark 414 in the view in which it is most readily visible.

After entering the reference mark 414, the user can use operator interface tools such as the keyboard or displayed icons in order to adjust the position of the reference mark 414 on any of the displayed views. The viewer also has the option to remove the entered reference mark and enter a new one.

The display interface 402 (Figure 5) provides zoom in/out utilities for re-sizing any or all of the displayed views. The viewer can thus manipulate the different images efficiently for improved reference mark positioning.

The collection of reference marks made with reference to and appearing on views of the 3-D image content, provides a set of cephalometric parameters that can be used for a more precise characterization of the patient's head shape and structure. Cephalometric parameters include coordinate information that is provided directly by the reference mark entry for particular features of the patient's head. Cephalometric parameters also include information on various measurable characteristics of the anatomy of a patient's head that are not directly entered as coordinate or geometric structures but are derived from coordinate information, termed "derived cephalometric parameters". Derived cephalometric parameters can provide information on relative size or volume, symmetry, orientation, shape, movement paths and possible range of movement, axes of inertia, center of mass, and other data. In the context of the present disclosure, the term "cephalometric parameters" applies to those that are either directly identified, such as by the reference marks, or those derived cephalometric parameters that are computed according to the reference marks. For example, as particular reference points are identified by their corresponding reference marks, framework connecting lines 522 are constructed to join the reference points for a suitable characterization of overall features, as is more clearly shown in Figure 6. Framework connecting lines 522 can be considered as vectors in 3-D space; their dimensional and spatial characteristics provide additional volume image data that can be used in computation for orthodontia and other purposes.

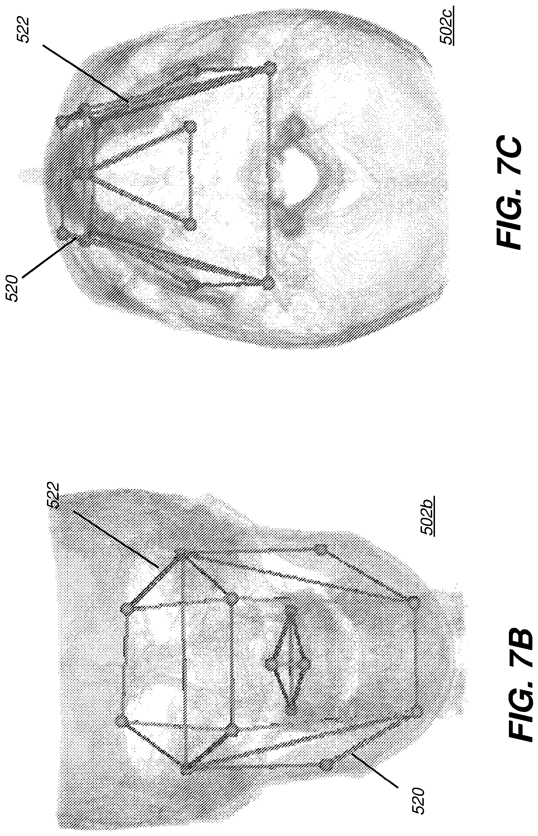

Each reference mark 414, 504, 506, 508, 510 is the terminal point for one or more framework connecting lines 522, generated automatically within the volume data by computer 106 of image processing apparatus 100 and forming a framework that facilitates subsequent analysis and measurement processing. Figures 7A, 7B, and 7C show, for displayed 3-D images 502a, 502b, and 502c from different perspective views, how a framework 520 of selected reference points, with the reference points at the vertices, helps to define dimensional aspects of the overall head structure. According to an embodiment of the present disclosure, an operator instruction allows the operator to toggle between 2-D views similar to those shown in Figure 5 and the volume representation shown in Figure 6, with partial transparency for voxels of the patient's head. This enables the operator to examine reference mark placement and connecting line placement from a number of angles; adjustment of reference mark position can be made on any of the displayed views. In addition, according to an embodiment of the present disclosure, the operator can type in more precise coordinates for a specific reference mark.

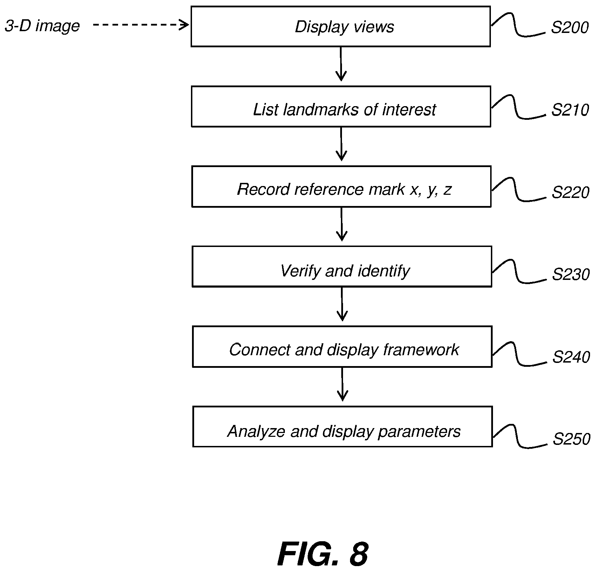

The logic flow diagram of Figure 8 shows steps in a sequence for accepting and processing operator instructions for reference mark entry and identification and for providing computed parameters according to the image data and reference marks. A display step S200 displays one or more 2-D views, from different angles, such as from mutually orthogonal angles, for example, of reconstructed 3-D image data from a computed tomographic scan of a patient's head. In an optional listing step S210, the system provides a text listing such as a tabular list, a series of prompts, or a succession of labeled fields for numeric entry that requires entry of positional data for a number of landmarks or anatomical features in the reconstructed 3-D image. This listing may be explicitly provided for the operator in the form of user interface prompts or menu selection, as described subsequently. Alternately, the listing may be implicitly defined, so that the operator need not follow a specific sequence for entering positional information. Reference marks that give the x, y, z positional data for different anatomical features are entered in a recording step S220. Anatomical features can lie within or outside of the mouth of the patient. Embodiments of the present disclosure can use a combination of anatomical features identified on the display, as entered in step S220, and segmentation data automatically generated for teeth and other dentition elements, as noted previously with reference to Figure 2.

In recording step S220 of Figure 8, the system accepts operator instructions that position a reference mark corresponding to each landmark feature of the anatomy. The reference mark is entered by the operator on either the first or the second 2-D view, or on any of the other views if more than two views are presented and, following entry, displays on each of the displayed views. An identification step S230 identifies the anatomical feature or landmark that corresponds to the entered reference mark and, optionally, verifies the accuracy of the operator entry. Proportional values are calculated to determine the likelihood that a given operator entry accurately identifies the position of a reference mark for a particular anatomical feature. For example, the infraorbital foramen is typically within a certain distance range from the palatine foramen; the system checks the entered distance and notifies the operator if the corresponding reference mark does not appear to be properly positioned.

Continuing with the sequence of Figure 8, in a construction step S240, framework connecting lines are generated to connect reference marks for frame generation. A computation and display step S250 is then executed, computing one or more cephalometric parameters according to the positioned reference marks. The computed parameters are then displayed to the operator.

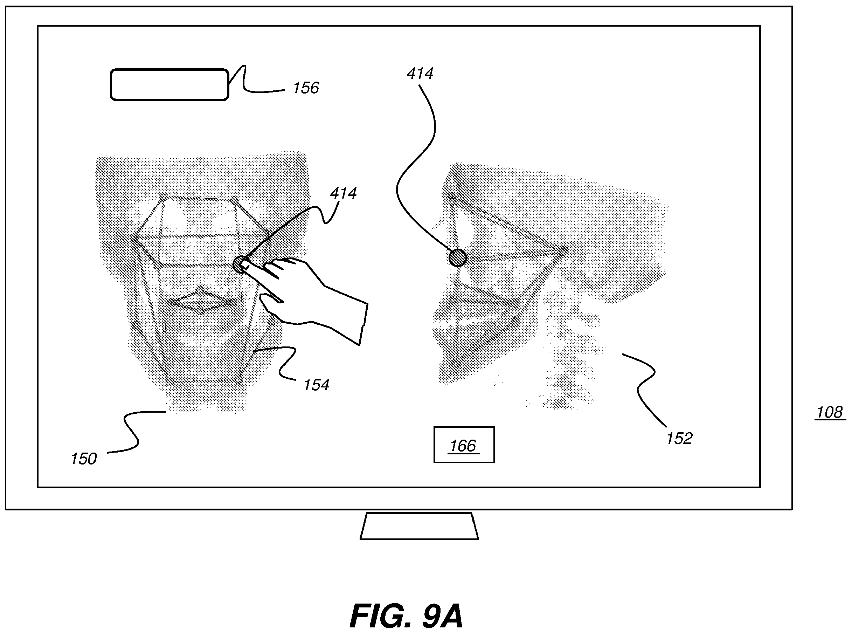

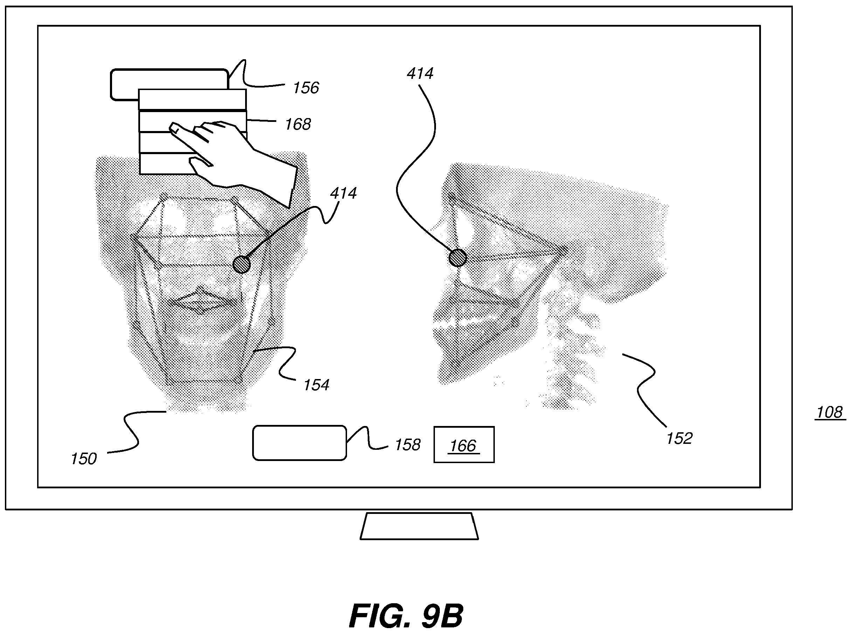

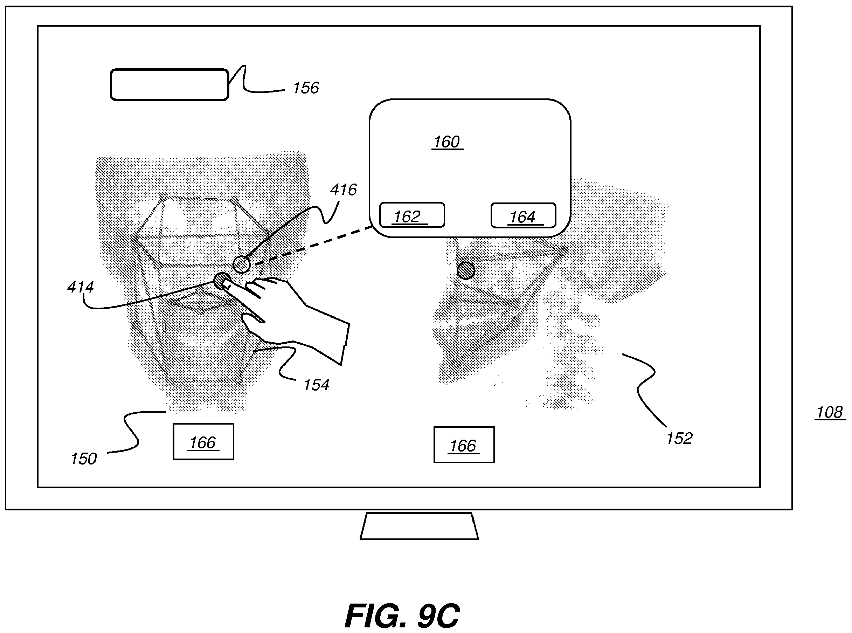

Figures 9A, 9B, and 9C show an operator interface appearing on display 108. The operator interface provides, on display 108, an interactive utility for accepting operator instructions and for displaying computation results for cephalometric parameters of a particular patient. Display 108 can be a touch screen display for entry of operator-specified reference marks and other instructions, for example. Display 108 simultaneously displays at least one 2-D view of the volume image data or two or more 2-D views of the volume image data from different angles or perspectives. By way of example, Figure 9A shows a frontal or coronal view 150 paired with a side or sagittal view 152. More than two views can be shown simultaneously and different 2-D views can be shown, with each of the displayed views independently positioned according to an embodiment of the present disclosure. Views can be mutually orthogonal or may simply be from different angles. As part of the interface of display 108, an optional control 166 enables the viewer to adjust the perspective angle from which one or more of the 2-D views are obtained, either by toggling between alternate fixed views or by changing the relative perspective angle in increments along any of the 3-D axes (x, y, z). A corresponding control 166 can be provided with each 2-D view, as shown in Figure 9-C. Using the operator interface shown for display 108, each reference mark 414 is entered by the operator using a pointer of some type, which may be a mouse or other electronic pointer or may be a touchscreen entry as shown in Figure 9A. As part of the operator interface, an optional listing 156 is provided to either guide the operator to enter a specific reference mark according to a prompt, or to identify the operator entry, such as by selection from a drop-down menu 168 as shown in the example of Figure 9B. Thus, the operator can enter a value in listing 156 or may enter a value in field 158, then select the name associated with the entered value from drop-down menu 168. Figures 9A-9C show a framework 154 constructed between reference points. As Figure 9A shows, each entered reference mark 414 may be shown in both views 150 and 152. A selected reference mark 414 is highlighted on display 108, such as appearing in bold or in another color. A particular reference mark is selected in order to obtain or enter information about the reference mark or to perform some action, such as to shift its position, for example.

In the embodiment shown in Figure 9B, the reference mark 414 just entered or selected by the operator is identified by selection from a listing 156. For the example shown, the operator selects the indicated reference mark 414, then makes a menu selection such as "infraorbital foramen" from menu 168. An optional field 158 identifies the highlighted reference mark 414. Calculations based on a model or based on standard known anatomical relationships can be used to identify reference mark 414, for example.

Figure 9C shows an example in which the operator enters a reference mark 414 instruction that is detected by the system as incorrect or unlikely. An error prompt or error message 160 displays, indicating that the operator entry appears to be in error. The system computes a probable location for a particular landmark or anatomical feature based on a model or based on learned data, for example. When the operator entry appears to be inaccurate, message 160 displays, along with an optional alternate location 416. An override instruction 162 is displayed, along with a repositioning instruction 164 for repositioning the reference mark according to the calculated information from the system. Repositioning can be done by accepting another operator entry from the display screen or keyboard or by accepting the system-computed reference mark location, at alternate location 416 in the example of Figure 9C.

According to an alternate embodiment of the present disclosure, the operator does not need to label reference marks as they are entered. Instead the display prompts the operator to indicate a specific landmark or anatomical feature on any of the displayed 2-D views and automatically labels the indicated feature. In this guided sequence, the operator responds to each system prompt by indicating the position of the corresponding reference mark for the specified landmark.

According to another alternate embodiment of the present disclosure, the system determines which landmark or anatomical feature has been identified as the operator indicates a reference mark; the operator does not need to label reference marks as they are entered. The system computes the most likely reference mark using known information about anatomical features that have already been identified and, alternately, by computation using the dimensions of the reconstructed 3-D image itself.

Using the operator interface shown in the examples of Figures 9A-9C, embodiments of the present disclosure provide a practical 3-D cephalometric analysis system that synergistically integrates the skills of the human operator of the system with the power of the computer in the process of 3-D cephalometric analysis. This takes advantage of human skills of creativity, use of heuristics, flexibility, and judgment, and combines these with computer advantages, such as speed of computation, capability for accurate and repeatable processing, reporting and data access and storage capabilities, and display flexibility.

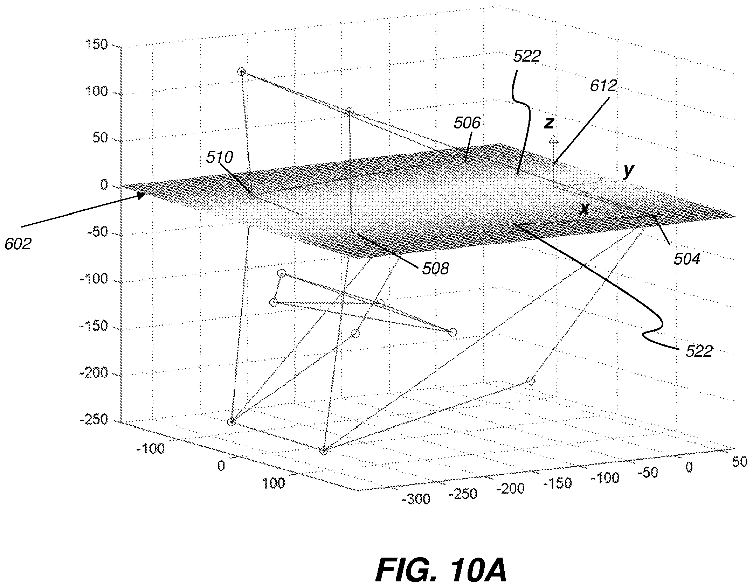

Referring back to the sequence of Figure 2, derived cephalometric parameters are computed in a computation step S108 once a sufficient set of landmarks is entered. Figures 10A through 10E show a processing sequence for computing and analyzing cephalometric data and shows how a number of cephalometric parameters are obtained from combined volume image data and anatomical features information according to operator entered instructions and according to segmentation of the dentition elements. According to an embodiment of the present disclosure, portions of the features shown in Figures 10A through 10E are displayed on display 108 (Figure 1).

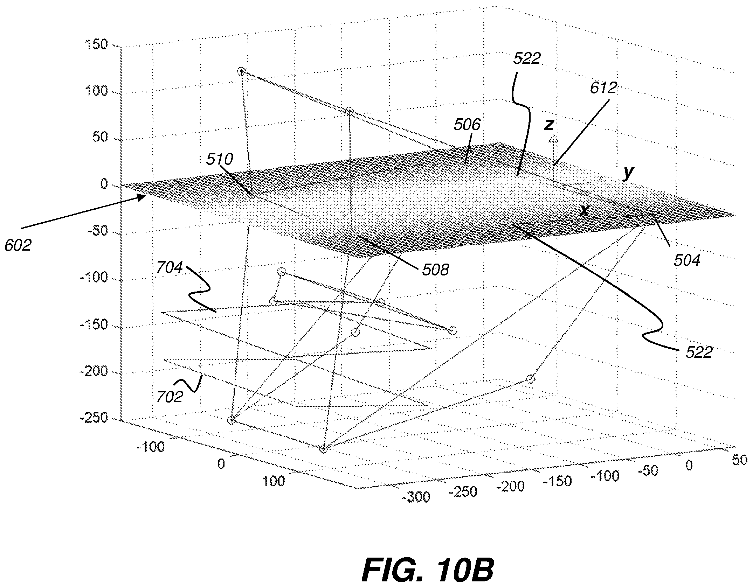

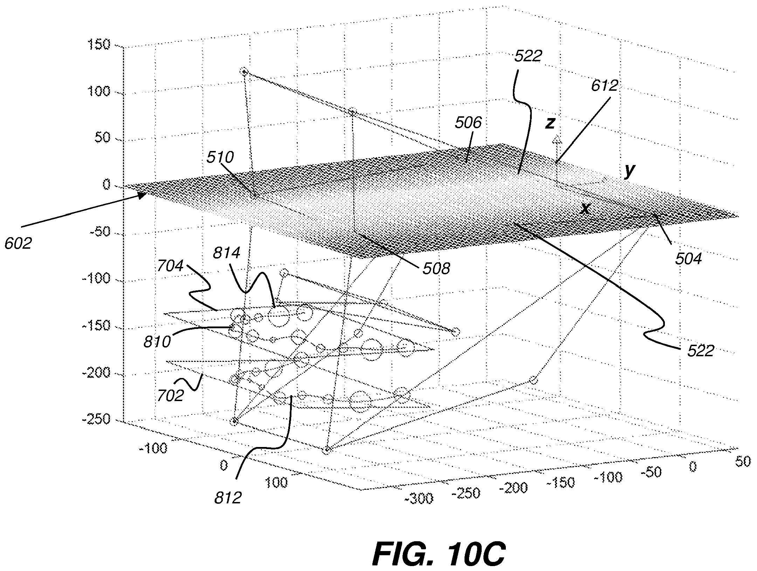

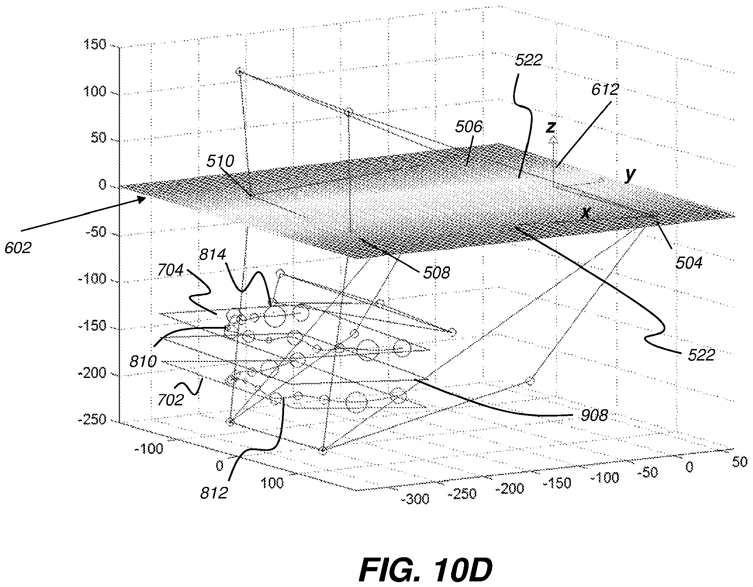

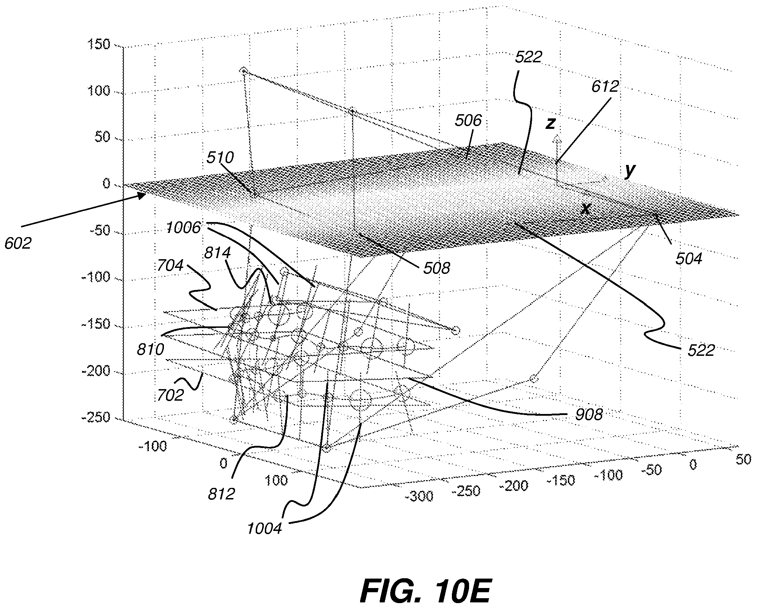

An exemplary derived cephalometric parameter shown in Figure 10A is a 3-D plane 602 (termed a t-reference plane in cephalometric analysis) that is computed by using a subset of the set of first geometric primitives with reference marks 504, 506, 508 and 510 as previously described with reference to Figure 6. A further derived cephalometric parameter is 3-D coordinate reference system 612 termed a t-reference system and described by Treil in publications noted previously. The z axis of the t-reference system 612 is chosen as perpendicular to the 3-D t-reference plane 602. The y axis of the t-reference system 612 is aligned with framework connecting line 522 between reference marks 508 and 504. The x axis of the t-reference system 612 is in plane 602 and is orthogonal to both z and x axes of the t-reference system. The directions of t-reference system axes are indicated in Figure 10A and in subsequent Figures 10B, 10C, 10D, and 10E. The origin of the t-reference system is at the middle of framework connecting line 522 that connects reference marks 504 and 506.

With the establishment of t-reference system 612, 3-D reference marks from step S106 and 3-D teeth data (3-D position list of a tooth) from step S104 are transformed from the CBCT volume coordinate system to t-reference system 612. With this transformation, subsequent computations of derived cephalometric parameters and analyses can now be performed with respect to t-reference system 612.

Referring to Figure 10B, a 3-D upper jaw plane 704 and a 3-D lower jaw plane 702 can be derived from cephalometric parameters from the teeth data in t-reference system 612. The derived upper jaw plane 704 is computed according to teeth data segmented from the upper jaw (maxilla). Using methods familiar to those skilled in cephalometric measurement and analysis, derived lower jaw plane 702 is similarly computed according to the teeth data segmented from the lower jaw (mandibular).

For an exemplary computation of a 3-D plane from the teeth data, an inertia tensor is formed by using the 3-D position vectors and code values of voxels of all teeth in a jaw (as described in the cited publications by Treil); eigenvectors are then computed from the inertia tensor. These eigenvectors mathematically describe the orientation of the jaw in the t-reference system 612. A 3-D plane can be formed using two of the eigenvectors, or using one of the eigenvectors as the plane normal.

Referring to Figure 10C, further derived parameters are shown. For each jaw, jaw curves are computed as derived parameters. An upper jaw curve 810 is computed for the upper jaw; a lower jaw curve 812 is derived for the lower jaw. The jaw curve is constructed to intersect with the mass center of each tooth in the respective jaw and to lie in the corresponding jaw plane. The mass center of the tooth can be calculated, in turn, using the 3-D position list and the code value list for the segmented teeth.

The mass of a tooth is also a derived cephalometric parameter computed from the code value list of a tooth. In Figure 10C, an exemplary tooth mass is displayed as a circle 814 or other type of shape for an upper jaw tooth. According to an embodiment of the present disclosure, one or more of the relative dimensions of the shape, such as the circle radius, for example, indicates relative mass value, the mass value of the particular tooth in relation to the mass of other teeth in the jaw. For example, the first molar of the upper jaw has a mass value larger than the neighboring teeth mass values.

According to an embodiment of the present disclosure, for each tooth, an eigenvector system is also computed. An inertia tensor is initially formed by using the 3-D position vectors and code values of voxels of a tooth, as described in the cited publications by Treil. Eigenvectors are then computed as derived cephalometric parameters from the inertia tensor. These eigenvectors mathematically describe the orientation of a tooth in the t-reference system.

As shown in Figure 10D, another derived parameter, an occlusal plane, 3-D plane 908, is computed from the two jaw planes 702 and 704. Occlusal plane, 3-D plane 908, lies between the two jaw planes 702 and 704. The normal of plane 908 is the average of the normal of plane 702 and normal of plane 704.

For an individual tooth, in general, the eigenvector corresponding to the largest computed eigenvalue is another derived cephalometric parameter that indicates the medial axis of the tooth. Figure 10E shows two types of exemplary medial axes for teeth: medial axes 1006 for upper incisors and medial axes 1004 for lower incisors.

The calculated length of the medial axis of a tooth is a useful cephalometric parameter in cephalometric analysis and treatment planning along with other derived parameters. It should be noted that, instead of using the eigenvalue to set the length of the axis as proposed in the cited publication by Triel, embodiments of the present disclosure compute the actual medial axis length as a derived parameter using a different approach. A first intersection point of the medial axis with the bottom slice of the tooth volume is initially located. Then, a second intersection point of the medial axis with the top slice of the tooth volume is identified. An embodiment of the present disclosure then computes the length between the two intersection points.

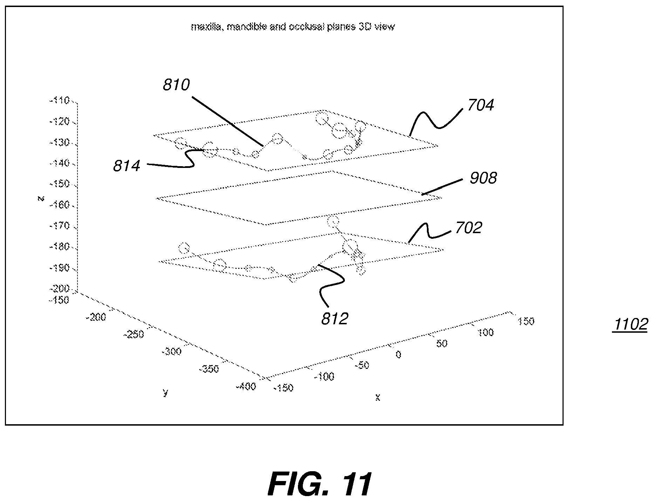

Figure 11 shows a graph 1102 that provides a closeup view that isolates the occlusal plane 908 in relation to upper jaw plane 704 and lower jaw plane 702 and shows the relative positions and curvature of jaw curves 810 and 812.

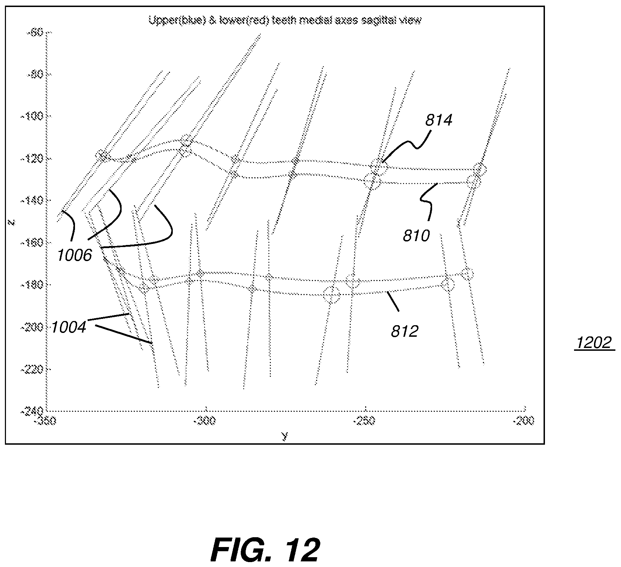

Figure 12 shows a graph 1202 that shows the positional and angular relationships between the upper teeth medial axes 1006 and the lower teeth medial axes 1004.

As noted in the preceding descriptions and shown in the corresponding figures, there are a number of cephalometric parameters that can be derived from the combined volume image data, including dentition element segmentation, and operator-entered reference marks. These are computed in a computer-aided cephalometric analysis step S110 (Figure 2).

One exemplary 3-D cephalometric analysis procedure in step S110 that can be particularly valuable relates to the relative parallelism of the maxilla (upper jaw) and mandibular (lower jaw) planes 702 and 704.. Both upper and lower jaw planes 702 and 704, respectively, are derived parameters, as noted previously. The assessment can be done using the following sequence:

- Project the x axis of the maxilla inertia system (that is, the eigenvectors) to the x-z plane of the t-reference system and compute an angle MX1_RF between the z axis of the t-reference system and the projection;
- Project the x axis of the mandibular inertia system (that is, the eigenvectors) to the x-z plane of the t-reference system and compute an angle MD1_RF between the z axis of the t-reference system and the projection;

- MX1_MD1_RF = MX1_RF - MD1_RF gives a parallelism assessment of upper and lower jaws in the x-z plane of the t-reference system;
- Project the y axis of the maxilla inertia system (that is, the eigenvectors) to the y-z plane of the t-reference system and compute the angle MX2_RS between the y axis of the t-reference system and the projection;
- Project the y axis of the mandibular inertia system (that is, the eigenvectors) to the y-z plane of the t-reference system and compute an angle MD2_RS between the y axis of the t-reference system and the projection;
- MX2_MD2_RS = MX2_RS - MD2_RS gives a parallelism assessment of upper and lower jaws in the y-z plane of the t-reference system.

Another exemplary 3-D cephalometric analysis procedure that is executed in step S110 is assessing the angular property between the maxilla (upper jaw) incisor and mandible (lower jaw) incisor using medial axes 1006 and 1004 (Figures 10E, 12). The assessment can be done using the following sequence:

- Project the upper incisor medial axis 1006 to the x-z plane of the t-reference system and compute an angle MX1_AF between the z axis of the t-reference system and the projection;
- Project the lower incisor medial axis 1004 to the x-z plane of the t-reference system and compute an angle MD1_AF between the z axis of the t-reference system and the projection;
- MX1_MD1_AF = MX1_AF - MD1_AF gives the angular property assessment of the upper and lower incisors in the x-z plane of the t-reference system;
- Project the upper incisor medial axis 1006 to the y-z plane of the t-reference system and compute an angle MX2_AS between the y axis of the t-reference system and the projection;
- Project the lower incisor medial axis 1004 to the y-z plane of the t-reference system and compute an angle MD2_AS between the y axis of the t-reference system and the projection;
- MX2_MD2_AS = MX2_AS - MD2_AS gives the angular property assessment of upper and lower incisors in the y-z plane of the t-reference system.

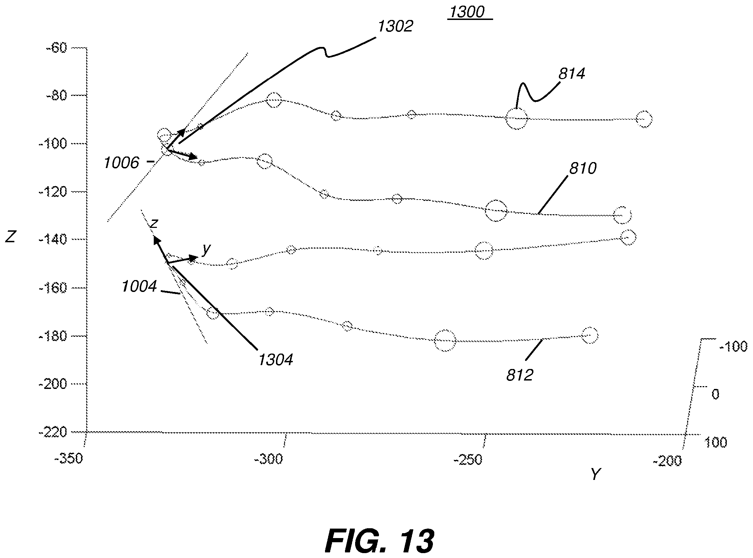

Figure 13 shows a graph 1300 that shows a local x-y-z coordinate system 1302 for an upper incisor, and a local x-y-z coordinate system 1304 for a lower incisor. The local axes of the x-y-z coordinate system align with the eigenvectors associated with that particular tooth. The x axis is not shown but satisfies the right-hand system rule.

In Figure 13, the origin of system 1302 can be selected at any place along axis 1006. An exemplary origin for system 1302 is the mass center of the tooth that is associated with axis 1006. Similarly, the origin of system 1304 can be selected at any place along axis 1004. An exemplary origin for system 1304 is the mass center of the tooth that is associated with axis 1004.

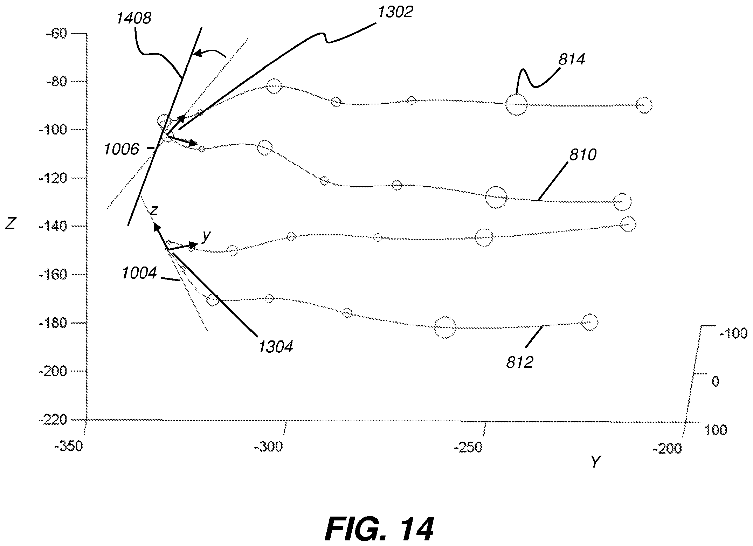

Based on the analysis performed in Step S110 (Figure 2), an adjustment or treatment plan is arranged in a planning step S112. An exemplary treatment plan is to rotate the upper incisor counter clockwise at a 3-D point, such as at its local coordinate system origin, and about an arbitrary 3-D axis, such as about the x axis of the local x-y-z system. The graph of Figure 14 shows rotation to an axis position 1408.

In a treatment step S 114 of Figure 2, treatment is performed based on the planning, for example, based on upper incisor rotation. The treatment planning can be tested and verified visually in a visualization step S 116 before the actual treatment takes place.

Referring back to Figure 2, there is shown a line 120 from Step S114 to Step S102. This indicates that there is a feedback loop in the sequence 200 workflow. After the patient undergoes treatment, an immediate evaluation or, alternately, a scheduled evaluation of the treatment can be performed by entering relevant data as input to the system. Exemplary relevant data for this purpose can include results from optical, radiographic, MRI, or ultrasound imaging and/or any meaningful related measurements or results.

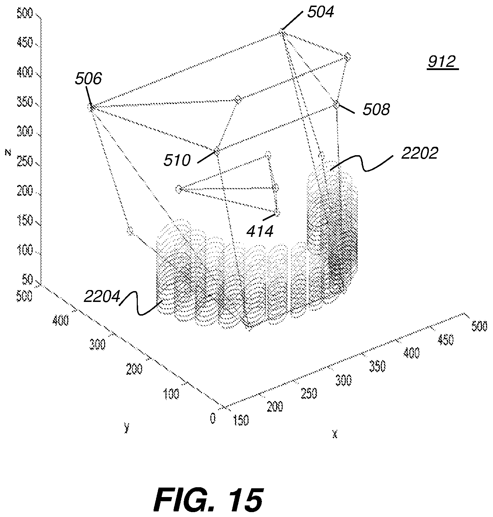

An optional tooth exclusion step S 124 is also shown in sequence 200 of Figure 2. For example, if the patient has had one or more teeth removed, then the teeth that complement the removed teeth can be excluded. For this step, the operator specifies one or more teeth, if any, to be excluded from the rest of the processing steps based on Treil's theory of jaw planes parallelism. The graph of Figure 15 shows how tooth exclusion can be learned by the system, using a virtual or digital phantom 912. Digital phantom 912 is a virtual model used for computation and display that is constructed using a set of landmarks and a set of upper teeth of a digital model of an upper jaw and a set of lower teeth of a digital model of a lower jaw. Digital phantom 912 is a 3-D or volume image data model that is representative of image data that is obtained from patient anatomy and is generated using the landmark and other anatomical information provided and can be stored for reference or may be generated for use as needed. The use of various types of digital phantom is well known to those skilled in the digital radiography arts. The landmarks such as reference marks 504, 506, 508 and 510 of the digital phantom 912 correspond to the actual reference marks identified from the CBCT volume 202 (Figure 3). These landmarks are used to compute the t-reference system 612 (Figures 10A-10E).

The operator can exclude one or more teeth by selecting the teeth from a display or by entering information that identifies the excluded teeth on the display.

In the Figure 15 representation, the upper and lower teeth, such as digital teeth 2202 and 2204 of digital phantom 912 are digitally generated. The exemplary shape of a digital tooth is a cylinder, as shown. The exemplary voxel value for a digital tooth in this example is 255. It can be appreciated that other shapes and values can be used for phantom 912 representation and processing.

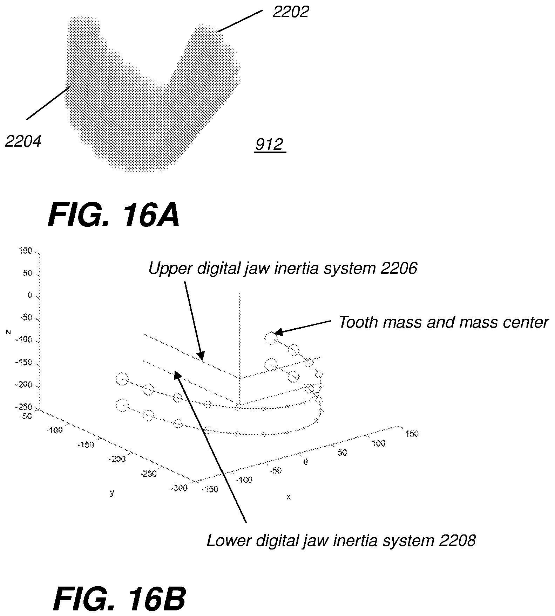

Figure 16A shows digital teeth 2202 and 2204 of digital phantom 912. The corresponding digital teeth in the upper digital jaw and lower digital jaw are generated in a same way, with the same size and same code value.

To assess parallelism of the upper and lower digital jaws, an inertia tensor for each digital jaw is formed by using the 3-D position vectors and code values of voxels of all digital teeth in a digital jaw (see the Treil publications, cited previously). Eigenvectors are then computed from the inertia tensor. These eigenvectors, as an inertial system, mathematically describe the orientation of the jaw in the t-reference system 612 (Figure 10A). As noted earlier, the eigenvectors, computed from the inertial tensor data, are one type of derived cephalometric parameter.

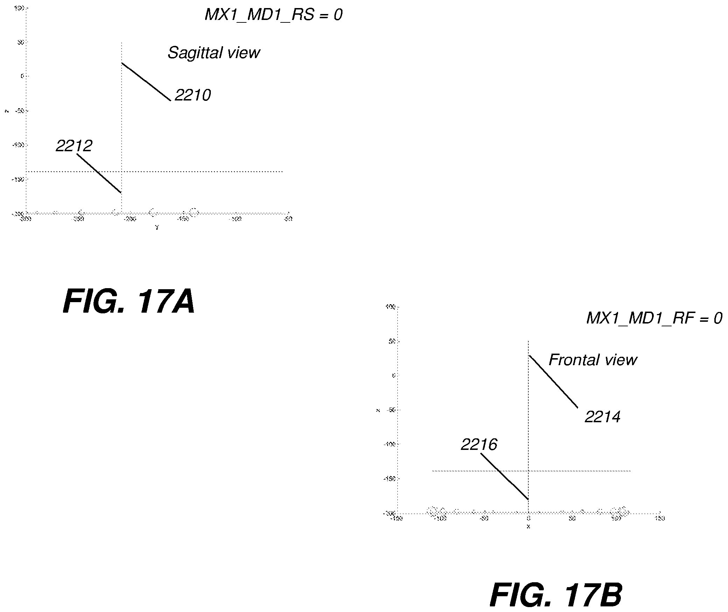

As shown in Figure 16B, the computed axes of an upper digital jaw inertia system 2206 and a lower digital jaw inertia system 2208 are in parallel for the generated digital phantom 912 as expected, since the upper and lower jaw teeth are created in the same way. Figure 17A shows this parallelism in the sagittal view along a line 2210 for the upper jaw and along a line 2212 for the lower jaw; Figure 17B shows parallelism in the frontal (coronal) view at a line 2214 for the upper jaw and at a line 2216 for the lower jaw.

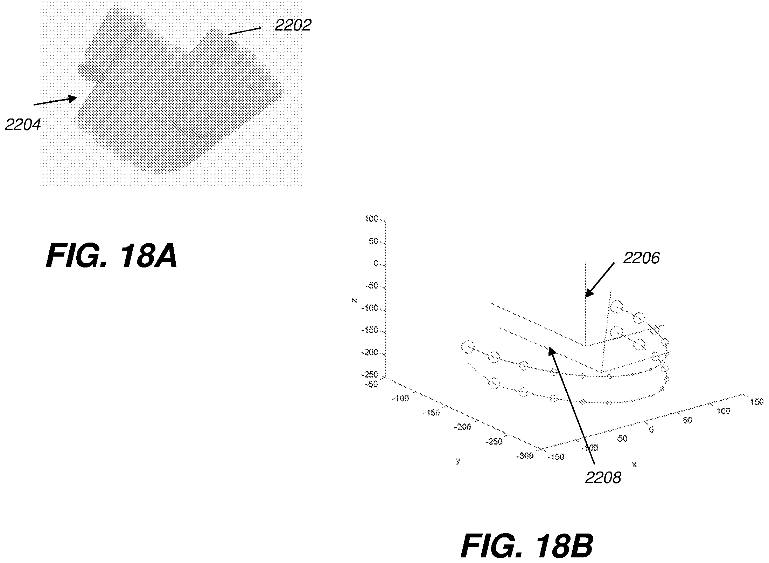

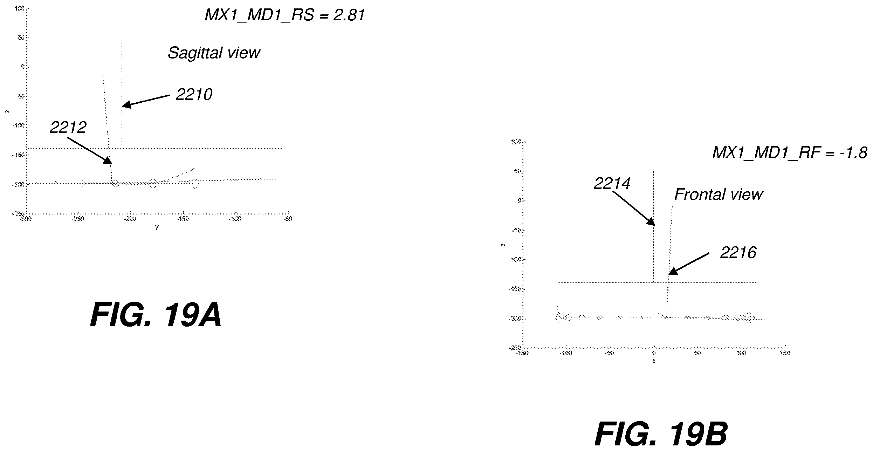

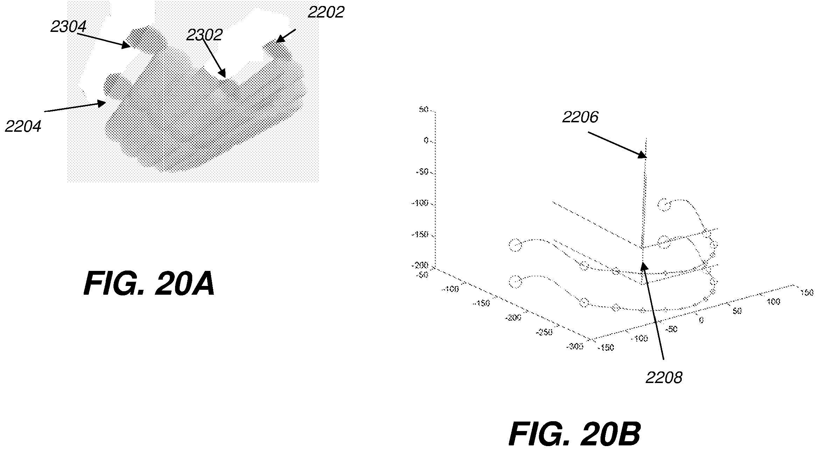

Referring to Figures 18A and 18B, there is shown a case in which digital tooth 2204 is missing. The computed axes of upper digital jaw inertia system 2206 and lower digital jaw inertia system 2208 are no longer in parallel. In corresponding Figures 19A and 19B, this misalignment can also be examined in a sagittal view along a line 2210 for the upper jaw and a line 2212 for the lower jaw; in the frontal view along a line 2214 for the upper jaw and a line 2216 for the lower jaw. According to an embodiment of the present disclosure, this type of misalignment of upper and lower jaw planes (inertia system) due to one or more missing teeth can be corrected by excluding companion teeth of each missing tooth as illustrated in Figures 20A and 20B. The companion teeth for tooth 2204 are teeth 2304, 2302 and 2202. Tooth 2304 is the corresponding tooth in the upper jaw for tooth 2204. Teeth 2202 and 2302 are the corresponding teeth at the other side for the teeth 2304 and 2204. After excluding the companion teeth for the missing tooth 2204, the computed axes of inertia system 2206 for the upper jaw and inertia system 2208 for the lower jaw are back in parallel.

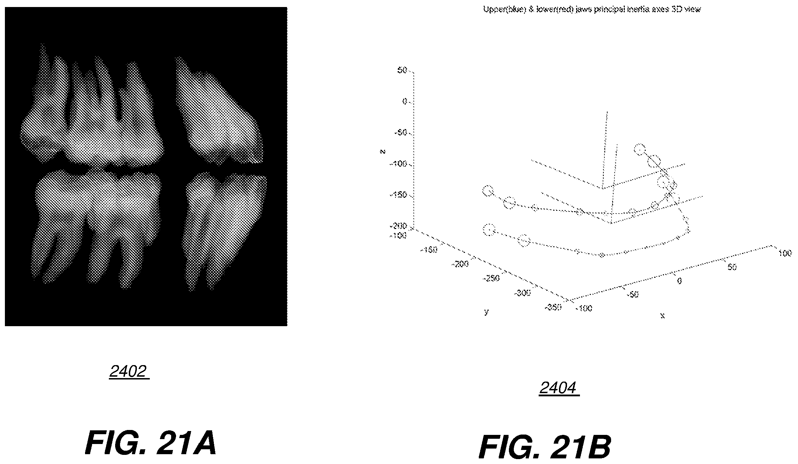

Figures 21A and 21B illustrate segmented teeth from a CBCT volume in a case where companion teeth are excluded for a missing tooth. The segmentation results are shown in an image 2402. The computed axes of inertia systems for the upper and lower jaws are in parallel as demonstrated in a graph 2404.

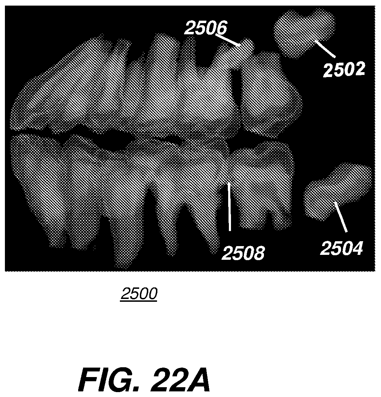

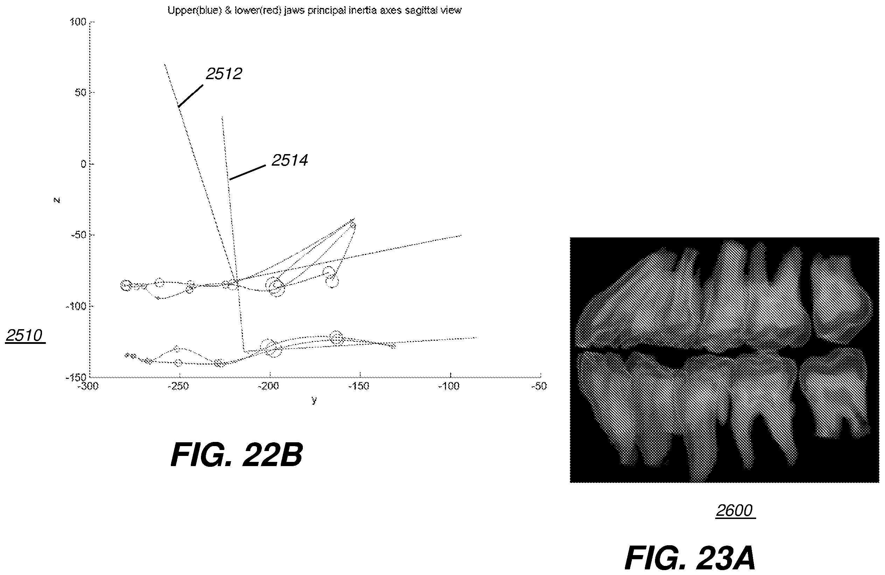

Figures 22A and 22B show the method of exclusion of companion teeth applied to another patient using tooth exclusion step S124 (Figure 2). As shown in an image 2500, teeth 2502, 2504, 2506 and 2508 are not fully developed. Their positioning, size, and orientation severely distort the physical properties of the upper jaw and lower jaw in terms of inertia system computation. A graph 2510 in Figure 22B depicts the situation where upper jaw inertia system 2512 and lower jaw inertia system 2514 are severely misaligned (not in parallel).

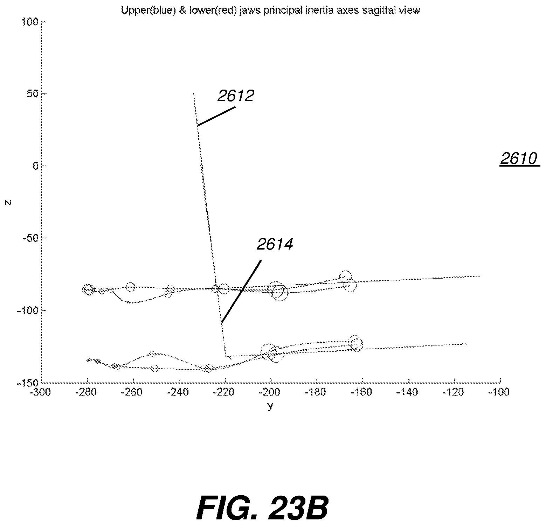

Figures 23A and 23B show the results of excluding specific teeth from the image. An image 2600 shows the results of excluding teeth 2502, 2504, 2506 and 2508 from image 2500 of Figure 22A. Without the disturbance of these teeth, the axes of inertia system 2612 of the upper jaw and inertia system 2614 lower jaw of the teeth shown in image 2600 are in parallel as depicted in a graph 2610.

Biometry Computation

Given the entered landmark data for anatomic reference points, segmentation of dentition elements such as teeth, implants, and jaws and related support structures, and the computed parameters obtained as described previously, detailed biometry computation can be performed and its results used to assist setup of a treatment plan and monitoring ongoing treatment progress. Referring back to Figure 8, the biometry computation described subsequently gives more details about step S250 for analyzing and displaying parameters generated from the recorded reference marks.

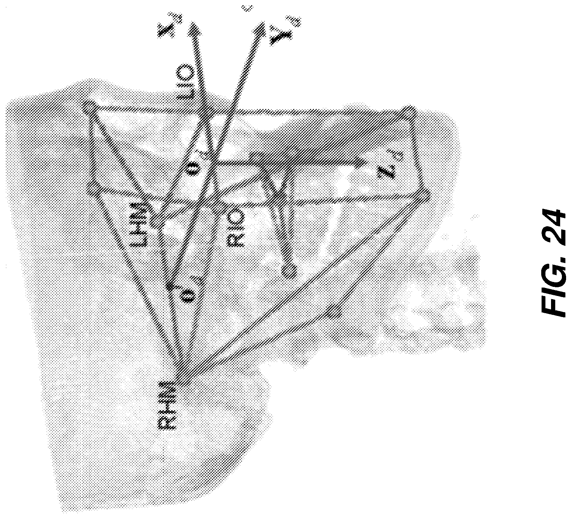

According to an embodiment of the present disclosure, the entered landmarks and computed inertia systems of teeth are transformed from the original CBCT image voxel space to an alternate reference system, termed the direct orthogonal landmark (DOL) reference system, with coordinates $(x_d, y_d, z_d)$. Figure 24 shows a number of landmarks and coordinate axes or vectors of the DOL reference system. Landmarks RIO and LIO indicate the infraorbital foramen; landmarks RHM and LHM mark the malleus. The origin $\mathbf{o}_d$ of $(\mathbf{x}_d, \mathbf{y}_d, \mathbf{z}_d)$ is selected at the middle of the line connecting landmarks RIO and LIO. Vector $\mathbf{x}_d$ direction is defined from landmark RIO to LIO. A YZ plane is orthogonal to vector $\mathbf{x}_d$ at point $\mathbf{o}_d$. There is an intersection point $\mathbf{o'}_d$ of plane YZ and the line connecting RHM and LHM. Vector $\mathbf{y}_d$ direction is from $\mathbf{o'}_d$ to $\mathbf{o}_d$. Vector $\mathbf{z}_d$ is the cross product of $\mathbf{x}_d$ and $\mathbf{y}_d$.

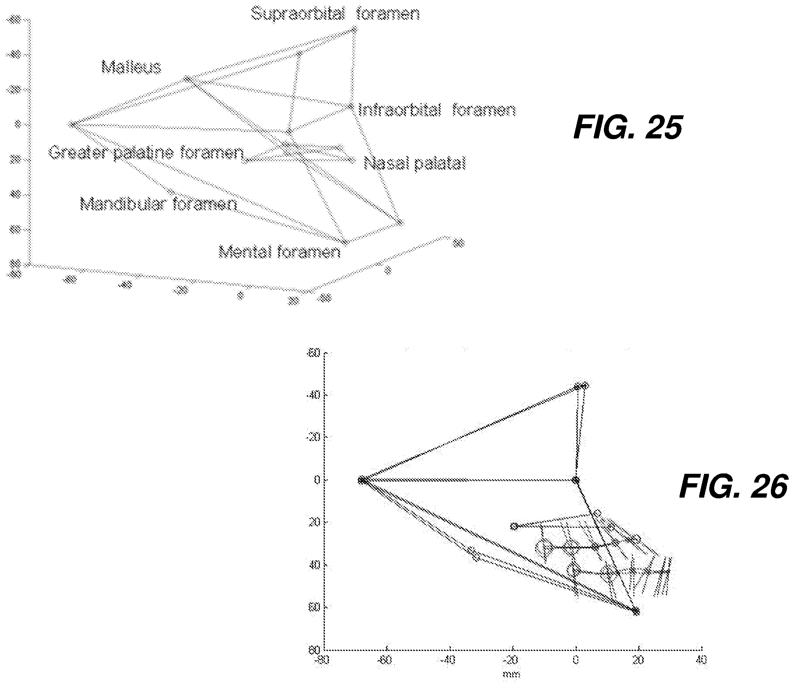

Using this transformation, the identified landmarks can be re-mapped to the coordinate space shown in Figure 25. Figure 26 shows, from a side view, an example with transformed inertia systems using this remapping.

By way of example, and not of limitation, the following listing identifies a number of individual data parameters that can be calculated and used for further analysis using the transformed landmark, dentition segmentation, and inertial system data.

A first grouping of data parameters that can be calculated using landmarks in the transformed space gives antero-posterior values:

1. Antero-posterior.alveolar.GIM-Gim: y position difference between the mean centers of inertia of upper and lower incisors.
2. Antero-posterior.alveolar.GM-Gm: difference between the mean centers of inertia of upper and lower teeth.
3. Antero-posterior.alveolar.TqIM: mean torque of upper incisors.
4. Antero-posterior.alveolar.Tqim: mean torque of lower incisors.
5. Antero-posterior.alveolar.(GIM+Gim)/2: average y position of GIM and Gim.
6. Antero-posterior.basis.MNP-MM: y position difference between mean nasal palatal and mean mental foramen.
7. Antero-posterior.basis.MFM-MM: actual distance between mean mandibular foramen and mean mental foramen.
8. Antero-posterior.architecture.MMy: y position of mean mental foramen.

9. Antero-posterior.architecture.MHM-MM: actual distance between mean malleus and mean mental foramen.

A second grouping gives vertical values:

10. Vertical.alveolar.Gdz: z position of inertial center of all teeth.

11. Vertical.alveolar.MxlI-MdlI: difference between the angles of second axes of upper and lower arches.

12. Vertical.basis.<MHM-MIO,MFM-MM>: angle difference between the vectors MHM-MIO and MFM-MM.

13. Vertical. architecture.MMz: z position of mean mental foramen.

14. Vertical. architecture.13: angle difference between the vectors MHM-MIO and MHM-MM.

Transverse values are also provided:

15. Transverse.alveolar.dM-dm: difference between upper right/left molars distance and lower right/left molars distance

16. Transverse.alveolar.TqM-Tqm: difference between torque of upper $1^{st}$ & $2^{nd}$ molars and torque of lower $1^{st}$ & $2^{nd}$ molars.

17. Transverse.basis.(RGP-LGP)/(RFM-LFM): ratio of right/left greater palatine distance and mandibular foramen distance.

18. Transverse.architecture.(RIO-LIO)/(RM-LM): ratio of right/left infraorbital foramen and mental foramen distances.

Other calculated or "deduced" values are given as follows:

19. Deduced.hidden.GIM: mean upper incisors y position.

20. Deduced.hidden.Gim: mean lower incisors y position.

21. Deduced.hidden.(TqIM+Tqim)/2: average of mean torque of upper incisors and mean torque of lower incisors.

22. Deduced.hidden.TqIM-Tqim: difference of mean torque of upper incisors and mean torque of lower incisors.

23. Deduced.hidden.MNPy: mean nasal palatal y position.

24. Deduced.hidden.GIM-MNP(y): difference of mean upper incisors y position and mean nasal palatal y position.

25. Deduced.hidden.Gim-MM(y): mean mental foramen y position.

26. Deduced.hidden.Gdz/(MMz-Gdz): ratio between value of Gdz and value of MMz-Gdz.

It should be noted that this listing is exemplary and can be enlarged, edited, or changed in some other way within the scope of the present disclosure.

In the exemplary listing given above, there are 9 parameters in the anterior-posterior category, 5 parameters in the vertical category and 4 parameters in the transverse category. Each of the above categories, in turn, has three types: alveolar, basis, and architectural. Additionally, there are 8 deduced parameters that may not represent a particular spatial position or relationship but that are used in subsequent computation. These parameters can be further labeled as normal or abnormal.

Normal parameters have a positive relationship with anterior-posterior disharmony, that is, in terms of their values:

Class III < Class I < Class II.

wherein Class I values indicate a normal relationship between the upper teeth, lower teeth and jaws or balanced bite; Class II values indicate that the lower first molar is posterior with respect to the upper first molar; Class III values indicate that the lower first molar is anterior with respect to the upper first molar.

Abnormal parameters have a negative relationship with anterior-posterior disharmony, that is, in terms of their bite-related values:

Class II < Class I < Class III.

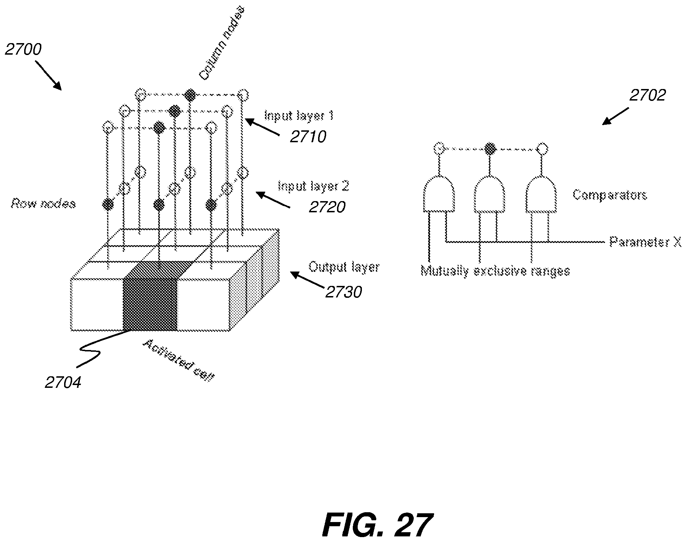

Embodiments of the present disclosure can use an analysis engine in order to compute sets of probable conditions that can be used for interpretation and as guides to treatment planning. Figures 27 - 38 show various aspects of analysis engine operation and organization and some of the text, tabular, and graphical results generated by the analysis engine. It should be noted that a computer, workstation, or host processor can be configured as an analysis engine according to a set of preprogrammed instructions that accomplish the requisite tasks and functions.

According to an embodiment of the present disclosure, an analysis engine can be modeled as a three-layer network 2700 as shown in Figure 27. In this model, row and column node inputs can be considered to be directed to a set of comparators 2702 that provide a binary output based on the row and column input signals. One output cell 2704 is activated for each set of possible input conditions, as shown. In the example shown, an input layer 1 2710 is fed with one of the 26 parameters listed previously and an input layer 2 2720 is fed with another one of the 26 parameters. An output layer 2730 contains 9 cells each one of which represents one probable analysis if the two inputs meet certain criterion, that is, when their values are within particular ranges.

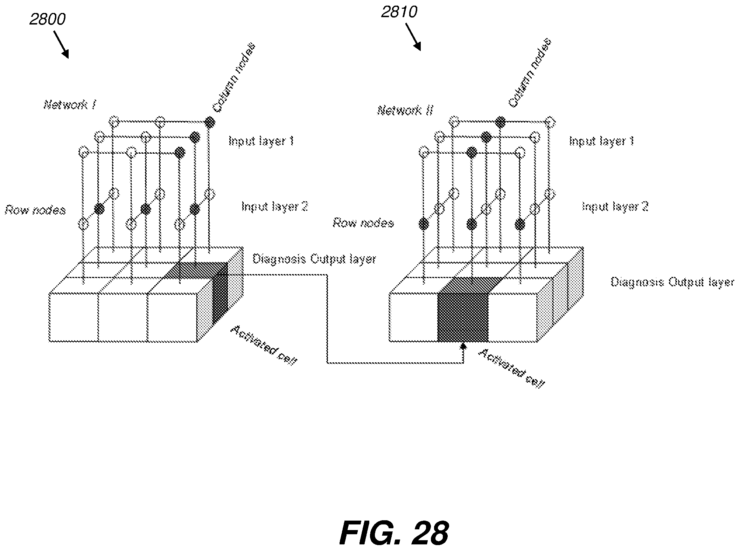

According to an embodiment of the present disclosure, the analysis engine has thirteen networks. These include independent networks similar to that shown in Figure 27 and coupled networks 2800 and 2810 as shown in Figure 28.

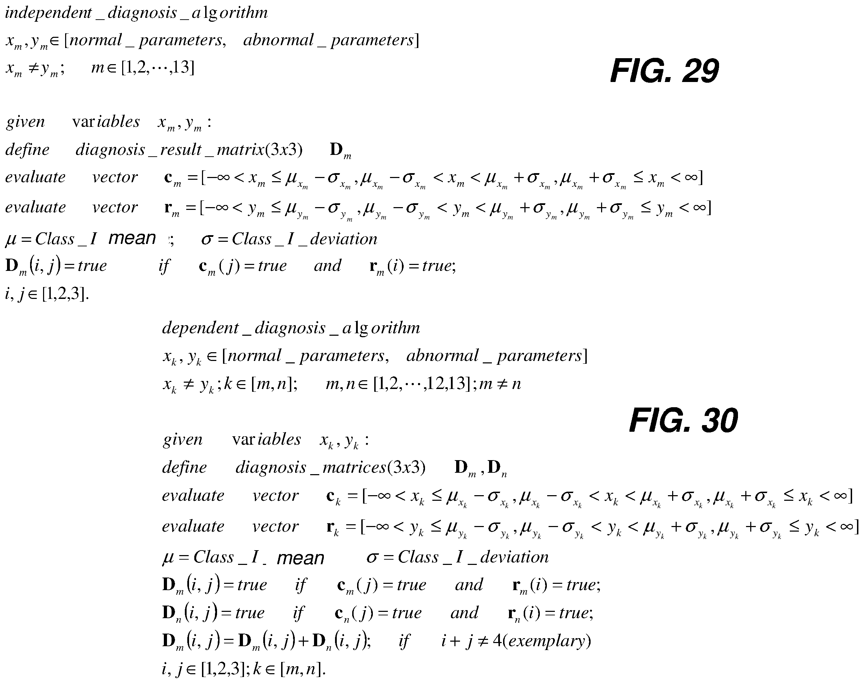

An algorithm shown in Figure 29 describes the operation of an independent analysis network, such as that shown in the example of Figure 27. Here, values $x$ and y are the input parameter values; $m$ represents the

network index; $\mathbf{D}(i,j)$ is the output cell. The steps of "evaluate vector $\mathbf{c}_m$" for column values and "evaluate vector $\mathbf{r}_m$" for row values check to determine what evaluation criterion the input values meet. For example, in the following formula, if $-\infty < x_m \leq \mu_{xm}$ then $\mathbf{c}_m =$ [true, false, false].

The coupled network of Figure 28 combines results from two other networks and can operate as described by the algorithm in Figure 30. Again, values $x$ and $y$ are the input values; $m$ represents the network index; $\mathbf{D}(i,j)$ is the output cell. The steps of "evaluate vector $\mathbf{c}_k$" for column values and "evaluate vector $\mathbf{r}_k$" for row values check to determine what evaluation criterion the input values meet.

In a broader aspect, the overall arrangement of networks using the independent network model described with reference to Figure 27 or the coupled network model described with reference to Figure 28 allow analysis to examine, compare, and combine various metrics in order to provide useful results that can be reported to the practitioner and used for treatment planning.

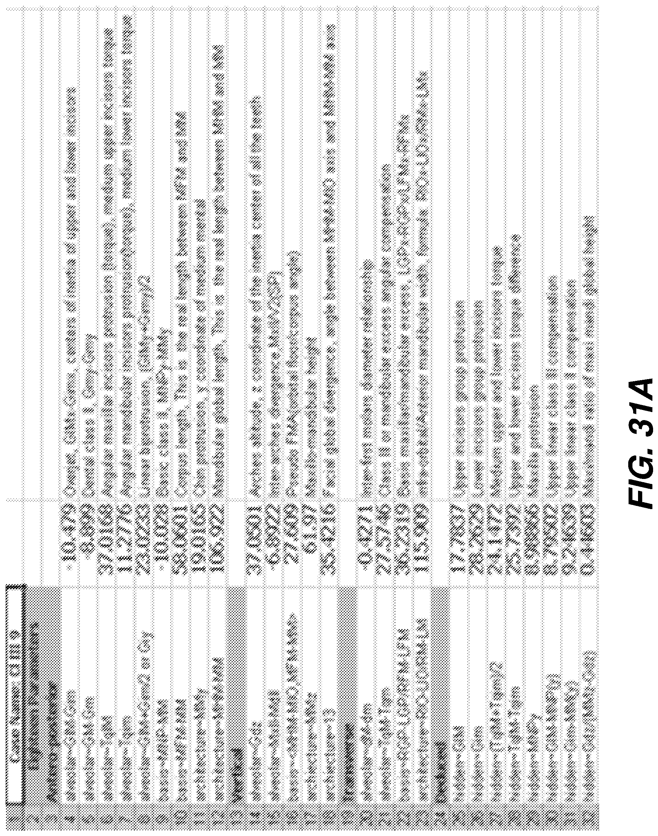

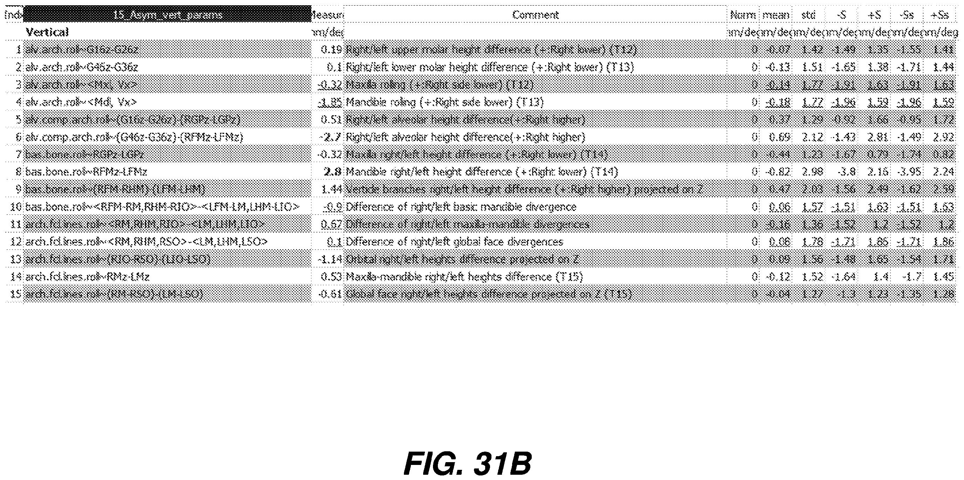

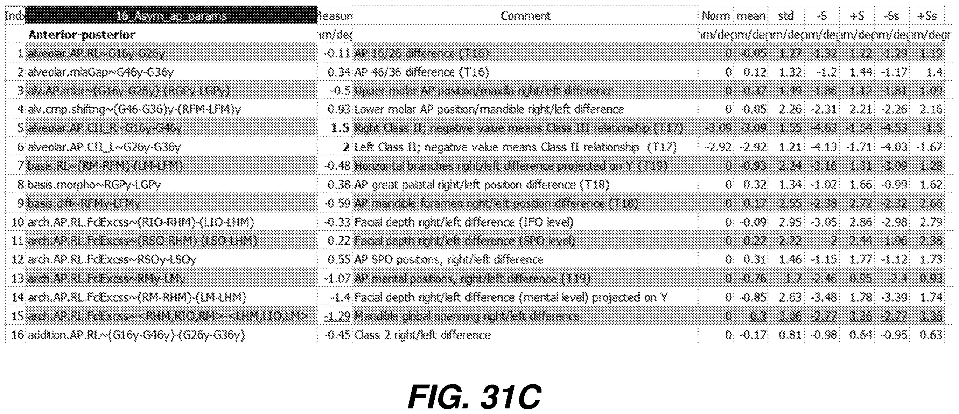

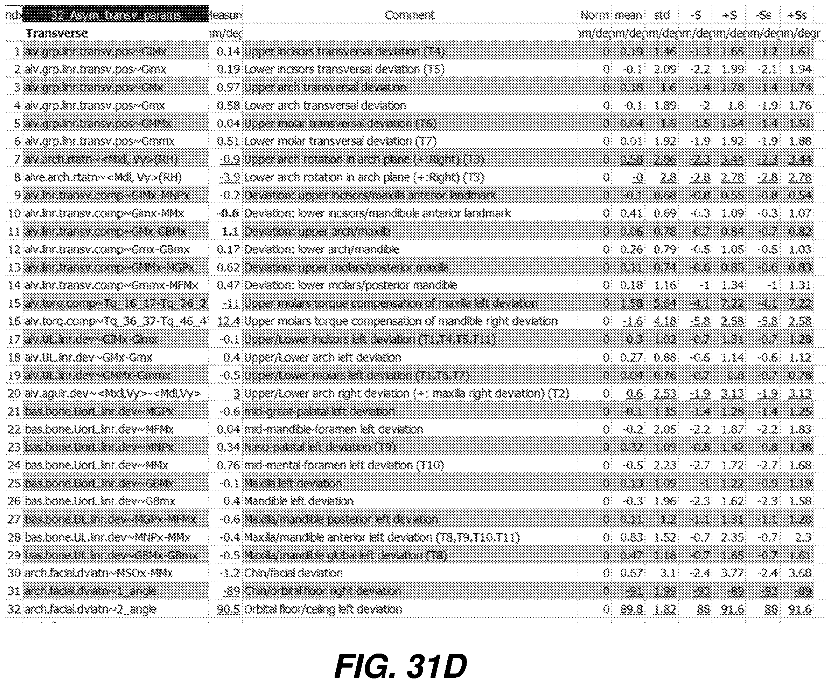

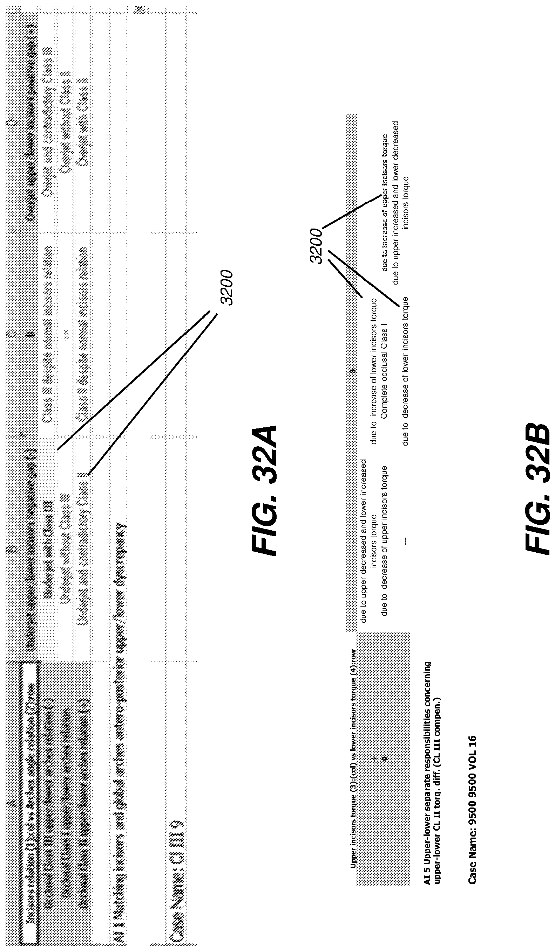

Figure 31A lists, for a particular patient, example parameters as numerical values and their interpretation with respect mainly to malocclusion of teeth, based on the listing of 26 parameters given previously. Figures 31B, 31C and 31D list, for a particular patient, example parameters as numerical values and their interpretation with respect to maxillofacial asymmetry, based on the listing of total 63 parameters given in an exemplary embodiment of this application. Figure 32A shows exemplary tabulated results 3200 for a particular example with bite analysis and arches angle characteristics. In the example of Figure 32A, the columns indicate an underjet, normal incisors relation, or overjet condition. Rows represent occlusal classes and arches angle conditions. As Figure 32A shows, highlighting can be used to accentuate the display of information that indicates an abnormal condition or other condition of particular interest. For the particular patient in the Figure 32A example, analysis indicates, as a result, an underjet condition with Class III bite characteristics. This result can be used to drive treatment planning, depending on severity and practitioner judgment.

Figure 32B shows exemplary tabulated results 3200 for another example with analysis of torque for upper and lower incisors, using parameters 3 and 4 from the listing given previously.

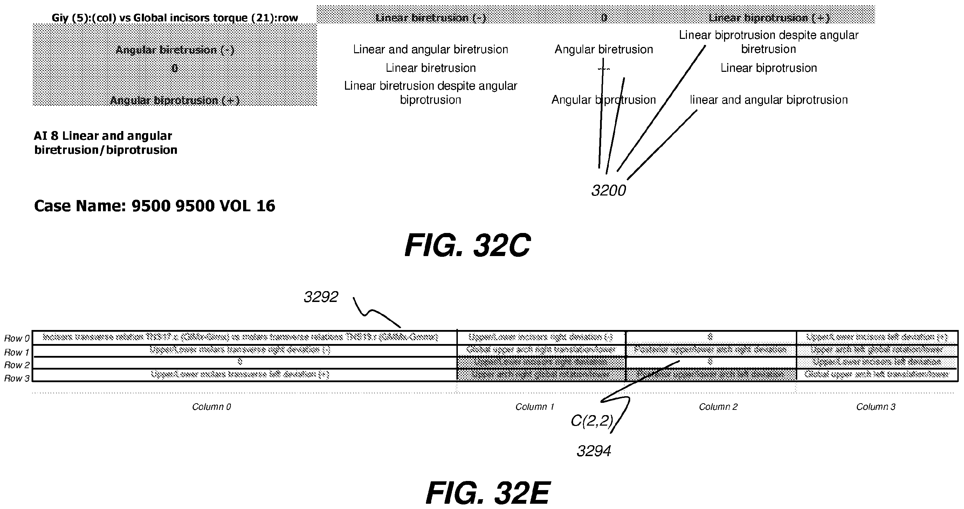

Figure 32C shows exemplary tabulated results 3200 for another example with assessment of biretrusion or biprotrusion using calculated parameters given earlier as parameters (5) and (21).

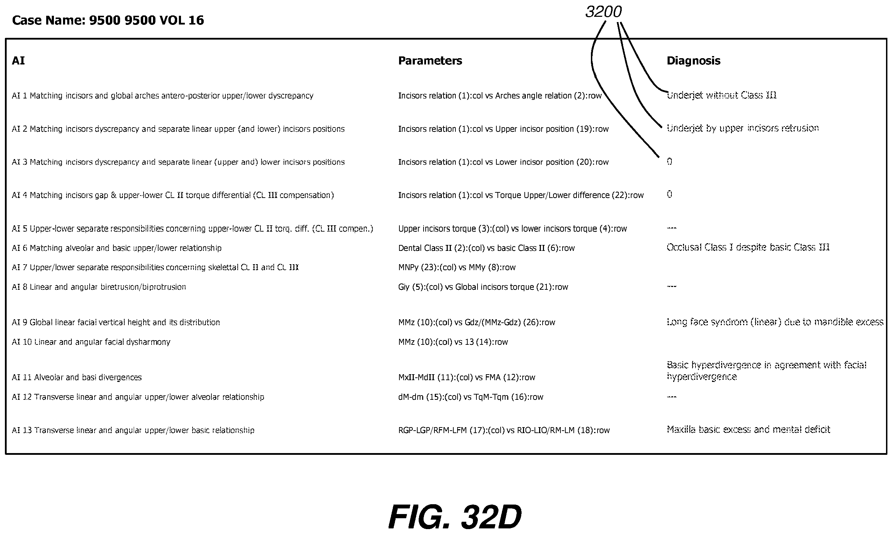

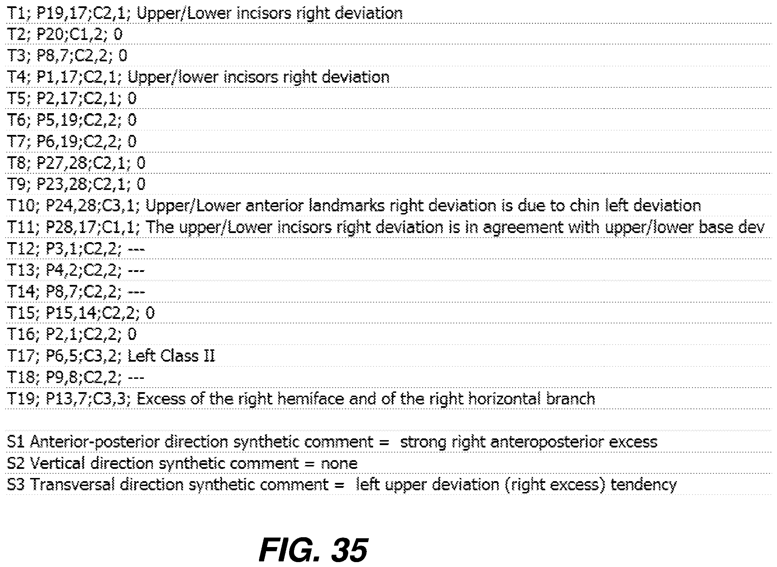

Figure 32D shows an exemplary summary listing of results for cephalometric analysis of a particular patient. The listing that is shown refers to analysis indications taken relative to parameters 1 - 26 listed previously. In the particular example of Figure 32D, there are 13 results for parameter comparisons using biometric parameters and dentition information derived as described herein. Additional or fewer results could be provided in practice. Figure 32E shows a detailed listing for one of the conditions reported in a tabular listing with a table 3292 with cells 3294 as shown subsequently (Figure 35).

Results information from the biometry computation can be provided for the practitioner in various different formats. Tabular information such as that shown in Figures 31A - 32E can be provided in file form, such as in a comma-separated value (CSV) form that is compatible for display and further calculation in tabular spreadsheet arrangement, or may be indicated in other forms, such as by providing a text message. A graphical display, such as that shown in Figure 26, can alternately be provided as output, with particular results highlighted, such as by accentuating the intensity or color of the display for features where measured and calculated parameters show abnormal biometric relations, such as overjet, underjet, and other conditions.

The computed biometric parameters can be used in an analysis sequence in which related parameters are processed in combination, providing results that can be compared against statistical information gathered from a patient population. The comparison can then be used to indicate abnormal relationships between various features. This relationship information can help to show how different parameters affect each other in the case of a particular patient and can provide resultant information that is used to guide treatment planning.

Referring back to Figure 1, memory 132 can be used to store a statistical database of cephalometric information gathered from a population of patients. Various items of biometric data that provides dimensional information about teeth and related supporting structures, with added information on bite, occlusion, and interrelationships of parts of the head and mouth based on this data can be stored from the patient population and analyzed. The analysis results can themselves be stored, providing a database of predetermined values capable of yielding a significant amount of useful information for treatment of individual patients. According to an embodiment of the present disclosure, the parameter data listed in Figures 31A and 31B are computed and stored for each patient, and may be stored for a few hundred patients or for at least a statistically significant group of patients. The stored information includes information useful for determining ranges that are considered normal or abnormal and in need of correction. Then, in the case of an individual patient, comparison between biometric data from the patient and stored values calculated from the database can help to provide direction for an effective treatment plan.

As is well known to those skilled in the orthodontic and related arts, the relationships between various biometric parameters measured and calculated for various patients can be complex, so that multiple variables must be computed and compared in order to properly assess the need for corrective action. The analysis engine described in simple form with respect to Figures 27 and 28 compares different pairs of parameters and provides a series of binary output values. In practice, however, more complex processing can be performed, taking into account the range of conditions and values that are seen in the patient population.

Highlighting particular measured or calculated biometric parameters and results provides useful data that can guide development of a treatment plan for the patient.

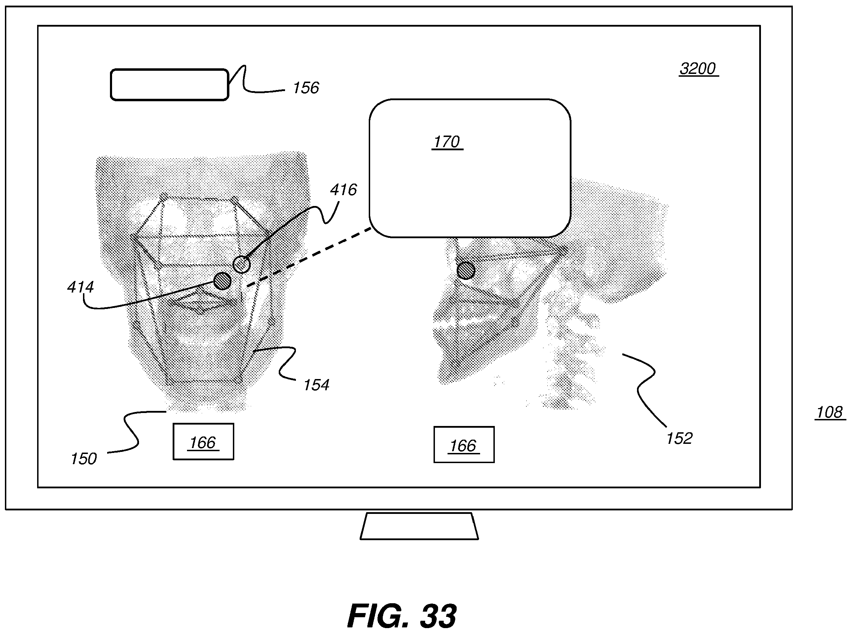

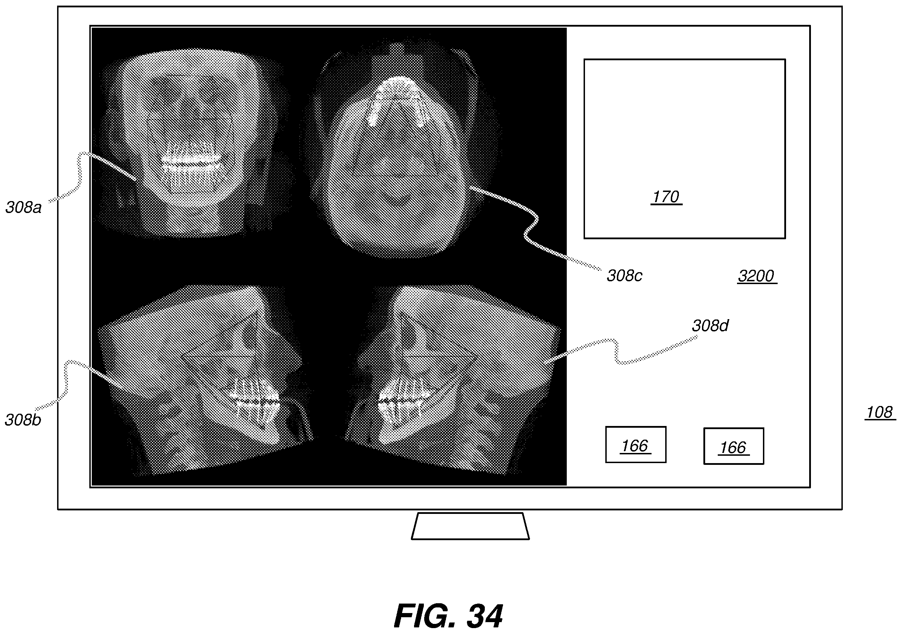

Figure 33 shows a system display of results 3200 with a recommendation message 170 based on analysis results and highlighting features of the patient anatomy related to the recommendation. Figure 34 shows a system display 108 with a graphical depiction of analysis results 3200. Annotated 3-D views (e.g., 308a-308d) are shown, arranged at different angles, along with recommendation message 170 and controls 166.

Certain exemplary method and/or apparatus embodiments according to the present disclosure can address the need for objective metrics and displayed data that can be used to help evaluate asymmetric facial/dental anatomic structure. Advantageously, exemplary method and/or apparatus embodiments present measured and analyzed results displayed in multiple formats suitable for assessment by the practitioner.

Figure 35 shows an exemplary text report for maxillofacial asymmetry assessment according to an embodiment of the present disclosure. The report lists a set of assessment tables (T1 - T19) available from the system, with cell entries (denoted by C with row and column indices, C(row, column)) providing maxillofacial/dental structural asymmetry property assessment comments organized based on the calculations related to relationships between obtained parameters, such as parameters P1-P15 in Figure 31B. An exemplary assessment table 3292 is depicted in Figure 32E, having four rows and four columns.

In one embodiment, for each exemplary assessment table (e.g., 19 assessment tables), only one cell 3294 can be activated at a time; the activated cell content is highlighted, such as by being displayed in red font. In the exemplary table 3292, the activated cell is C(2,2)(3294) with a content "0" indicating that asymmetry is not found for the property of incisors and molars upper/lower deviations.

For a quick reference to the exemplary assessment tables, the system of the present disclosure generates a checklist type concise summary page (e.g., Figure 35) that provides information with respect to table numbers *(Tn)*, parameter numbers *(Pk, j)*, cell indices *(Cs, t)*, and actual assessment comments from assessment tables T1-T19. The information obtained from this type of text report can be helpful for the practitioner, providing at least some objective metrics that can be useful in developing a treatment plan for a particular patient or evaluating treatment progress. Further beneficial to the practitioner can be cumulative summative evaluations directed to overall condition assessments of a patient. This can be the situation, in particular, when numbers of conditional reference points and relationships therebetween utilized to determine asymmetric facial/dental anatomic structures or relationships for a patient involve large numbers of view-oriented and 3D-oriented treatment conditions, with variable underlying causes.

In one exemplary asymmetric determination table embodiment, 19 assessment tables can be included with hundreds of reference points and several hundreds of relationships therebetween. In this exemplary asymmetric determination table embodiment, tables include:

T1: Asymmetric matching incisors and molars upper/lower deviations;

T2: Arch rotation;

T3: Upper/lower arch right rotation and upper or lower arch responsibility;

T4: Asymmetric matching incisors upper/lower deviations with upper inc transverse deviation, response of upper or lower arch in the upper/lower incisors trans deviation;

T5: Asymmetric matching incisors upper/lower deviations with anterior bases transverse deviation, response of upper or lower arch anterior deviation in the upper/lower incisors trans deviation;

T6: Asymmetric matching incisors upper/lower molar deviations with upper molars transverse deviation, response of upper or lower molars trans deviation;

T7: Asymmetric matching incisors upper/lower molar deviations with lower molars transverse deviation;

T8: Asymmetric matching basic bones upper/lower deviations;

T9: Asymmetric matching basic bones upper/lower anterior relations with anterior maxilla deviation;

T10: Asymmetric matching basic bones upper/lower anterior relations with anterior mandible deviation;

T11: Asymmetric matching incisors upper/lower deviations with anterior bases transverse deviation;

T12: Vertical asymmetric comparing L/R molars altitudes difference with maxillary arch rolling;

T13: Asymmetric comparing L/R molars altitudes difference with mandible arch rolling;

T14: Vertical asymmetric comparing basic bones R/L posterior differences (maxillary & mandible);

T15: Vertical asymmetric comparing L/R difference at mental points level (measuring maxilla-facial area and global face);

T16: Anterior-posterior asymmetric comparing R/L upper/lower molars anterior-posterior difference with lower ones;

T17: Anterior-posterior asymmetric comparing R/L upper/lower molars anterior-posterior relationship difference with lower ones;

T18: Anterior-posterior asymmetric comparing L/R upper basis lateral landmarks anterior-posterior difference with lower ones;

T19: Anterior-posterior asymmetric matching mandibular horizontal branch with R/L global hemifaces;

In such complex asymmetric facial/dental anatomic structures or relationships determinations according to this application, optional cumulative summative evaluations directed to overall condition assessments of a patient are preferably used. In some embodiments, exemplary cumulative summative or overall diagnosis comments can include: Asymmetry anterior posterior direction (AP comment or S1), Asymmetry vertical direction (VT comment or S2), and Asymmetry Transverse direction (TRANS comment or S3). Still further, highest level evaluation score(s) can be used by using one or more or combining S1, S2 and S3 to determine an Asymmetry global score (Asymmetry Global determination). For example, the exemplary Asymmetry global score can be a summary (e.g., overall class I,II, III), broken into few, limited, categories (e.g., normal, limited evaluation, detailed assessment suggested) or represented/characterized by dominant asymmetry condition (e.g., S1, S2, S3).

As shown in Figure 35, the exemplary text report also presents S1 anterior-posterior direction "synthetic" asymmetry comment, S2 vertical direction synthetic asymmetry comment, and S3 transversal direction synthetic asymmetry comment.

The "synthetic" terminology is derived in this application to form a pair of tables in each direction. In certain exemplary embodiments, the "synthetic" terminology can be determined from a combination of a plurality of tables from each assessment type (e.g., AP, V, Trans involving or representing substantial (e.g., >50%) portions of the skull) or a pair of tables in each direction.

For example, S1 synthetic comment is derived from Table 17 and Table 19. The derivation first assigns a score to each of the cells of Table 17 and Table 19. An exemplary score assignment is explained as follows

For Table 17, $C(1,3) = -2$; $C(1,2) = C(2,3) = -1$; $C(2,1) = C(3,2) = 1$; $C(3,1) = 2$; other cells are assigned with a value 0.

For Table 19, $C(1,1) = -2$; $C(1,2) = C(2,1) = -1$; $C(2,3) = C(3,2) = 1$; $C(3,3) = 2$; other cells are assigned with a value 0.

The derivation of S1 synthetic comment evaluates the combined score by adding the scores from Table 17 and Table 19.

For instance, if $C(1,3)$ in Table 17 is activated and $C(1,1)$ in Table 19 is activated then the combined score will be the summation of the scores of $C(1,3)$ of Table 17 and $C(1,1)$ of Table 19. Since $C(1,3)$ in Table 17 is assigned with a value -2 and $C(1,1)$ in Table 19 is assigned with a value -2, therefore, the combined the score is -4. Obviously, the possible combined sore values for S1 are -4, -3, -2, -1, 0, 1, 2, 3 and 4.

The exemplary S1 synthetic comments can be based on the combined score value are summarized below.

If the combined score = -4 or -3, the S1 synthetic comment = strong left anterior-posterior excess.

If the combined score = -2, the S1 synthetic comment = left anterior-posterior excess tendency.

If the combined score = 2, the S1 synthetic comment = right anterior-posterior excess tendency.

If the combined score = 4 or 3, the S1 synthetic comment = strong right anterior-posterior excess.

If the combined score = 0, no comment.

Similar synthetic comment derivations are applied to the vertical direction and transversal direction.

Referring back to Figure 35, the exemplary text report displays S1 = strong right anteroposterior excess, S2 = none and S3 = left upper deviation tendency.

In very rare cases, synthetic comments show up in all three directions, or the comments present some type of mixture of synthetic comments, which can prompt further extended diagnosis and/or treatment.

Further, selected exemplary method and/or apparatus embodiments according to the application can also provide a quick visual assessment of the asymmetry property of the maxillofacial/dental structural of a patient.

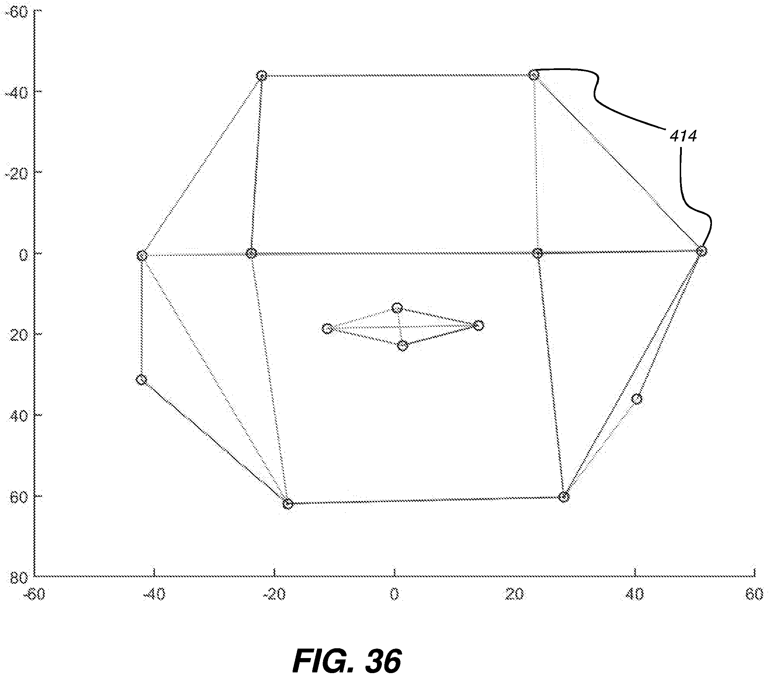

Figure 36 is a plot or graph that shows the maxillofacial/dental structural features for a patient with respect to a front view, plotted using the landmarks, reference marks 414, selected by the operator (see Figure 5). This type of displayed plot clearly shows asymmetry (left vs. right) in an objective fashion for this exemplary patient.

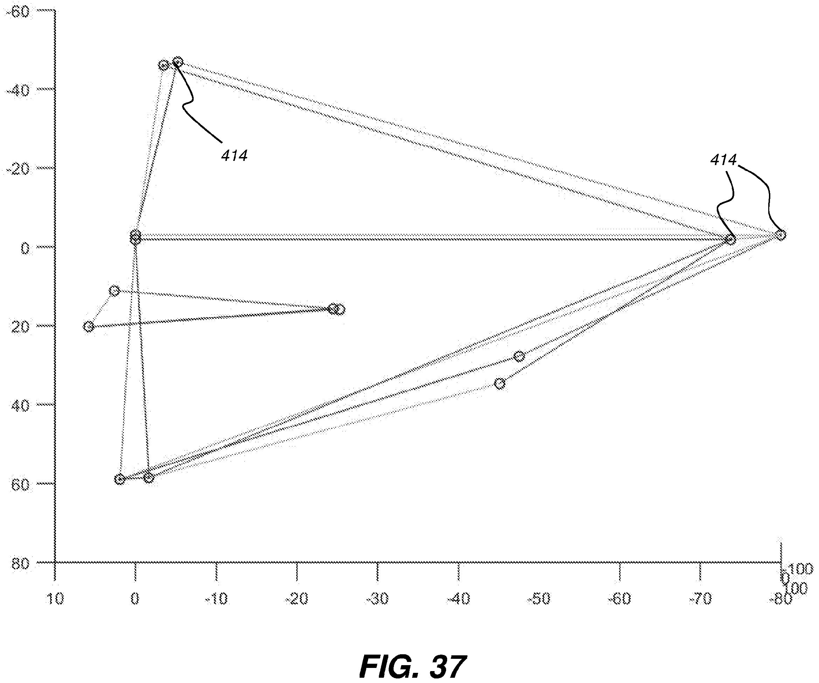

Likewise, Figure 37 is a plot or graph of a sagittal view, with reference marks 414 showing how closely the left and right sides of a patient's face overlap, as another objective indicator of asymmetry.

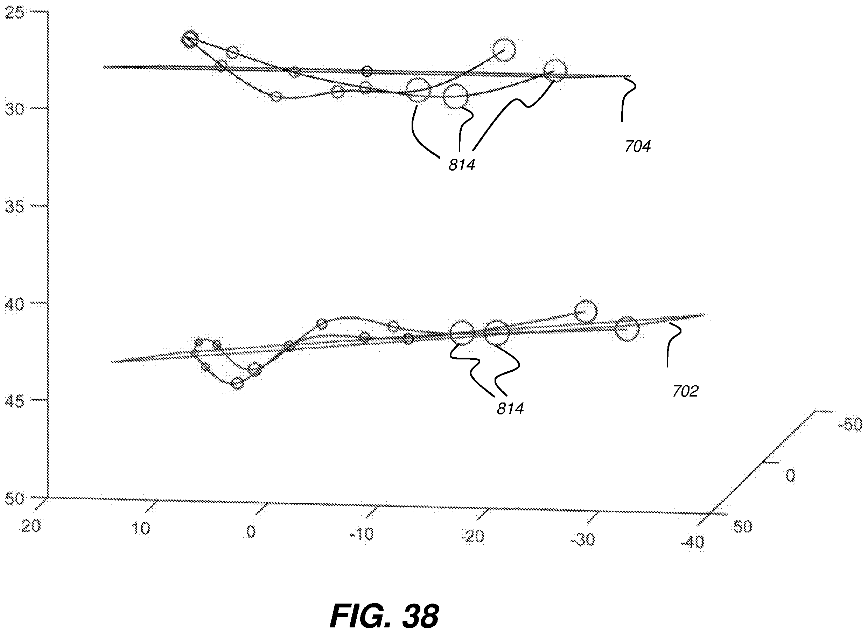

Figure 38 is a plot or graph providing a quick visual assessment of noticeable improper alignment of the bite of a patient in a sagittal view of upper and lower jaw planes 704 and 702. Jaw plane 704 is computed based on the derived upper jaw marks 814, lower jaw plane 702 is computed based on the derived lower jaw marks 814. Derived marks 814 are computed based on the segmented teeth 304 shown in Figure 4 and show the location of the tooth. The example shown in Figure 38 depicts exemplary visual cues for a patient with a hyperdivergent pattern.

An embodiment of the present disclosure uses measurements of relative positions of teeth and related anatomy from either CBCT, optical scanning, or both as input to an analysis processor or engine for maxillofacial/dental biometrics. The biometrics analysis processor, using artificial intelligence (AI) algorithms and related machine-learning approaches, generates diagnostic orthodontic information that can be useful for patient assessment and ongoing treatment. Using the generated AI output data and analysis from the biometrics analysis processor, an AI inverse operation then generates and displays quantitative data to support corrective orthodontics.

According to an embodiment of the present disclosure, the described method provides an automated solution for defining an optimal arch form based on the teeth positions of the individual patient prior to orthodontic treatment.

Guidance can also be provided for use of dental appliances, including design, use, placement arrangements and combinations having one or more aligners, braces, positioners, retainers, and the like. To support deployment of orthodontic appliances, an embodiment of the present disclosure provides guidance for a multi-step process toward achieving an optimal arch form. For the individual patient, the method computes a set having multiple recommended motion vectors that can be used to direct tooth repositioning. According to an alternate embodiment, one or more of the appropriate dental appliances can be fabricated in whole or in part using a 3D printer, if feasible; an appropriate appliance can alternately be assembled using an arrangement of standard brackets and braces.

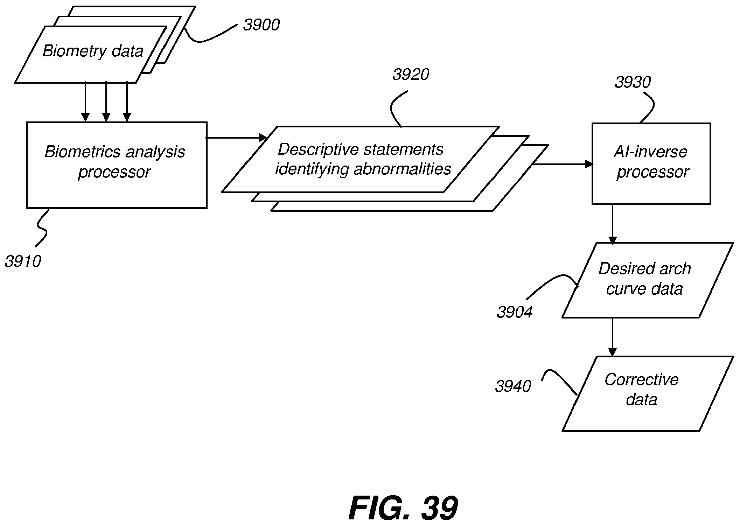

The flow diagram of Figure 39 shows the logic processing mechanisms and data that can be used for providing assessment and guidance to support orthodontic applications. Biometry data 3900, obtained from CBCT, optical scanning, or other source, and population data is input to a biometrics analysis processor 3910. In response, biometrics analysis processor 3910, an artificial intelligence (AI) engine, performs calculations as described previously and generates descriptive statements 3920 identifying one or more dental/maxillofacial abnormalities. According to an exemplary embodiment of the present disclosure, descriptive statements describing one or more dental/maxillofacial abnormalities can include some of those given previously in Figures 31A-32D.

Continuing with the Figure 39 process, an AI-inverse processor 3930 provides a logic engine that generates recommended or desired arch curve data 3904 based on and generated from anatomy of the individual patient. Based on the descriptive statements 3920 and on the desired arch curve data 3904, AI-inverse processor 3930 then generates corresponding corrective data 3940 that can include the motion vectors needed for tooth repositioning, and related data for guidance in orthodontics.

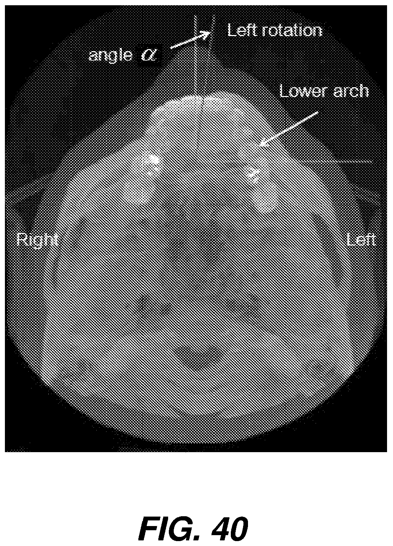

AI-inverse processing can begin with arch form optimization. The example case shown in Figure 40 shows an image of a typical patient condition, arch-left rotation by an angle $\alpha$. A non-zero angle indicates tooth arch form asymmetry. The sequence performed for arch rotation correction provides an example of the activity of AI-inverse processor 3930 for this typical case. Sequence steps are outlined following:

1. The AI engine, biometry analysis processor 3910 of Figure 39, analyzes biometric data from the CBCT reconstruction and generates descriptive statements indicative of the arch-left rotation condition shown in Figure 40.

2. The AI-inverse engine, AI-inverse processor 3930, based on the descriptive statements and on recommended or desired results for arch characteristics, generates patient-specific, coherent and optimized geometric primitives and related parameters that indicate quantitative tooth movement values for the current stage of the treatment process.

For the example of Figure 40:

(i) The AI engine detects an arch rotation that is a function $f(\mathbf{t})$ of tooth vector $\mathbf{t}$ representing the set $\{t_1,...t_N\}$, wherein N is the number of teeth in the arch. The positions (in an exemplary 2D space) of $\mathbf{t}$ can be corrected by the AI engine inverse operation by rearranging the teeth $\mathbf{t}$ to minimize the arch rotation in a systematic and automated manner:

min $f(\mathbf{t})$;
this expression is subject to an exemplary function
$g(\mathbf{t})$ = $4^{th}$ order polycurve, which, in turn, leads to solving an over-determined system in an exemplary 2D space: $\mathbf{X}^T\beta = \mathbf{y}$
wherein $\mathbf{X}^T$ signifies a matrix that contains all the teeth's 0 to $n^{th}$ order x positions in the exemplary 2D space. An exemplary value is $n = 4$. Variable $\mathbf{y}$ signifies a vector $\{y_1, .. y_N\}$ that contains all the teeth's $1^{st}$ order y positions in the exemplary 2D space; again, variable N is the number of teeth included in the arch.
Where $\beta$ signifies a vector $\{\beta_0 \Lambda\ \beta_n\}$ with the object function

$$\hat{\boldsymbol{\beta}} = \arg\min_{\beta} S(\boldsymbol{\beta})$$

The concept of corrective means is applicable to 3D space.

(ii) With the goal of achieving: $\min(f(\mathbf{t}) = \alpha)$, the sequence can be as follows:

(1) First, compute a tensor matrix

$$\mathbf{I} = I_d trace(\mathbf{C}) - \mathbf{C};$$

where $$\mathbf{C} = \sum_k m_k \mathbf{p}_k \mathbf{p}_k^T$$

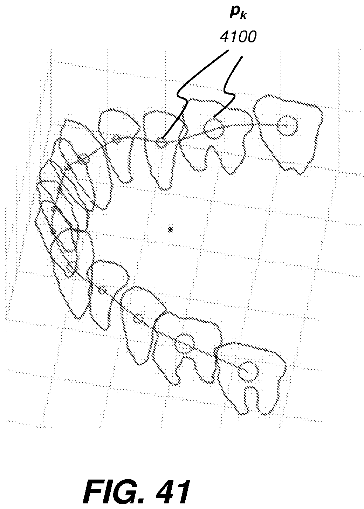

wherein $d = 2$ or 3, for 2D or 3D calculation, correspondingly;
$\mathbf{p}_k$ represents the (x,y,z) position vector of an element (a voxel of a tooth, for example). In the arch rotation case of Figure 40, $\mathbf{p}_k$ is the center of inertia of tooth $k$. $k$ = {1, 2, 3, ...N}. Again, variable N is the number of teeth included in the arch. Figure 41 shows, in a 3D perspective view, an exemplary representation of inertia centers $\mathbf{p}_k$ 4100 along an arch. Embodiments of the present disclosure display the inertia centers in a 2D plane, showing recommended adjustments for inertia center positioning. The procedures for generating recommended adjustments can alternately be extended to correction in 3D space.

(2) Second, compute eigenvectors of tensor **I** in order to compute the arch rotation angle $\alpha$;
(3) Third, compute the arch curve, such as $g(\mathbf{t})$ = $4^{th}$ *polycurve* using tooth inertia centers $\mathbf{p}_k$ as input points.

It is noted that the set of inertia centers $\mathbf{p}_k$ can be either augmented with additional input points or, alternately, reduced in size by removing outlier input points. Exemplary additional input points could be the original inertia centers with flipped sign (x direction); exemplary outlier points could be those whose coordinates show significant deviation from an ideal arch shape.

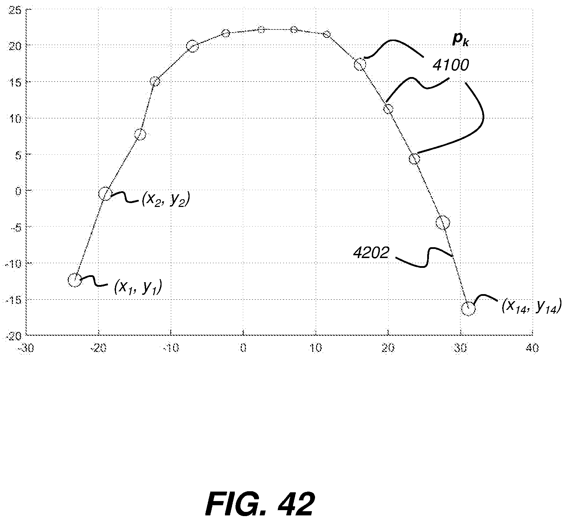

To simplify the problem, the $4^{th}$ order poly-curve (polynomial curve) $\hat{\beta}$ can be computed in (x,y) space, that is, with variable $d$ = 2. Figure 42 shows inertial centers 4100 plotted in x,y space (2D) along an initial, uncorrected piecewise linear arch curve 4202 with 14 teeth. A few of the inertial centers 4100 are labeled with exemplary coordinate designations $\{(x_1, y_1), (x_2, y_2), ... (x_{14}, y_{14})\}$.

The poly-curve (polynomial curve) $\hat{\beta}$ computation can be obtained by minimizing:

$$S(\boldsymbol{\beta}) = \sum_{i=1}^{m} \left| y_i - \sum_{j=1}^{n} x_{ij}\beta_j \right|^2 ;$$

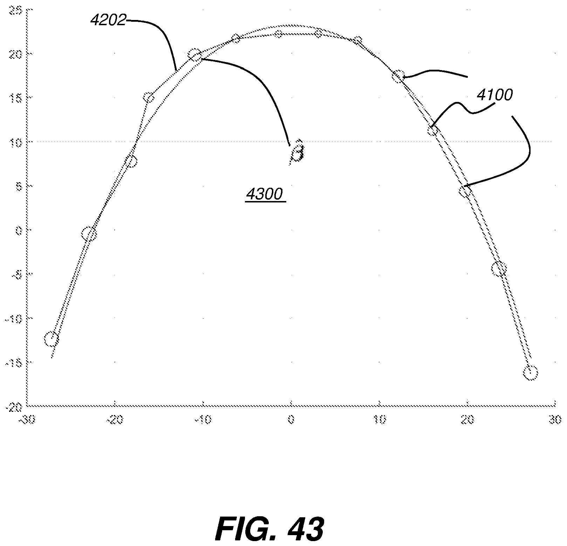

wherein $y_i$ is an element of $\{y_1, .. y_N\}$, $x_{ij}$ is an element of matrix $\mathbf{X}^T$. The above $S(\beta)$ equation signifies an overdetermined linear system that can be solved, for example, by using the well known pseudo-inverse method familiar to individuals skilled in the art. Following this calculation, the tooth inertia centers 4100 shown in Figure 42 can then be directly mapped. In one direct mapping method, the $x_n$ value for each inertia center $\mathbf{p}_k$ 4100 is fixed and the $y_n$ value adjusted to vertically shift the inertia center onto the polynomial curve $\hat{\beta}$. As shown in Figure 43, the individual tooth inertia centers $\mathbf{p}_k$ 4100 can be vertically moved by a slight amount in order to fit the polynomial curve, while holding the corresponding x values constant.

The original uncorrected centers can also be moved onto the polynomial curve $\hat{\beta}$ by appropriately choosing one of the roots of the $n^{th}$ order polynomial curve, holding the $y_n$ value as the fixed input and $x_n$ (the roots) as the output. The tensor matrix **I** can then be recomputed using the moved centers. Eigenvectors of the tensor can be recomputed and arch rotation angle $\alpha$ (Figure 40) recalculated.

The processing described above can be repeated until angle $\alpha$ reaches a predetermined minimal value or zero (indicating a symmetric tooth arch form).

Figure 43 shows the desired optimized arch curve $\hat{\beta}$ 4300 overlaid for comparison with the original arch form before optimization, computed as described previously.

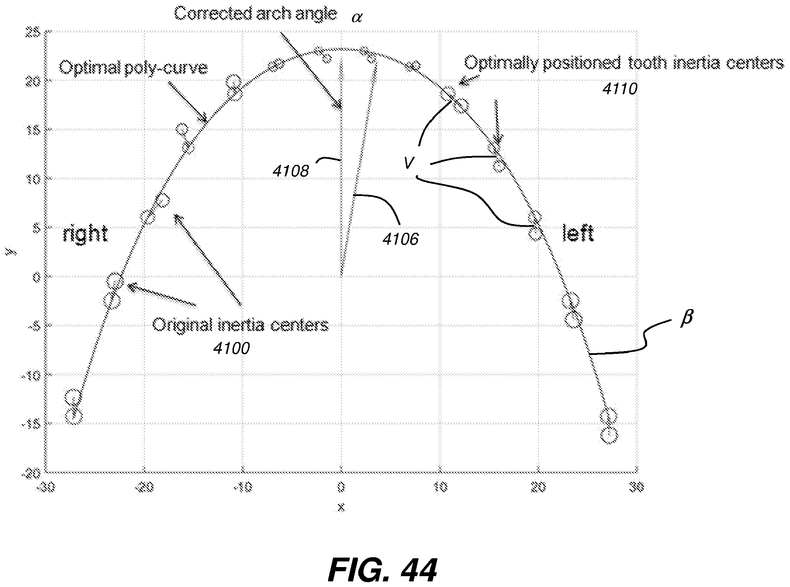

The recommended movement of the teeth from their original positions to desired positions based upon the computed optimization can be displayed and reported to the practitioner, as shown in Figure 44.

In the reported data of Figure 44, the original tooth inertia centers 4100 for the arch can be shown, such as highlighted in a particular color or with other suitable display treatment. Motion vectors V then show the desired movement from the original inertia center 4100 position to an optimized inertia center 4110. The original arch rotation is represented by a vector 4106. Corrected arch rotation angle $\alpha$ is represented by a vector 4108.

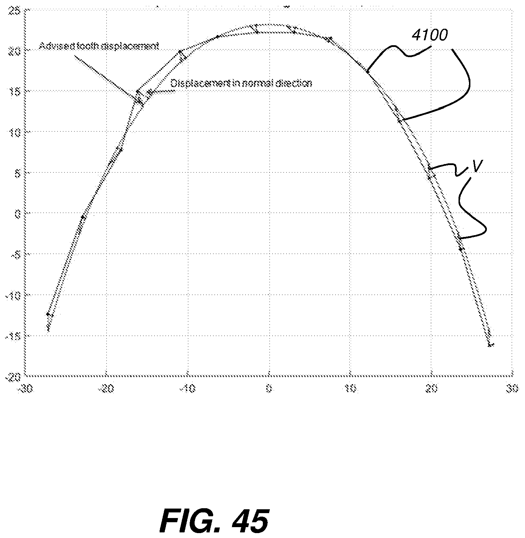

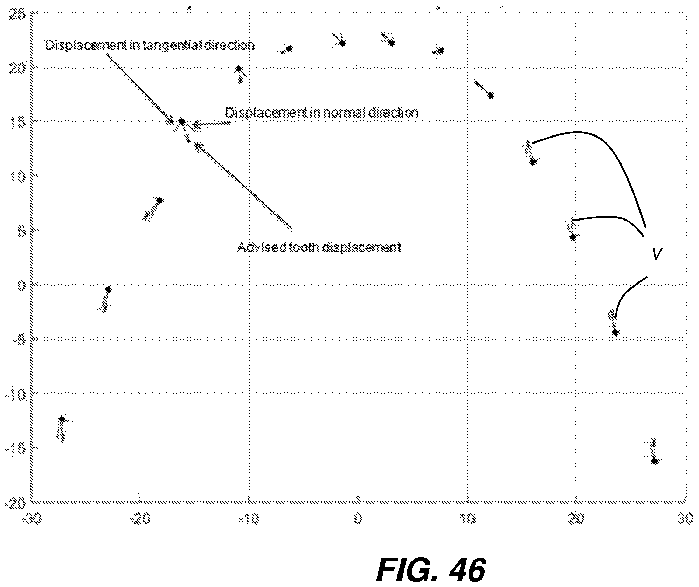

Figure 45 shows a graph with tooth displacement vectors V indicated, based on the above computation. Figure 46 shows a graph with vector displacement decomposed into mutually orthogonal tangential and normal component directions.

For the example shown in Figure 40, corrective data showing teeth displacement vectors can help to serve as a guideline for orthodontic treatment. Exemplary optimized $4^{th}$ order polynomial curve intercept and coefficient parameters can be as follows:

$$\hat{\boldsymbol{\beta}} \ni \hat{\beta}_i; i = \{0,...4\}$$

23.174417248277855
-0.000000000000000
-0.037164341067977
0.000000000000000
-0.000018751015683

According to an embodiment of the present disclosure, the system provides the type of data presented in Figure 46, in some form, to the practitioner to support an orthodontic treatment plan for a patient. For each tooth in the dental arch, a corresponding vector V is calculated based on the recommended or desired adjustment to tooth inertia center position.

Multi-stage embodiment

An alternate embodiment of the present disclosure extends the logic for tooth position adjustment to correspond more closely to a multi-stage sequence for orthodontic treatment. This embodiment provides multiple iterations of the repositioning calculation and reporting process, tracking patient progress more closely to recommend the necessary adjustments at each stage.

The system of the present disclosure receives, at a time $T_0$, first positional data of the components (teeth) of the patient's dentition with the digital data extracted, through an AI-Engine, from at least one 3D digital volume acquisition modality applied to the dentition. The 3D volume could be acquired by using a CBCT scanner or an optical scanner or a laser scanner.

Automatically, through an AI-Engine inverse operation, the system of the present disclosure produces second positional digital data of the components (teeth) of the patient's dentition based on the first positional digital data of the dentition components with the second positional digital data highly optimized so that, at time $T_1$ after orthodontic treatment, the resultant arch form of the dentition components (teeth) is an improved fit for a number of aesthetic and functional requirements.

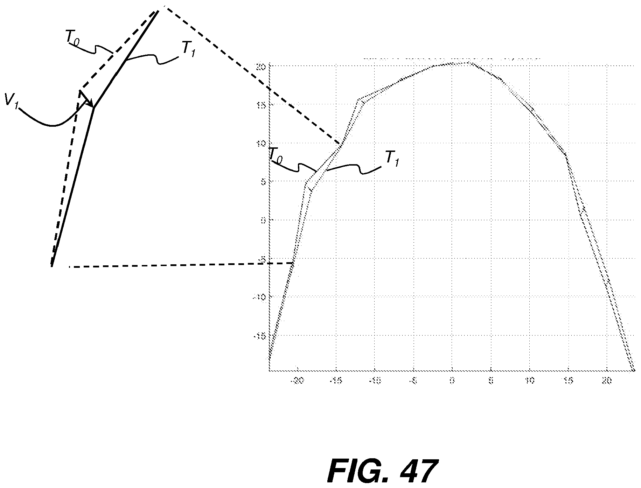

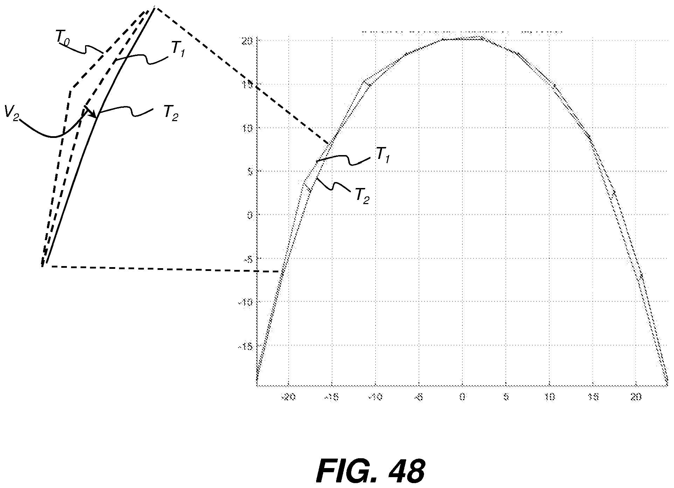

Figures 47 and 48 show a succession for arch improvement by moving teeth in stages using the iterative arch approximation calculations described herein. The graphical representation of Figure 47 shows a first step executed using the AI-inverse operation, for dental arch shape from a 3D volume, acquired by using a CBCT scanner or an optical scanner or a laser scanner. The dashed line representation, in the partial enlarged section, shows a portion of the arch curve following extraction of the original patient data at time $T_0$.

In practice, at time $T_0$, both the CBCT scan and intraoral optical scan have been acquired. 3D tooth models, with roots, can be generated from the CBCT scan; 3D crown models, without roots, can be generated from the intraoral optical scan. Crown models without roots and tooth models with roots can then be registered at time $T_0$.

In the process shown in Figures 47 and 48, the tooth arch form optimization procedure described previously is applied to the data acquired at time $T_0$, producing a first set of motion vectors $\mathbf{V_1}$. These initial movement vectors can be considered as "scaled" or "scaled-back" versions of the full scale movement vectors V described with reference to Figure 46, for example, $\mathbf{V_1} = 0.5V$.

These scaled vectors provide data for a single stage in the treatment procedure. With respect to the arch mapping graph of Figure 47, for example, vector $\mathbf{V_1}$ provides the indicated movement of the corresponding tooth inertial center from time $T_0$ to time $T_1$.

Using this multi-stage sequence, the iterative logic repeats its processing at the end of the first stage, effectively using the second positional data of time $T_1$ as a starting point, so that the $T_1$ position replaces the $T_0$ position and processing continues.

In practice, at time $T_k$ where k > 0, the 3D crown models without roots can be acquired by using an intraoral optical scanner without acquiring another CBCT scan; this sequence helps to reduce patient X-ray exposure. Tooth models with roots obtained at time $T_0$ can be aligned with crown models without roots at time $T_k$, so that a new set of tooth models with roots that are aligned with crown models without roots is formed at time $T_k$.

This new set of tooth models with roots can be used to assess the treatment performance. A revised treatment plan can be designed and a set of new tooth movement vectors can be computed accordingly.

Through a follow-up AI-inverse operation, the system of the present disclosure produces another second positional digital data of patient dentition based on the new first positional digital data computed at time $T_1$. As shown in Figure 48, optimization of the calculated patient dentition yields a further improved arch form, using motion along vector $V_2$ to produce the new positions at time $T_2$. Following orthodontic treatment, the resultant arch form provides an improved fit of the dentition components (teeth) for aesthetic and functional requirements.

The exemplary two-stage process ($T_0$-$T_1$, $T_1$-$T_2$) described with reference to Figures 47 and 48 can be generalized as a multi-stage process represented by $T_i$-$T_{i+1}$. The multi-stage process can be terminated, in general, at the time Tn when the difference between the first positional digital data and the second positional data diminishes. Or, the completion of the multi-stage adjustment process can be determined using a predefined number of iterations, for example, or by evaluating further movement distance according to a predetermined threshold value.

As shown in the example of Figures 46, 47, and 48, the system of the present disclosure can automatically, at time $T_i$ (where i = 0,1,2,3,...n-1) determined by the orthodontic strategy, decompose the displacement data of the second positional data along tangential and normal directions with respect to the resultant arch form at time $T_i$.

The process of the present disclosure can automatically determine and/or fabricate, at time $T_i$, a positional corrective device for the dentition components based on the decomposed displacement data and vector V. If the decomposed displacement data is predominantly tangential, a brace may be preferred. If the dominant component of the decomposed displacement data is normal, an aligner may be preferred. In many cases, a combined aligner and brace device is preferred. Vectors V for each step in the process can be reported, such as displayed, printed, or stored, as well as provided to an appliance design system that provides fabrication of a suitable brace, aligner, or other dental appliance for tooth re-positioning, as described in more detail subsequently.

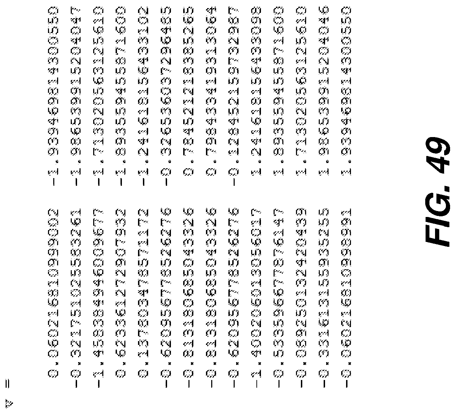

By way of example, Figure 49 shows a computer-generated listing of movement vectors V for each of 14 teeth of a dental arch for an orthodontic patient. Values for x- and y-axis movement are shown for each tooth vector V. Movement vector V values can be provided as a listing, such as in a file, or displayed to the practitioner.

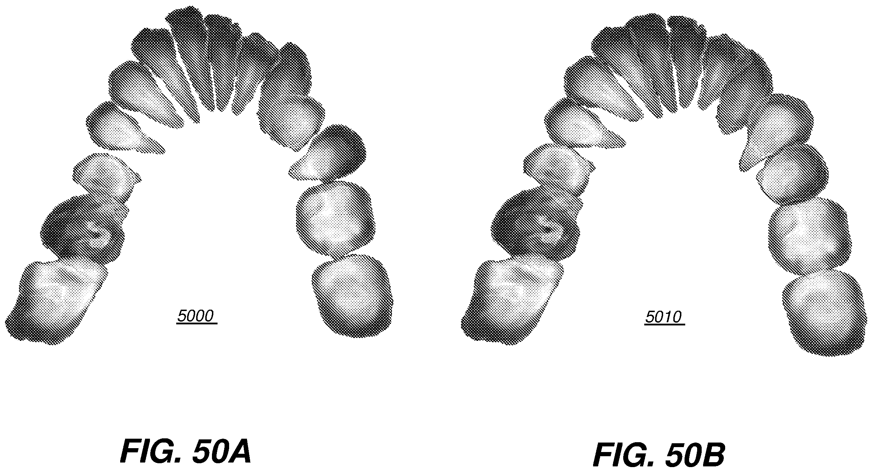

Figure 50A (a 3D rendering view) shows the arrangement of teeth in an initial, uncorrected arch 5000 for an exemplary orthodontic patient. Figure 50B shows the corrected arch 5010 following treatment recommendations provided by the system of the present disclosure.

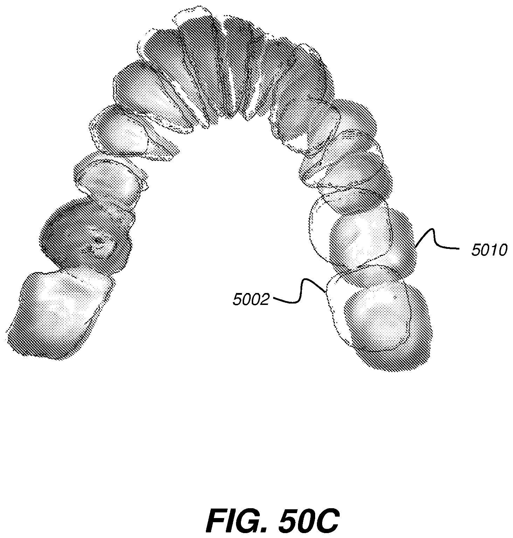

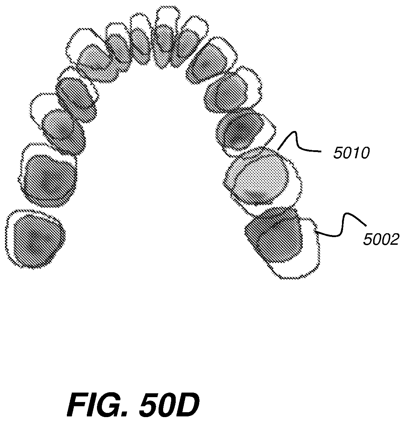

Figure 50C (also partially a 3D rendering view) shows the initial and optimized arches overlaid, with the original tooth positions indicated in outline 5002. Figure 50D (a slice of a 2D axial view) shows another example with the initial arch in outline 5002, overlaid on the optimized arch 5010.

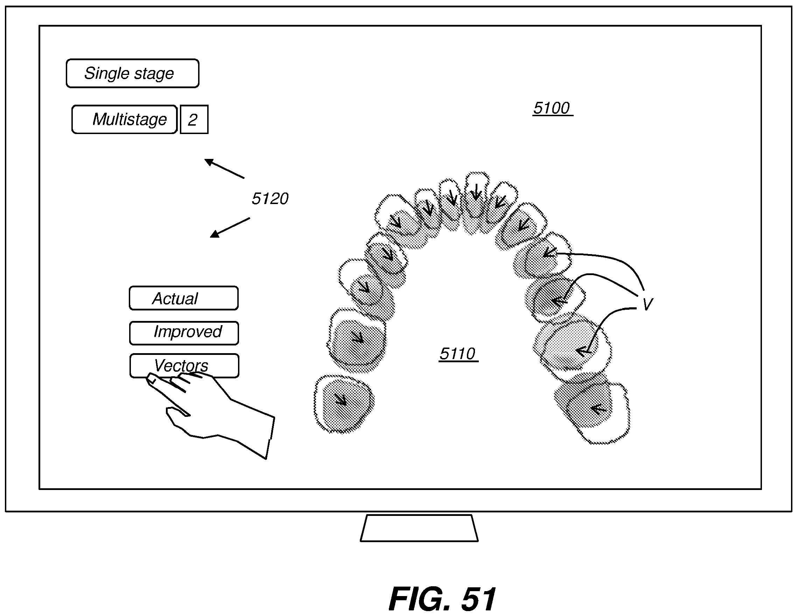

By way of an example of the present invention, the schematic view of Figure 51 shows an operator interface display 5100 for controlling the processing to provide orthodontic data and for display of processing results. An image portion 5110 provides a graphic representation of actual scanned results (as in Figure 50A), improved positioning (as in Figure 50B), and vectors V for proposed tooth movement (as in Figure 46). These different displayed data can be overlaid, for example, or shown separately as selected by the operator using controls 5120. The operator can also select different calculations and results depending on variable selections, such as for single stage or multi-stage treatment, as described with reference to Figures 47 and 48.

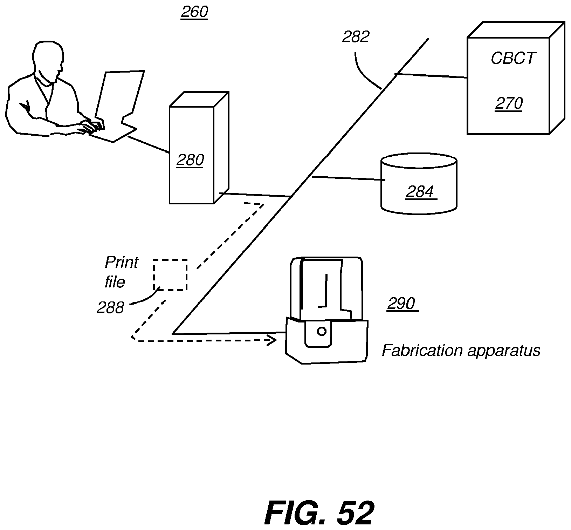

Embodiments of the present disclosure can generate vector and positioning information suitable for various types of appliance fabrication systems. The schematic diagram of Figure 52 shows an exemplary fabrication system 260 for forming an orthodontic appliance using the optimization methods of the present disclosure. Image data from an imaging apparatus 270, such as a CBCT imaging apparatus 100 as described with reference to Figure 1, can be provided as a data file or as streamed data over a wired or wireless network 282, such as an ethernet network with internet connectivity. The network 282 can be used to transfer this 3D image data to a memory 284 or storage on a networked server or workstation 280. Clinical indications and related parameters for the patient can be entered or otherwise associated with the 3D data through workstation 280. An automated or partially automated process can then execute at workstation 280, generating an appliance design by forming a print file 288 or other data structure supportive of appliance fabrication. Print file 288 or other fabrication instructions can go to a fabrication apparatus 290, which is also in signal communication with network 282.

The fabrication process of Figure 52 can be highly automated or partially automated, or may be a manual fabrication process that uses the vector data provided by the system. According to an embodiment of the present disclosure, fabrication apparatus 290 is a networked 3D printer that can be used to generate various arrangements of aligner or other appliance. The operator or practitioner at workstation 280 can provide various control functions and commands for 3D printer operation using the biometric analysis tools described herein. Other types of fabrication apparatus 290 may require additional setup or control and may require more extensive operator interaction or practitioner input in order to apply biometric analysis results.

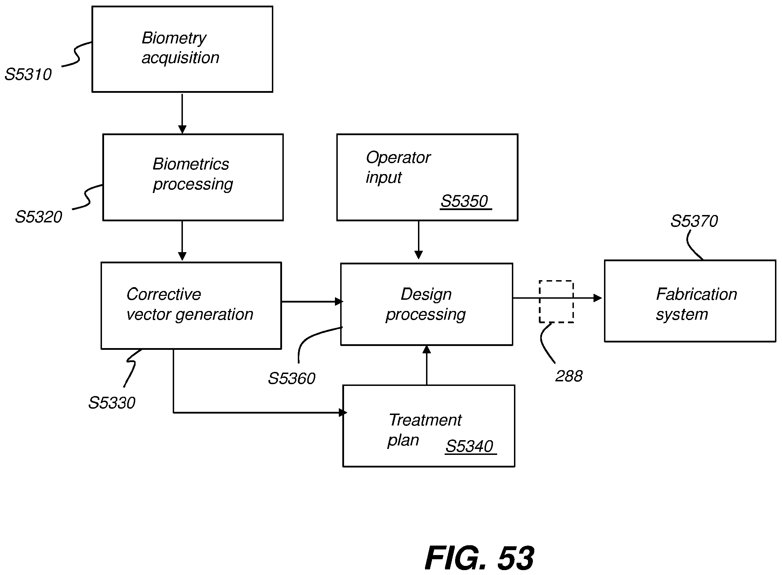

The flow diagram of Figure 53 shows a sequence for applying results of the orthodontic optimization to the task of appliance design and fabrication. A biometry acquisition step S5310 obtains the biometry data described hereinabove to characterize the cephalometric geometry of interest for orthodontic treatment. A biometrics processing step S5320 then performs the processing of the cephalometric data, generating the type of data describing one or more dental/maxillofacial abnormalities shown in the examples of Figures 31A-32D. A vector generation step S5330 then generates corrective data that dictates desired movement vectors for individual teeth, as was described with reference to Figure 51. The corrective data can be generated using the AI-inverse processor described previously, for example. This corrective information can be integrated with a treatment plan in a treatment plan generation step S5340. An operator input entry step S5350 can provide additional data that is used to support a design processing step S5360, which can be fully automated, partially automated, or predominantly manual. The output of design processing step S5360, such as a print file 288 or a stream of design data, goes to a fabrication step S5370, executed by a fabrication system, such as a 3D printer or other device for appliance manufacture.

According to an embodiment of the present disclosure, design processing step S5360 translates the movement vector data generated automatically for the treatment plan into design data that supports the automated fabrication of a suitable dental appliance. As its output, design processing step S5360 can generate a suitable file or data stream for 3D printing, such as a file in .STL (Standard Triangulation Language) format, commonly used with 3D printers, or a file in .OBJ format that represents 3D geometry. Other types of print file data can be in a proprietary format, such as X3G or FBX format.

Automated fabrication systems can be additive, such as 3D printing apparatus that uses stereolithography (SLA) or other additive method that generates an object or form by depositing small amounts of material onto a base structure. Some alternate methods for additive fabrication include fused deposition modeling that applies material in a liquid state and allows the material to harden, and selective laser sintering, using a focused radiant energy to sinter metal, ceramic, or polymer particulates for forming a structure. Alternately, automated fabrication devices can be subtractive, such as using a computerized numerical control (CNC) device for machining an appliance from a block of a suitable material.

User interaction can be employed as part of the fabrication process, such as to verify and confirm results generated and displayed automatically, or to modify generated results at practitioner discretion. Thus, for example, the operator can accept some guidance from the automated system, but can still alter the generated movement vector data according to particular patient needs. Alternately, an operator at a design workstation can input and manipulate the design data in order to generate an appliance design that is customized for the patient.

Described herein is a computer-executed method that extends and enhances 3-D cephalometric analysis of maxillofacial asymmetry for a patient to provide orthodontic assessment and guidance for subsequent treatment, including fabrication of appropriate dental appliances using manual or automated methods.

Consistent with exemplary embodiments herein, a computer program can use stored instructions that perform 3D biometric analysis on image data that is accessed from an electronic memory. As can be appreciated by those skilled in the image processing arts, a computer program for operating the imaging system and probe and acquiring image data in exemplary embodiments of the application can be utilized by a suitable, general-purpose computer system operating as control logic processors as described herein, such as a personal computer or workstation. However, many other types of computer systems can be used to execute the computer program of the present invention, including an arrangement of networked processors, for example. The computer program for performing exemplary method embodiments may be stored in a computer readable storage medium. This medium may include, for example; magnetic storage media such as a magnetic disk such as a hard drive or removable device or magnetic tape; optical storage media such as an optical disc, optical tape, or machine readable optical encoding; solid state electronic storage devices such as random access memory (RAM), or read only memory (ROM); or any other physical device or medium employed to store a computer program. Computer programs for performing exemplary method embodiments may also be stored on computer readable storage medium that is connected to the image processor by way of the internet or other network or communication medium. Those skilled in the art will further readily recognize that the equivalent of such a computer program product may also be constructed in hardware.

It should be noted that the term "memory", equivalent to "computer-accessible memory" in the context of the application, can refer to any type of temporary or more enduring data storage workspace used for storing and operating upon image data and accessible to a computer system, including a database, for example. The memory could be non-volatile, using, for example, a long-term storage medium such as magnetic or optical storage. Alternately, the memory could be of a more volatile nature, using an electronic circuit, such as random-access memory (RAM) that is used as a temporary buffer or workspace by a microprocessor or other control logic processor device. Display data, for example, is typically stored in a temporary storage buffer that is directly associated with a display device and is periodically refreshed as needed in order to provide displayed data. This temporary storage buffer is also considered to be a type of memory, as the term is used in the application. Memory is also used as the data workspace for executing and storing intermediate and final results of calculations and other processing. Computer-accessible memory can be volatile, non-volatile, or a hybrid combination of volatile and non-volatile types.

It will be understood that computer program products of the application may make use of various image manipulation algorithms and processes that are well known. Additional aspects of such algorithms and systems, and hardware and/or software for producing and otherwise processing the images or co-operating with the computer program product exemplary embodiments of the application, are not specifically shown or described herein and may be selected from such algorithms, systems, hardware, components and elements known in the art.

The term "at least one of" is used to mean one or more of the listed items can be selected. The term "about" indicates that the value listed can be somewhat altered, as long as the alteration does not result in nonconformance of the process or structure to the illustrated embodiment. Finally, "exemplary" indicates the description is used as an example, rather than implying that it is an ideal.

The true scope of the invention is defined by the following claims.

**Claims**

1. A method for orthodontic treatment planning, executed at least in part by a computer apparatus, comprising:

    (a) acquiring (200) three-dimensional data from scans of maxillofacial and dental anatomy of a patient;
    (b) computing a plurality of cephalometric values from the acquired three-dimensional data;
    (c) processing the computed cephalometric values and generating metrics indicative of initial tooth positioning along a dental arch of the patient;
    (d) generating metrics indicative of corrected tooth positioning according to a desired dental arch form;
    (e) analyzing the metrics generated in steps (c) and (d) to calculate desired movement vectors (V) for individual teeth within the dental arch; and
    (f) displaying the generated metrics for both the initial and corrected tooth positioning and the calculated desired movement vectors (V) along the dental arch in an overlaid manner.

2. The method of claim 1, further comprising storing and/or transmitting the generated metrics for both the initial and corrected tooth positioning and the calculated desired movement vectors (V).

3. The method of claim 1 or 2, further comprising decomposing the calculated desired movement vectors (V) along orthogonal tangential and normal directions and displaying vector data indicative of the decomposition.

4. The method of claim 1 or 2, wherein acquiring three-dimensional data comprises acquiring data from a cone beam computed tomography system and/or an intraoral optical scanner.

5. The method of claim 1 or 2, further comprising fabricating one or more orthodontic appliances according to the calculated desired movement vectors (V).

6. The method of claim 5, wherein transmitting the calculated desired movement vectors (V) comprises transmitting to an automated fabrication apparatus.

7. The method of claim 5, wherein fabricating comprises accepting operator commands related to desired tooth movement.

8. The method of claim 1 or 2, wherein processing generates a target arch form using patient anatomy and population data; and/or
wherein processing specifies a predetermined target arch form according to patient anatomy and population data.

9. The method of claim 1 or 2, wherein the desired movement vectors show repositioning of inertia centers of the teeth for orthodontic treatment having a single stage; and/or
wherein the desired movement vectors show repositioning of inertia centers of the teeth for a single stage of orthodontic treatment having a plurality of stages.

10. The method of claim 1 or 2, wherein the calculated movement vectors are provided as a listing of coordinate values.

11. The method of claim 1 or 2, wherein displaying the movement vectors further comprises displaying the vectors overlaid onto a 2D outline of the teeth in the dental arch for actual or desired tooth movement; and/or wherein displaying the movement vectors further comprises displaying the vectors overlaid on a 3D representation of the teeth in some portion of an arch.

12. The method of claim 1 or 2, further comprising generating a 3D print file according to the calculated movement vectors.

13. A method for fabricating a positional corrective device,
using the method for orthodontic treatment planning of any one of claims 1-12, according to the calculated desired movement vectors (V).

14. An apparatus for providing guidance for orthodontics, the apparatus comprising:

(a) a scan apparatus (100; 270) configured to acquire three-dimensional data from scans of maxillofacial and dental anatomy of a patient;
(b) a computer apparatus (280) programmed with instructions for:

(i) computing a plurality of cephalometric values from the acquired three-dimensional data;
(ii) processing the computed cephalometric values and generating metrics indicative of initial tooth positioning along a dental arch of the patient;
(iii) generating metrics indicative of corrected tooth positioning according to a desired dental arch form; and
(iv) analyzing the metrics generated under (ii) and (iii) to calculate desired movement vectors for individual teeth within the dental arch; and

(c) a display in signal communication (282) with the computer apparatus (280) configured to display the generated metrics for both the initial and corrected tooth positioning and the calculated desired movement vectors (V) along the dental arch in an overlaid manner.

15. The apparatus of claim 14, further comprising a fabrication apparatus (290) for automated fabrication of a dental appliance using the the calculated desired movement vectors (V), wherein the fabrication apparatus (290) is in signal communication with the computer apparatus (280).

**Patentansprüche**

1. Ein Verfahren zur kieferorthopädischen Behandlungsplanung, das zumindest teilweise durch eine Computervorrichtung ausgeführt wird, das Folgendes aufweist:

(a) Erfassen (200) von dreidimensionalen Daten aus Scans maxillofazialer und dentaler Anatomie eines Patienten;
(b) Berechnen einer Vielzahl von kephalometrischen Werten aus den erfassten dreidimensionalen Daten;
(c) Verarbeiten der berechneten kephalometrischen Werte und Erzeugen von Metriken, die die anfängliche Zahnpositionierung entlang eines Zahnbogens des Patienten anzeigen;

(d) Erzeugen von Metriken, die eine korrigierte Zahnpositionierung gemäß einer gewünschten Zahnbogenform anzeigen;

(e) Analysieren der in den Schritten (c) und (d) erzeugten Metriken, um gewünschte Bewegungsvektoren (V) für einzelne Zähne innerhalb des Zahnbogens zu berechnen; und

(f) Anzeigen der erzeugten Metriken sowohl für die anfängliche als auch für die korrigierte Zahnpositionierung und der berechneten gewünschten Bewegungsvektoren (V) entlang des Zahnbogens in einer überlagerten Weise.

2. Das Verfahren nach Anspruch 1, das ferner Speichern und/oder Übertragen der erzeugten Metriken sowohl für die anfängliche und korrigierte Zahnpositionierung, als auch für die berechneten gewünschten Bewegungsvektoren (V) aufweist.

3. Das Verfahren nach Anspruch 1 oder 2, das ferner Zerlegen der berechneten gewünschten Bewegungsvektoren (V) entlang orthogonaler tangentialer und normaler Richtungen und Anzeigen von Vektordaten aufweist, die die Zerlegung anzeigen.

4. Das Verfahren nach Anspruch 1 oder 2, wobei Erfassen dreidimensionaler Daten ein Erfassen von Daten von einem Kegelstrahl-Computertomographiesystem und/oder von einem intraoralen optischen Scanner aufweist.

5. Das Verfahren nach Anspruch 1 oder 2, das ferner Herstellen einer oder mehrerer kieferorthopädischer Apparaturen entsprechend den berechneten gewünschten Bewegungsvektoren (V) aufweist.

6. Das Verfahren nach Anspruch 5, wobei Übertragen der berechneten gewünschten Bewegungsvektoren (V) ein Übertragen an eine automatisierte Herstellungsvorrichtung aufweist.

7. Das Verfahren nach Anspruch 5, wobei Herstellen ein Annehmen von Bedienerbefehlen in Bezug auf eine gewünschte Zahnbewegung aufweist.

8. Das Verfahren nach Anspruch 1 oder 2, wobei Verarbeiten eine Ziel-Bogenform unter Verwendung von Patientenanatomie- und Bevölkerungsdaten erzeugt; und/oder wobei Verarbeiten eine vorbestimmte Ziel-Bogenform entsprechend der Patientenanatomie- und der Bevölkerungsdaten spezifiziert.

9. Das Verfahren nach Anspruch 1 oder 2, wobei die gewünschten Bewegungsvektoren eine Repositionierung von Massenträgheitszentren der Zähne für eine kieferorthopädische Behandlung mit einer einzigen Stufe zeigen; und/oder

wobei die gewünschten Bewegungsvektoren Repositionierung der Massenträgheitszentren der Zähne für eine einzige Stufe einer kieferorthopädischen Behandlung mit einer Vielzahl von Stufen zeigen.

10. Das Verfahren nach Anspruch 1 oder 2, wobei die berechneten Bewegungsvektoren als eine Auflistung von Koordinatenwerten bereitgestellt werden.

11. Das Verfahren nach Anspruch 1 oder 2, wobei Anzeigen der Bewegungsvektoren ferner ein Anzeigen der Vektoren als Überlagerung auf einem 2D-Umriss der Zähne im Zahnbogen für eine tatsächliche oder gewünschte Zahnbewegung aufweist; und/oder wobei Anzeigen der Bewegungsvektoren ferner ein Anzeigen der Vektoren als Überlagerung auf einer 3D-Darstellung der Zähne in einem Teil des Zahnbogens aufweist.

12. Das Verfahren nach Anspruch 1 oder 2, das ferner Erzeugen einer 3D-Druckdatei entsprechend den berechneten Bewegungsvektoren aufweist.

13. Das Verfahren zur Herstellung einer Positionskorrektureinrichtung unter Verwendung des Verfahrens zur kieferorthopädischen Behandlungsplanung nach einem der Ansprüche 1 bis 12, und zwar entsprechend den berechneten gewünschten Bewegungsvektoren (V).

14. Eine Vorrichtung zum Bereitstellen einer Orientierungshilfe für die Kieferorthopädie, wobei die Vorrichtung Folgendes aufweist:

(a) eine Scan-Vorrichtung (100; 270), die eingerichtet ist, dreidimensionale Daten von Scans einer maxillofazialen und dentalen Anatomie eines Patienten zu erfassen;
(b) eine Computervorrichtung (280), die mit Anweisungen programmiert ist, zum:

(i) Berechnen einer Vielzahl von kephalometrischen Werten aus den erfassten dreidimensionalen Daten;
(ii) Verarbeiten der berechneten kephalometrischen Werte und Erzeugen von Metriken, die eine anfängliche Zahnpositionierung entlang eines Zahnbogens des Patienten anzeigen;
(iii) Erzeugen von Metriken, die eine korrigierte Zahnpositionierung gemäß einer gewünschten Zahnbogenform anzeigen; und
(iv) Analysieren der unter (ii) und (iii) erzeugten Metriken, um gewünschte Bewegungsvektoren für einzelne Zähne innerhalb des Zahnbogens zu berechnen; und

(c) eine Anzeige in Signalkommunikation (282) mit der Computervorrichtung (280), die eingerichtet ist, die erzeugten Metriken sowohl für die anfängliche, als auch für die korrigierte Zahnpositionierung und die berechneten gewünschten Bewegungsvektoren (V) entlang des Zahnbogens in einer überlagerten Weise anzuzeigen.

15. Die Vorrichtung nach Anspruch 14, die ferner eine Herstellungsvorrichtung (290) zur automatisierten Herstellung einer dentalen Apparatur unter Verwendung der berechneten gewünschten Bewegungsvektoren (V) aufweist, wobei die Herstellungsvorrichtung (290) in Signalkommunikation mit der Computervorrichtung (280) steht.

**Revendications**

1. Procédé de planification de traitement orthodontique, exécuté au moins en partie par un ordinateur, comprenant :

    (a) l'acquisition (200) de données tridimensionnelles à partir d'échographies de l'anatomie maxillo-faciale et dentaire d'un patient ;
    (b) le calcul d'une pluralité de valeurs céphalométriques à partir des données tridimensionnelles acquises ;
    (c) le traitement des valeurs céphalométriques calculées et la génération de mesures indicatives du positionnement initial de dents le long d'une arcade dentaire du patient ;
    (d) la génération de mesures indicatives du positionnement corrigé des dents conformément à une forme souhaitée de l'arcade dentaire ;
    (e) l'analyse des mesures générées dans les étapes (c) et (d) pour calculer les vecteurs de mouvement souhaités (V) pour des dents individuelles au sein de l'arcade dentaire ; et
    (f) l'affichage des mesures générées à la fois pour les positionnements initial et souhaité des dents et pour les vecteurs de mouvement souhaités calculés (V) le long d'une arcade dentaire d'une façon superposée.

2. Procédé selon la revendication 1, comprenant en outre le stockage et/ou la transmission des mesures générées à la fois pour les positionnements initial et souhaité des dents et pour les vecteurs de mouvement souhaités calculés (V).

3. Procédé selon la revendication 1 ou 2, comprenant en outre la décomposition des vecteurs de mouvement souhaités calculés (V) le long de directions normales et tangentielles orthogonales et l'affichage de données vectorielles indicatives de la décomposition.

4. Procédé selon la revendication 1 ou 2, dans lequel l'acquisition de données tridimensionnelles comprend l'acquisition de données provenant d'un système de tomodensitométrie calculé à faisceau conique et/ou d'un scanner optique intra-oral.

5. Procédé selon la revendication 1 ou 2, comprenant en outre la fabrication d'un ou de plusieurs appareils orthodontiques conformément aux vecteurs de mouvement souhaités calculés.

6. Procédé selon la revendication 5, dans lequel la transmission des vecteurs de mouvement souhaités calculés (V) comprend la transmission à un appareil de fabrication automatisé.

7. Procédé selon la revendication 5, dans lequel la fabrication comprend l'acceptation de commandes d'opérateur liées au mouvement souhaité d'une dent.

8. Procédé selon la revendication 1 ou 2, dans lequel le traitement génère une forme d'arcade cible en utilisant des données sur l'anatomie du patient et sur la population ; et/ou
dans lequel le traitement spécifie une forme d'arcade cible prédéterminée conformément à l'anatomie du patient et des données de population.

9. Procédé selon la revendication 1 ou 2, dans lequel les vecteurs de mouvement souhaités montrent le repositionnement de centres d'inertie des dents pour un traitement orthodontique comportant une seule étape ; et/ou
dans lequel les vecteurs de mouvement souhaités montrent le repositionnement de centres d'inertie des dents pour une seule étape de traitement orthodontique comprenant une pluralité d'étapes.

10. Procédé selon la revendication 1 ou 2, dans lequel les vecteurs de mouvement calculés sont fournis sous la forme d'une liste de valeurs de coordonnées.

11. Procédé selon la revendication 1 ou 2, dans lequel l'affichage des vecteurs de mouvement comprend en outre l'affichage des vecteurs superposés sur un contour 2D des dents dans l'arcade dentaire pour un mouvement dentaire réel ou souhaité ; et/ou dans lequel l'affichage des vecteurs de mouvement comprend en outre l'affichage des vecteurs superposés sur une représentation 3D des dents dans une certaine partie d'une arcade.

12. Procédé selon la revendication 1 ou 2, comprenant en outre la génération d'un fichier d'impression 3D conformément aux vecteurs de mouvement calculés.

**13.** Procédé de fabrication d'un dispositif de correction de position, utilisant le procédé de planification de traitement orthodontique selon l'une quelconque des revendications 1 à 12, conformément aux vecteurs de mouvement souhaités calculés (V).

**14.** Appareil pour fournir un guidage en orthodontie, l'appareil comprenant :

(a) un appareil d'échographie (100 ; 270) configuré pour acquérir des données tridimensionnelles à partir d'échographies de l'anatomie maxillo-faciale et dentaire d'un patient ;

(b) un appareil informatique (280) programmé avec des instructions pour :

(i) calculer une pluralité de valeurs céphalométriques à partir des données tridimensionnelles acquises ;

(ii) traiter les valeurs céphalométriques calculées et générer des mesures indicatives du positionnement initial des dents le long d'une arcade dentaire du patient ;

(iii) générer des mesures indicatives du positionnement corrigé des dents conformément à une forme souhaitée de l'arcade dentaire ;

(iv) analyser des mesures générées dans les étapes (ii) et (iii) pour calculer les vecteurs de mouvement souhaités pour les dents individuelles au sein de l'arcade dentaire ;

(c) un affichage en communication de signal (282) avec l'appareil informatique (280) configuré pour afficher les mesures générées à la fois pour les positionnements initial et souhaité des dents et pour les vecteurs de mouvement souhaités calculés (V) le long d'une arcade dentaire d'une façon superposée.

**15.** Appareil selon la revendication 14, comprenant en outre un appareil de fabrication (290) pour la fabrication automatisée d'un appareil dentaire en utilisant les vecteurs de mouvement souhaités calculés (V), dans lequel l'appareil de fabrication (290) est en communication de signal avec l'appareil informatique (280).

**FIG. 1**

**FIG. 2**

202

FIG. 3

302

304

FIG. 4

*FIG. 5*

**FIG. 6**

**FIG. 7A**

**FIG. 7B**

**FIG. 7C**

EP 3 672 477 B1

3-D image - - - - - - - -> | Display views | S200

| List landmarks of interest | S210

| Record reference mark x, y, z | S220

| Verify and identify | S230

| Connect and display framework | S240

| Analyze and display parameters | S250

**FIG. 8**

**FIG. 9A**

EP 3 672 477 B1

**FIG. 9B**

EP 3 672 477 B1

FIG. 9C

**FIG. 10A**

**FIG. 10B**

EP 3 672 477 B1

FIG. 10C

**FIG. 10D**

EP 3 672 477 B1

*FIG. 10E*

FIG. 11

*FIG. 12*

**FIG. 13**

*FIG. 14*

*FIG. 15*

2202

2204

912

## FIG. 16A

Upper digital jaw inertia system 2206

Tooth mass and mass center

Lower digital jaw inertia system 2208

## FIG. 16B

MX1_MD1_RF = 0

Frontal view

2214

2216

**FIG. 17B**

MX1_MD1_RS = 0

Sagittal view

2210

2212

**FIG. 17A**

FIG. 18B

FIG. 18A

MX1_MD1_RS = 2.81

Sagittal view

2210

2212

**FIG. 19A**

MX1_MD1_RF = -1.8

Frontal view

2214

2216

**FIG. 19B**

**FIG. 20B**

**FIG. 20A**

2402

**FIG. 21A**

2404

**FIG. 21B**

2500

**FIG. 22A**

Upper(blue) & lower(red) jaws principal inertia axes sagittal view

2510

FIG. 22B

2600

FIG. 23A

Upper(blue) & lower(red) jaws principal inertia axes sagittal view

2610

2612

2614

*FIG. 23B*

FIG. 24

FIG. 25

FIG. 26

**FIG. 27**

*FIG. 28*

$independent\_diagnosis\_a\lg orithm$

$x_m, y_m \in [normal\_parameters, \quad abnormal\_parameters]$

$x_m \neq y_m; \quad m \in [1,2,\cdots,13]$

**FIG. 29**

$given \quad variables \quad x_m, y_m:$

$define \quad diagnosis\_result\_matrix(3x3) \quad \mathbf{D}_m$

$evaluate \quad vector \quad \mathbf{c}_m = [-\infty < x_m \leq \mu_{x_m} - \sigma_{x_m}, \mu_{x_m} - \sigma_{x_m} < x_m < \mu_{x_m} + \sigma_{x_m}, \mu_{x_m} + \sigma_{x_m} \leq x_m < \infty]$

$evaluate \quad vector \quad \mathbf{r}_m = [-\infty < y_m \leq \mu_{y_m} - \sigma_{y_m}, \mu_{y_m} - \sigma_{y_m} < y_m < \mu_{y_m} + \sigma_{y_m}, \mu_{y_m} + \sigma_{y_m} \leq y_m < \infty]$

$\mu = Class\_I \; mean \; ; \quad \sigma = Class\_I\_deviation$

$\mathbf{D}_m(i,j) = true \quad if \quad \mathbf{c}_m(j) = true \quad and \quad \mathbf{r}_m(i) = true;$

$i,j \in [1,2,3].$

$dependent\_diagnosis\_a\lg orithm$

$x_k, y_k \in [normal\_parameters, \quad abnormal\_parameters]$

$x_k \neq y_k; k \in [m,n]; \quad m,n \in [1,2,\cdots,12,13]; m \neq n$

**FIG. 30**

$given \quad variables \quad x_k, y_k:$

$define \quad diagnosis\_matrices(3x3) \quad \mathbf{D}_m, \mathbf{D}_n$

$evaluate \quad vector \quad \mathbf{c}_k = [-\infty < x_k \leq \mu_{x_k} - \sigma_{x_k}, \mu_{x_k} - \sigma_{x_k} < x_k < \mu_{x_k} + \sigma_{x_k}, \mu_{x_k} + \sigma_{x_k} \leq x_k < \infty]$

$evaluate \quad vector \quad \mathbf{r}_k = [-\infty < y_k \leq \mu_{y_k} - \sigma_{y_k}, \mu_{y_k} - \sigma_{y_k} < y_k < \mu_{y_k} + \sigma_{y_k}, \mu_{y_k} + \sigma_{y_k} \leq y_k < \infty]$

$\mu = Class\_I\_ \; mean \quad \sigma = Class\_I\_deviation$

$\mathbf{D}_m(i,j) = true \quad if \quad \mathbf{c}_m(j) = true \quad and \quad \mathbf{r}_m(i) = true;$

$\mathbf{D}_n(i,j) = true \quad if \quad \mathbf{c}_n(j) = true \quad and \quad \mathbf{r}_n(i) = true;$

$\mathbf{D}_m(i,j) = \mathbf{D}_m(i,j) + \mathbf{D}_n(i,j); \quad if \quad i+j \neq 4(exemplary)$

$i,j \in [1,2,3]; k \in [m,n].$

| Case Name: Cl III @ | | |
|---|---|---|
| **Explicited Parameters** | | |
| **Antero-posterior** | | |
| alveolar~GM-Gm | -10.479 | Overjet, GMx-Gmx, centers of inertia of upper and lower incisors |
| alveolar~GM-Gm | -8.899 | Dental class I, Gmy-Gmy |
| alveolar~TgM | 37.0168 | Angular maxillar incisors protrusion (torque), medium upper incisors torque |
| alveolar~Tgm | 11.2776 | Angular mandibular incisors protrusion(torque), medium lower incisors torque |
| alveolar~GM+Gm/2 or Gy | 23.0233 | Linear bipretrusion, (GMy+Gmy)/2 |
| base~MrlP-MM | -10.028 | Basic class II, MMPy-MMy |
| base~MFM-MM | 58.0601 | Corpus length, This is the real length between MFM and MM |
| architecture~MMy | 19.0165 | Chin protrusion, y coordinate of medium mental |
| architecture~MFM-MM | 106.922 | Mandibular global length, This is the real length between MFM and MM |
| **Vertical** | | |
| alveolar~Gdz | 37.0501 | Arches altitude, z coordinate of the inertia center of all the teeth |
| alveolar~MM-MM | -6.8922 | Inter-arches divergence MxW2(SP) |
| base~MFM-MO-MFM-MM | 27.689 | Pseudo FMA(orbital floor-corpus angle) |
| architecture~MMz | 61.97 | Maxillo-mandibular height |
| architecture~13 | 35.4216 | Facial global divergence, angle between MFM-MO axis and MFM-MM axis |
| **Transverse** | | |
| alveolar~gM-gm | -0.4271 | inter-first molars diameter relationship |
| alveolar~TgM-Tgm | 27.5746 | Class II or mandibular excess angular compensation |
| base~RGP-LGP/RFM-LFM | 36.2319 | Base maxillar/mandibular excess, LGPx-RGPx/LFMx-RFMx |
| architecture~RO-LO/RM-LM | 115.909 | ante-orbital/Antero mandibular width, formula: ROx-LOx/RMx-LMx |
| **Mechanical** | | |
| hidden~GM | 17.7837 | Upper incisors group protrusion |
| hidden~Gm | 28.2629 | Lower incisors group protrusion |
| hidden~(TgM+Tgm)/2 | 24.1472 | Medium upper and lower incisors torque |
| hidden~TgM-Tgm | 25.7392 | Upper and lower incisor torque difference |
| hidden~MMPy | 8.98965 | Maxilla protrusion |
| hidden~GM-MMP(y) | 8.79502 | Upper linear class II compensation |
| hidden~Gm-MM(y) | 9.24639 | Upper linear class II compensation |
| hidden~Gdz/(MMz-Gdz) | 0.44600 | Maxi-mand ratio of maxi-mand global height |

| Ind | 15_Asym_vert_params | Measur | Comment | Norm | mean | std | -S | +S | -Ss | +Ss |
|---|---|---|---|---|---|---|---|---|---|---|
| | Vertical | m/de | | m/de | m/de | m/de | m/de | m/de | m/de | m/de |
| 1 | alv.arch.rol~G16z-G36z | 0.19 | Right/left upper molar height difference (+:Right lower) (T12) | 0 | -0.07 | 1.42 | -1.49 | 1.35 | -1.55 | 1.41 |
| 2 | alv.arch.rol~G46z-G36z | 0.1 | Right/left lower molar height difference (+:Right lower) (T13) | 0 | -0.13 | 1.51 | -1.65 | 1.38 | -1.71 | 1.44 |
| 3 | alv.arch.rol~<Mx), Vx> | -0.32 | Maxilla roling (+:Right side lower) (T12) | 0 | -0.14 | 1.77 | -1.91 | 1.63 | -1.91 | 1.63 |
| 4 | alv.arch.rol~<Mdl, Vx> | -1.85 | Mandible roling (+:Right side lower) (T13) | 0 | -0.18 | 1.77 | -1.96 | 1.59 | -1.96 | 1.59 |
| 5 | alv.comp.arch.rol~(G16z-G26z)-(RGPz-LGPz) | 0.51 | Right/left alveolar height difference(+:Right higher) | 0 | 0.37 | 1.29 | -0.92 | 1.66 | -0.95 | 1.72 |
| 6 | alv.comp.arch.rol~(G46z-G36z)-(RFMz-LFMz) | -2.7 | Right/left alveolar height difference(+:Right higher) | 0 | 0.69 | 2.12 | -1.43 | 2.81 | -1.49 | 2.92 |
| 7 | bas.bone.rol~RGPz-LGPz | -0.32 | Maxilla right/left height difference (+:Right lower) (T14) | 0 | -0.44 | 1.23 | -1.67 | 0.79 | -1.74 | 0.82 |
| 8 | bas.bone.rol~RFMz-LFMz | 2.8 | Mandible right/left height difference (+:Right lower) (T14) | 0 | -0.82 | 2.98 | -3.8 | 2.16 | -3.95 | 2.24 |
| 9 | bas.bone.rol~(RFM-RHM)-(LFM-LHM) | 1.44 | Vertical branches right/left height difference (+:Right higher) projected on Z | 0 | 0.47 | 2.03 | -1.56 | 2.49 | -1.62 | 2.59 |
| 10 | bas.bone.rol~<RFM-RM,RHM-RIO>-<LFM-LM,LHM-LIO> | -0.9 | Difference of right/left basic mandible divergence | 0 | 0.06 | 1.57 | -1.51 | 1.63 | -1.51 | 1.63 |
| 11 | arch.fd.lines.rol~<RM,RHM,RIO>-<LM,LHM,LIO> | 0.67 | Difference of right/left maxilla-mandible divergences | 0 | -0.16 | 1.36 | -1.52 | 1.2 | -1.52 | 1.2 |
| 12 | arch.fd.lines.rol~<RM,RHM,RSO>-<LM,LHM,LSO> | 0.1 | Difference of right/left global face divergences | 0 | 0.08 | 1.78 | -1.71 | 1.86 | -1.71 | 1.86 |
| 13 | arch.fd.lines.rol~(RIO-RSO)-(LIO-LSO) | -1.14 | Orbital right/left heights difference projected on Z | 0 | 0.09 | 1.56 | -1.48 | 1.65 | -1.54 | 1.71 |
| 14 | arch.fd.lines.rol~RMz-LMz | 0.53 | Maxilla-mandible right/left heights difference (T15) | 0 | -0.12 | 1.52 | -1.64 | 1.4 | -1.7 | 1.45 |
| 15 | arch.fd.lines.rol~(RM-RSO)-(LM-LSO) | -0.61 | Global face right/left heights difference projected on Z (T15) | 0 | -0.04 | 1.27 | -1.3 | 1.23 | -1.35 | 1.28 |

**FIG. 31B**

| Ind | 16_Asym_ap_params | Measur | Comment | Norm | mean | std | -S | +S | -Ss | +Ss |
|---|---|---|---|---|---|---|---|---|---|---|
| | Anterior-posterior | nm/deg | | nm/degnm/degnm/degnm/degnm/degnm/degnm/degr |
| 1 | alveolar.AP.RL~G16y-G26y | -0.11 | AP 16/26 difference (T16) | 0 | -0.05 | 1.27 | -1.32 | 1.22 | -1.29 | 1.19 |
| 2 | alveolar.miaGap~G46y-G36y | 0.34 | AP 46/36 difference (T16) | 0 | 0.12 | 1.32 | -1.2 | 1.44 | -1.17 | 1.4 |
| 3 | alv.AP.miar~(G16y-G26y)-(RGPy-LGPy) | -0.5 | Upper molar AP position/maxilla right/left difference | 0 | -0.37 | 1.49 | -1.86 | 1.12 | -1.81 | 1.08 |
| 4 | alv.cmp.shiftng~(G46-G36)y-(RFM-LFM)y | 0.93 | Lower molar AP position/mandible right/left difference | 0 | -0.05 | 2.26 | -2.31 | 2.21 | -2.26 | 2.16 |
| 5 | alveolar.AP.CII_R~G16y-G46y | 1.5 | Right Class II; negative value means Class III relationship (T17) | -3.09 | -3.09 | 1.55 | -4.63 | -1.54 | -4.53 | -1.5 |
| 6 | alveolar.AP.CII_L~G26y-G36y | 2 | Left Class II; negative value means Class II relationship (T17) | -2.92 | -2.92 | 1.21 | -4.13 | -1.71 | -4.03 | -1.67 |
| 7 | base.RL~(RM-RFM)-(LM-LFM) | -0.48 | Horizontal branches right/left difference projected on Y (T19) | 0 | -0.93 | 2.24 | -3.16 | 1.11 | -3.09 | 1.28 |
| 8 | basis.morpho~RGPy-LGPy | 0.38 | AP great palatal right/left position difference (T18) | 0 | 0.32 | 1.34 | -1.02 | 1.66 | -0.99 | 1.62 |
| 9 | base.off~RFMy-LFMy | -0.59 | AP mandible foramen right/left position difference (T18) | 0 | 0.17 | 2.55 | -2.38 | 2.72 | -2.32 | 2.66 |
| 10 | arch.AP.RL.FdExcss~(RIO-RHM)-(LIO-LHM) | -0.33 | Facial depth right/left difference (IFO level) | 0 | -0.09 | 2.95 | -3.05 | 2.86 | -2.98 | 2.79 |
| 11 | arch.AP.RL.FdExcss~(RSO-RHM)-(LSO-LHM) | 0.22 | Facial depth right/left difference (SPO level) | 0 | 0.22 | 2.22 | -2 | 2.44 | -1.96 | 2.38 |
| 12 | arch.AP.RL.FdExcss~RSOy-LSOy | 0.55 | AP SPO positions, right/left difference | 0 | 0.31 | 1.46 | -1.15 | 1.77 | -1.12 | 1.73 |
| 13 | arch.AP.RL.FdExcss~RMy-LMy | -1.07 | AP mental positions, right/left difference (T19) | 0 | -0.76 | 1.7 | -2.46 | 0.95 | -2.4 | 0.93 |
| 14 | arch.AP.RL.FdExcss~(RM-RHM)-(LM-LHM) | -1.4 | Facial depth right/left difference (mental level) projected on Y | 0 | -0.85 | 2.63 | -3.48 | 1.78 | -3.39 | 1.74 |
| 15 | arch.AP.RL.FdExcss~<RHM,RIO,RM>-<LHM,LIO,LM> | -1.29 | Mandible global opening right/left difference | 0 | 0.3 | 3.06 | -2.77 | 3.36 | -2.72 | 3.26 |
| 16 | addition.AP.RL~(G16y-G46y)-(G26y-G36y) | -0.45 | Class 2 right/left difference | 0 | -0.17 | 0.81 | -0.98 | 0.64 | -0.95 | 0.63 |

## FIG. 31C

| nd | 32_Asym_transv_params | Measur | Comment | Norm | mean | std | -S | +S | -Ss | +Ss |
|---|---|---|---|---|---|---|---|---|---|---|
| | Transverse | m/deg | | m/deg | m/deg | m/deg | m/deg | m/deg | m/deg | m/degr |
| 1 | alv.grp.lnr.transv.pos~GlMx | 0.14 | Upper incisors transversal deviation (T4) | 0 | 0.19 | 1.46 | -1.3 | 1.65 | -1.2 | 1.61 |
| 2 | alv.grp.lnr.transv.pos~Gmx | 0.19 | Lower incisors transversal deviation (T5) | 0 | -0.1 | 2.09 | -2.2 | 1.99 | -2.1 | 1.94 |
| 3 | alv.grp.lnr.transv.pos~GMx | 0.97 | Upper arch transversal deviation | 0 | 0.18 | 1.6 | -1.4 | 1.78 | -1.4 | 1.74 |
| 4 | alv.grp.lnr.transv.pos~Gmx | 0.58 | Lower arch transversal deviation | 0 | -0.1 | 1.89 | -2 | 1.8 | -1.9 | 1.76 |
| 5 | alv.grp.lnr.transv.pos~GMMx | 0.04 | Upper molar transversal deviation (T6) | 0 | 0.04 | 1.5 | -1.5 | 1.54 | -1.4 | 1.51 |
| 6 | alv.grp.lnr.transv.pos~Gmmx | 0.51 | Lower molar transversal deviation (T7) | 0 | 0.01 | 1.92 | -1.9 | 1.92 | -1.9 | 1.86 |
| 7 | alv.arch.rtatn~<Mxl,Vy>(RH) | -0.9 | Upper arch rotation in arch plane (+:Right) (T3) | 0 | 0.58 | 2.65 | -2.3 | 3.44 | -2.3 | 3.44 |
| 8 | alve.arch.rtatn~<Mdl,Vy>(RH) | -3.3 | Lower arch rotation in arch plane (+:Right) (T3) | 0 | -0 | 2.8 | -2.8 | 2.78 | -2.8 | 2.78 |
| 9 | alv.lnr.transv.comp~GlMx-MNPx | -0.2 | Deviation: upper incisors/maxilla anterior landmark | 0 | -0.1 | 0.68 | -0.8 | 0.55 | -0.8 | 0.54 |
| 10 | alv.lnr.transv.comp~Gmx-MMx | -0.6 | Deviation: lower incisors/mandible anterior landmark | 0 | 0.41 | 0.69 | -0.3 | 1.09 | -0.3 | 1.07 |
| 11 | alv.lnr.transv.comp~GMx-GBMx | 1.1 | Deviation: upper arch/maxilla | 0 | 0.06 | 0.78 | -0.7 | 0.84 | -0.7 | 0.82 |
| 12 | alv.lnr.transv.comp~Gmx-GBmx | 0.17 | Deviation: lower arch/mandible | 0 | 0.26 | 0.79 | -0.5 | 1.05 | -0.5 | 1.03 |
| 13 | alv.lnr.transv.comp~GMMx-MGPx | 0.02 | Deviation: upper molars/posterior maxilla | 0 | 0.11 | 0.74 | -0.6 | 0.85 | -0.6 | 0.83 |
| 14 | alv.lnr.transv.comp~Gmmx-MFMx | 0.47 | Deviation: lower molars/posterior mandible | 0 | 0.18 | 1.16 | -1 | 1.34 | -1 | 1.31 |
| 15 | alv.torq.comp~Tq_16_17-Tq_26_2 | -1.1 | Upper molars torque compensation of maxilla left deviation | 0 | 1.58 | 5.64 | -4.1 | 7.32 | -4.1 | 7.32 |
| 16 | alv.torq.comp~Tq_36_37-Tq_46_4 | 12.4 | Upper molars torque compensation of mandible right deviation | 0 | -1.6 | 4.18 | -5.8 | 2.58 | -5.8 | 2.58 |
| 17 | alv.UL.lnr.dev~GlMx-Gmx | -0.1 | Upper/Lower incisors left deviation (T1,T4,T5,T11) | 0 | 0.3 | 1.02 | -0.7 | 1.31 | -0.7 | 1.28 |
| 18 | alv.UL.lnr.dev~GMx-Gmx | 0.4 | Upper/Lower arch left deviation | 0 | 0.27 | 0.88 | -0.6 | 1.14 | -0.6 | 1.12 |
| 19 | alv.UL.lnr.dev~GMMx-Gmmx | -0.5 | Upper/Lower molars left deviation (T1,T6,T7) | 0 | 0.04 | 0.76 | -0.7 | 0.8 | -0.7 | 0.78 |
| 20 | alv.agular.dev~<Mxl,Vy>-<Mdl,Vy> | 3 | Upper/Lower arch right deviation (+: maxilla right deviation) (T2) | 0 | 0.6 | 2.53 | -1.9 | 3.13 | -1.9 | 3.13 |
| 21 | bas.bone.UorL.lnr.dev~MGPx | -0.6 | mid-great-palatal left deviation | 0 | -0.1 | 1.35 | -1.4 | 1.28 | -1.4 | 1.25 |
| 22 | bas.bone.UorL.lnr.dev~MFMx | 0.04 | mid-mandible-foramen left deviation | 0 | -0.2 | 2.05 | -2.2 | 1.87 | -2.2 | 1.83 |
| 23 | bas.bone.UorL.lnr.dev~MNPx | 0.34 | Naso-palatal left deviation (T9) | 0 | 0.32 | 1.09 | -0.8 | 1.42 | -0.8 | 1.38 |
| 24 | bas.bone.UorL.lnr.dev~MMx | 0.76 | mid-mental-foramen left deviation (T10) | 0 | -0.5 | 2.23 | -2.7 | 1.72 | -2.7 | 1.68 |
| 25 | bas.bone.UorL.lnr.dev~GBMx | -0.1 | Maxilla left deviation | 0 | 0.13 | 1.09 | -1 | 1.22 | -0.9 | 1.19 |
| 26 | bas.bone.UorL.lnr.dev~GBmx | 0.4 | Mandible left deviation | 0 | -0.3 | 1.96 | -2.3 | 1.62 | -2.3 | 1.58 |
| 27 | bas.bone.UL.lnr.dev~MGPx-MFMx | -0.6 | Maxilla/mandible posterior left deviation | 0 | 0.11 | 1.2 | -1.1 | 1.31 | -1.1 | 1.28 |
| 28 | bas.bone.UL.lnr.dev~MNPx-MMx | -0.4 | Maxilla/mandible anterior left deviation (T8,T9,T10,T11) | 0 | 0.83 | 1.52 | -0.7 | 2.35 | -0.7 | 2.3 |
| 29 | bas.bone.UL.lnr.dev~GBMx-GBmx | -0.5 | Maxilla/mandible global left deviation (T8) | 0 | 0.47 | 1.18 | -0.7 | 1.65 | -0.7 | 1.61 |
| 30 | arch.facial.dvatn~MSOx-MMx | -1.2 | Chin/facial deviation | 0 | 0.67 | 3.1 | -2.4 | 3.77 | -2.4 | 3.68 |
| 31 | arch.facial.dvatn~1_angle | -89 | Chin/orbital floor right deviation | 0 | -89 | 1.99 | -91 | -89 | -91 | -89 |
| 32 | arch.facial.dvatn~2_angle | 90.5 | Orbital floor/ceiling left deviation | 0 | 89.8 | 1.82 | 88 | 91.6 | 88 | 91.6 |

## FIG. 31D

FIG. 32A

FIG. 32B

EP 3 672 477 B1

Giy (5):(col) vs Global incisors torque (21):row | Linear biretrusion (-) | 0 | Linear biprotrusion (+)

Angular biretrusion (-)
0
Angular biprotrusion (+)

AI 8 Linear and angular
biretrusion/biprotrusion

Linear and angular biretrusion
Linear biretrusion
Linear biretrusion despite angular biprotrusion

Angular biretrusion

Linear biprotrusion despite angular biretrusion
Linear biprotrusion

Angular biprotrusion    linear and angular biprotrusion

3200

**Case Name: 9500 9500 VOL 16**

## FIG. 32C

3292

Row 0
Row 1
Row 2
Row 3

Column 0    Column 1    C(2,2)    Column 2    Column 3

3294

## FIG. 32E

**Case Name: 9500 9500 VOL 16**

*3200*

| AI | Parameters | Diagnosis |
|---|---|---|
| AI 1 Matching incisors and global arches antero-posterior upper/lower dyscrepancy | Incisors relation (1):col vs Arches angle relation (2):row | Underjet without Class III |
| AI 2 Matching incisors dyscrepancy and separate linear upper (and lower) incisors positions | Incisors relation (1):col vs Upper incisor position (19):row | Underjet by upper incisors retrusion |
| AI 3 Matching incisors dyscrepancy and separate linear (upper and) lower incisors positions | Incisors relation (1):col vs Lower incisor position (20):row | 0 |
| AI 4 Matching incisors gap & upper-lower CL II torque differential (CL III compensation) | Incisors relation (1):col vs Torque Upper/Lower difference (22):row | 0 |
| AI 5 Upper-lower separate responsibilities concerning upper-lower CL II torq. diff. (CL III compen.) | Upper incisors torque (3):(col) vs lower incisors torque (4):row | --- |
| AI 6 Matching alveolar and basic upper/lower relationship | Dental Class II (2):(col) vs basic Class II (6):row | Occlusal Class I despite basic Class III |
| AI 7 Upper/lower separate responsibilities concerning skelettal CL II and CL III | MNPy (23):(col) vs MMy (8):row | |
| AI 8 Linear and angular biretrusion/biprotrusion | Giy (5):(col) vs Global incisors torque (21):row | --- |
| AI 9 Global linear facial vertical height and its distribution | MMz (10):(col) vs Gdz/(MMz-Gdz) (26):row | Long face syndrom (linear) due to mandible excess |
| AI 10 Linear and angular facial dysharmony | MMz (10):(col) vs 13 (14):row | |
| AI 11 Alveolar and basi divergences | MxII-MdII (11):(col) vs FMA (12):row | Basic hyperdivergence in agreement with facial hyperdivergence |
| AI 12 Transverse linear and angular upper/lower alveolar relationship | dM-dm (15):(col) vs TqM-Tqm (16):row | --- |
| AI 13 Transverse linear and angular upper/lower basic relationship | RGP-LGP/RFM-LFM (17):(col) vs RIO-LIO/RM-LM (18):row | Maxilla basic excess and mental deficit |

# FIG. 32D

**FIG. 33**

EP 3 672 477 B1

**FIG. 34**

EP 3 672 477 B1

T1; P19,17;C2,1; Upper/Lower incisors right deviation
T2; P20;C1,2; 0
T3; P8,7;C2,2; 0
T4; P1,17;C2,1; Upper/lower incisors right deviation
T5; P2,17;C2,1; 0
T6; P5,19;C2,2; 0
T7; P6,19;C2,2; 0
T8; P27,28;C2,1; 0
T9; P23,28;C2,1; 0
T10; P24,28;C3,1; Upper/Lower anterior landmarks right deviation is due to chin left deviation
T11; P28,17;C1,1; The upper/Lower incisors right deviation is in agreement with upper/lower base dev
T12; P3,1;C2,2; ---
T13; P4,2;C2,2; ---
T14; P8,7;C2,2; ---
T15; P15,14;C2,2; 0
T16; P2,1;C2,2; 0
T17; P6,5;C3,2; Left Class II
T18; P9,8;C2,2; ---
T19; P13,7;C3,3; Excess of the right hemiface and of the right horizontal branch

S1 Anterior-posterior direction synthetic comment = strong right anteroposterior excess
S2 Vertical direction synthetic comment = none
S3 Transversal direction synthetic comment = left upper deviation (right excess) tendency

## FIG. 35

EP 3 672 477 B1

FIG. 36

*FIG. 37*

**FIG. 38**

**FIG. 39**

*FIG. 40*

$\boldsymbol{p_k}$

4100

FIG. 41

**FIG. 42**

EP 3 672 477 B1

**FIG. 43**

**FIG. 44**

FIG. 45

**FIG. 46**

FIG. 47

FIG. 48

EP 3 672 477 B1

$v =$

$$
\begin{array}{rr}
0.060216810999002 & -1.939469814300550 \\
-0.321751025583261 & -1.986539915204047 \\
-1.458384946009677 & -1.713020563125610 \\
0.623361272907932 & -1.893559455871600 \\
0.137803478571172 & -1.241618156433102 \\
-0.620956778526276 & -0.326536037296485 \\
-0.813180685043326 & 0.784521218385265 \\
-0.813180685043326 & 0.798433419313064 \\
-0.620956778526276 & -0.128452159732987 \\
-1.400206013056017 & 1.241618156433098 \\
-0.533596677876147 & 1.893559455871600 \\
-0.089250132420439 & 1.713020563125610 \\
-0.331613155935255 & 1.986539915204046 \\
-0.060216810998991 & 1.939469814300550
\end{array}
$$

**FIG. 49**

5000

**FIG. 50A**

5010

**FIG. 50B**

*FIG. 50C*

FIG. 50D

FIG. 51

EP 3 672 477 B1

**FIG. 52**

```
S5310 ──  Biometry
           acquisition
              │
              ▼
S5320 ──  Biometrics              Operator
           processing              input
                                      S5350
              │                        │
              ▼                        ▼
S5330 ──  Corrective    ──►   Design        ──►   Fabrication   ── S5370
           vector                processing             system
           generation      S5360 ──
              │                     ▲
              └──────────►  Treatment
                            plan    S5340
                                          288
```

**FIG. 53**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6879712 B2, Tuncay et al.
- US 6250918 B1, Sachdeva et al.
- US 20090191503 A1
- US 2014288894 A
- US 20130022252, Chen
- US 20130022255, Chen
- US 20130022254, Chen

### Non-patent literature cited in the description

- **STEINER.** Cephalometrics in Clinical Practice. Charles H. Tweed Foundation for Orthodontic Research, October 1956, 8-29
- **VANDANA KUMAR et al.** In vivo comparison of conventional and cone beam CT synthesized cephalograms. *Angle Orthodontics,* September 2008, 873-879
- **TREIL et al.** The human face as a 3D model for cephalometric analysis. *World Journal of Orthodontics,* 1-6
- Cephalometrics for the next millennium. **P. PLANCHE ; J. TREIL.** The Future of Orthodontics. Leuven University Press, 1998, 181-192
- **JACQUES TREIL ; B, WAYSENSON ; J. BRAGA ; J. CASTEIGT.** The Human Face as a 3D Model for Cephalometric Analysis. *World Journal of Orthodontics,* 2005, vol. 6 (5), 33-38
- **J. TREIL ; J. BRAGA ; J.-M. LOUBES ; E. MAZA ; J.-M. INGLESE ; J. CASTEIGT ; B. WAYSENSON.** 3D Tooth Modeling for Orthodontic Assessment. *Seminars in Orthodontics,* March 2009, vol. 15 (1